(19) Europäisches Patentamt
European Patent Office
Office européen des brevets

(11) Veröffentlichungsnummer: **0 294 668 B1**

(12) # EUROPÄISCHE PATENTSCHRIFT

(45) Veröffentlichungstag der Patentschrift: **07.04.93**

(51) Int. Cl.[5]: **C07C 251/00**, C07D 209/49, A01N 37/44, A01N 43/38

(21) Anmeldenummer: **88108491.7**

(22) Anmeldetag: **27.05.88**

Die Akte enthält technische Angaben, die nach dem Eingang der Anmeldung eingereicht wurden und die nicht in dieser Patentschrift enthalten sind.

(54) **(2-Cyan-2-oximinoacetyl)-aminosäure-Derivate.**

(30) Priorität: **09.06.87 DE 3719226**

(43) Veröffentlichungstag der Anmeldung:
**14.12.88 Patentblatt 88/50**

(45) Bekanntmachung des Hinweises auf die Patenterteilung:
**07.04.93 Patentblatt 93/14**

(84) Benannte Vertragsstaaten:
**AT BE CH DE ES FR GB GR IT LI NL**

(56) Entgegenhaltungen:
**EP-A- 0 201 999**
**CH-A- 637 268**

(73) Patentinhaber: **BAYER AG**

**W-5090 Leverkusen 1 Bayerwerk(DE)**

(72) Erfinder: **Lunkenheimer, Winfried, Dr.**
**Bismarckstrasse 29**
**W-5600 Wuppertal 1(DE)**
Erfinder: **Brandes, Wilhelm, Dr.**
**Eichendorffstrasse 3**
**W-5653 Leichlingen(DE)**
Erfinder: **Hänssler, Gerd, Dr.**
**Am Arenzberg 58a**
**W-5090 Leverkusen 3(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

## Beschreibung

Die vorliegende Erfindung betrifft neue (2-Cyan-2-oximino-acetyl)-aminosäure-Derivate, mehrere Verfahren zu ihrer Herstellung und ihre Verwendung als Schädlingsbekämpfungsmittel, insbesondere als Fungizide.

Es ist bereits bekannt, daß bestimmte substituierte 2-Cyan-2-methoximino-acetamide gute fungizide Wirksamkeit besitzen (vgl. z.B. EP-OS 0 201 999 und DE-OS 3 602 243). Die Wirkung dieser Verbindungen ist jedoch, insbesondere bei niedrigen Aufwandmengen und -konzentrationen, nicht immer voll befriedigend.

Es wurden neue (2-Cyan-2-oximinoacetyl)-aminosäure-Derivate der allgemeinen Formel (I),

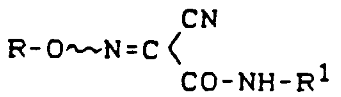

(I)

in welcher

$R^1$ für die Gruppierungen $-A-COOR^2$ oder $-B-CONR^3R^4$ steht,

A für eine gegebenenfalls substituierte, geradkettige oder verzweigte Alkylenkette steht,

B für eine gegebenenfalls substituierte, geradkettige oder verzweigte Alkylenkette steht,

$R^2$ für gegebenenfalls substituiertes Alkyl steht mit folgenden Substituenten: Halogen, Hydroxy, Alkoxy, Acyloxy, gegebenenfalls substituiertes Aryl, gegebenenfalls substituiertes Cycloalkyl und gegebenenfalls substituiertes Heterocyclyl; $R^2$ ferner für gegebenenfalls substituiertes Cycloalkyl oder gegebenenfalls substituiertes Cycloalkenyl steht,

$R^3$ für Wasserstoff oder für gegebenenfalls substituiertes Alkyl steht mit folgenden Substituenten : Halogen, Cyano, $-COOR^{I}$, $-CONR^{II}R^{III}$, $-NR^{II}R^{III}$, $-OR^{IV}$, $-S(O)_nR^{IV}$,Acyl, jeweils gegebenenfalls substituiertes Aryl, Heterocyclyl, Cycloalkyl und Cycloalkenyl; $R^3$ ferner für jeweils gegebenenfalls substituiertes Alkenyl, Alkinyl, Cycloalkyl, Cycloalkenyl, Aryl oder Heterocyclyl steht,

$R^4$ für Wasserstoff oder für gegebenenfalls substituiertes Alkyl steht mit folgenden Substituenten: Halogen, Cyano, $-COOR^{I}$, $-CONR^{II}R^{III}$, $-NR^{II}R^{III}$, $-OR^{IV}$, $-S(O)_nR^{IV}$, Acyl, jeweils gegebenenfalls substituiertes Aryl, Heterocyclyl, Cycloalkyl und Cycloalkenyl; $R^4$ ferner für jeweils gegebenenfalls substituiertes Alkenyl, Alkinyl, Cycloalkyl, Cycloalkenyl, Aryl, Heterocyclyl oder polycyclische Carbocyclen oder die Gruppierung $-OR^{IV}$ steht,
oder

A und $R^2$ gemeinsam mit den Atomen, an die sie gebunden sind, für einen gegebenenfalls substituierten Ring stehen,
oder

B und $R^3$ gemeinsam mit den Atomen, an die sie gebunden sind, für einen gegebenenfalls substituierten Ring stehen,
oder

$R^3$ und $R^4$ gemeinsam mit dem Stickstoffatom, an das sie gebunden sind, für einen gegebenenfalls substituierten Heterocyclus stehen, der weitere Heteroatome enthalten kann,

R für substituiertes Alkyl steht mit folgenden Substituenten: Halogen, $-COOR^{I}$, $-CONR^{II}R^{III}$, $-OR^{IV}$, Acyl oder gegebenenfalls substituiertes Heterocyclyl, ausgenommen Azolyl; R ferner für gegebenenfalls substituiertes Cycloalkenyl steht;

$R^{I}$ für Wasserstoff oder gegebenenfalls substituiertes Alkyl steht mit folgenden Substituenten: Halogen, jeweils gegebenenfalls substituiertes Aryl, Cycloalkyl und Cycloalkenyl,

$R^{II}$ und $R^{III}$ gleich oder verschieden sind und für Wasserstoff oder gegebenenfalls substituiertes Alkyl stehen mit folgenden Substituenten: Halogen, $-COOR^{IV}$, $-CONR^{I}R^{IV}$, jeweils gegebenenfalls substituiertes Aryl, Cycloalkyl und Cycloalkenyl; $R^{II}$ und $R^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann,

$R^{IV}$ für Wasserstoff, Alkyl, Aralkyl oder Acyl steht und

n für die Zahlen 0, 1 oder 2 steht,

gefunden.

Die Verbindungen der Formel (I) können in verschiedenen geometrischen Isomeren vorkommmen, je nach Anordnung der Substituenten an der C = N-Gruppierung (E- bzw. Z-Isomere).

Gegebenenfalls besitzen die Verbindungen ein oder mehrere asymmetrische Kohlenstoffatome; sie können somit auch als optische Isomere vorliegen (D- und L-Konfiguration), die in unterschiedlichen Mengenverhältnissen anfallen können. Vorwiegend fallen sie als Racemate an.

Weiterhin wurde gefunden, daß man die neuen (2-Cyan-2-oximinoacetyl)-aminosäure-Derivate der allgemeinen Formel (I),

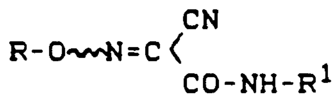

(I)

in welcher

| | |
|---|---|
| $R^1$ | für die Gruppierungen -A-COOR$^2$ oder -B-CONR$^3$R$^4$ steht, |
| A | für eine gegebenenfalls substituierte, geradkettige oder verzweigte Alkylenkette steht, |
| B | für eine gegebenenfalls substituierte, geradkettige oder verzweigte Alkylenkette steht, |
| $R^2$ | für gegebenenfalls substituiertes Alkyl steht mit folgenden Substituenten: Halogen, Hydroxy, Alkoxy, Acyloxy, gegebenenfalls substituiertes Aryl, gegebenenfalls substituiertes Cycloalkyl und gegebenenfalls substituiertes Heterocyclyl; $R^2$ ferner für gegebenenfalls substituiertes Cycloalkyl oder gegebenenfalls substituiertes Cycloalkenyl steht, |
| $R^3$ | für Wasserstoff oder für gegebenenfalls substituiertes Alkyl steht mit folgenden Substituenten : Halogen, Cyano, -COOR$^I$, -CONR$^{II}$R$^{III}$, -NR$^{II}$R$^{III}$, -OR$^{IV}$, -S(O)$_n$R$^{IV}$,Acyl, jeweils gegebenenfalls substituiertes Aryl, Heterocyclyl, Cycloalkyl und Cycloalkenyl; $R^3$ ferner für jeweils gegebenenfalls substituiertes Alkenyl, Alkinyl, Cycloalkyl, Cycloalkenyl, Aryl oder Heterocyclyl steht, |
| $R^4$ | für Wasserstoff oder für gegebenenfalls substituiertes Alkyl steht mit folgenden Substituenten: Halogen, Cyano, -COOR$^I$, -CONR$^{II}$R$^{III}$, -NR$^{II}$R$^{III}$, -OR$^{IV}$, -S(O)$_n$R$^{IV}$, Acyl, jeweils gegebenenfalls substituiertes Aryl, Heterocyclyl, Cycloalkyl und Cycloalkenyl; $R^4$ ferner für jeweils gegebenenfalls substituiertes Alkenyl, Alkinyl, Cycloalkyl, Cycloalkenyl, Aryl, Heterocyclyl oder polycyclische Carbocyclen oder die Gruppierung -OR$^{IV}$ steht, oder |
| A und $R^2$ | gemeinsam mit den Atomen, an die sie gebunden sind, für einen gegebenenfalls substituierten Ring stehen, oder |
| B und $R^3$ | gemeinsam mit den Atomen, an die sie gebunden sind, für einen gegebenenfalls substituierten Ring stehen, oder |
| $R^3$ und $R^4$ | gemeinsam mit dem Stickstoffatom, an das sie gebunden sind, für einen gegebenenfalls substituierten Heterocyclus stehen, der weitere Heteroatome enthalten kann, |
| R | für substituiertes Alkyl steht mit folgenden Substituenten: Halogen, -COOR$^I$, -CONR$^{II}$R$^{III}$, -OR$^{IV}$, Acyl und gegebenenfalls substituiertes Heterocyclyl, ausgenommen Azolyl; R ferner für gegebenenfalls substituiertes Cycloalkenyl steht; |
| $R^I$ | für Wasserstoff oder gegebenenfalls substituiertes Alkyl steht mit folgenden Substituenten: Halogen, jeweils gegebenenfalls substituiertes Aryl, Cycloalkyl und Cycloalkenyl, |
| $R^{II}$ und $R^{III}$ | gleich oder verschieden sind und für Wasserstoff oder gegebenenfalls substituiertes Alkyl stehen mit folgenden Substituenten: Halogen, -COOR$^{IV}$, -CONR$^I$R$^{IV}$, jeweils gegebenenfalls substituiertes Aryl, Cycloalkyl und Cycloalkenyl; $R^{II}$ und $R^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann, |
| $R^{IV}$ | für Wasserstoff, Alkyl, Aralkyl oder Acyl steht und |
| n | für die Zahlen 0, 1 oder 2 steht, |

erhält, wenn man

a) 2-Cyan-2-oximino-essigsäureester der allgemeinen Formel (II),

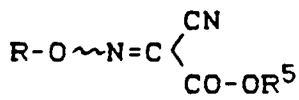 (II)

in welcher
R die oben angegebene Bedeutung hat und
$R^5$ für Alkyl steht,
mit Aminen der Formel (III),

$R^1$-$NH_2$ (III)

in welcher
$R^1$ die oben angegebene Bedeutung hat,
gegebenenfalls in Gegenwart eines Verdünnungsmittels und gegebenenfalls in Gegenwart einer Base umsetzt;
oder
b) carboxy-aktivierte Derivate von Carbonsäuren der allgemeinen Formel (IV),

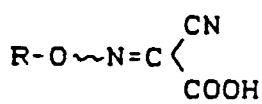 (IV)

in welcher
R die oben angegebene Bedeutung hat,
mit Aminen der Formel (III),

$R^1$-$NH_2$ (III)

in welcher
$R^1$ die oben angegebene Bedeutung hat,
gegebenenfalls in Gegenwart eines Katalysators und gegebenenfalls in Gegenwart eines Säurebindemittels sowie gegebenenfalls in Gegenwart eines Verdünnungsmittels umsetzt;
oder
c) 2-Cyan-2-oximino-acetamide der allgemeinen Formel (V),

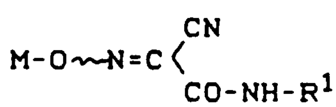 (V)

in welcher
M für Wasserstoff, ein Alkalimetallion, eine protonierte tertiäre Base oder ein quarternäres Ammoniumion steht und
$R^1$ die oben angegebene Bedeutung hat,
mit Verbindungen der Formel (VI),

R-X (VI)

in welcher
X für Halogen oder einen Sulfonyloxy-Rest steht und
R die oben angegebene Bedeutung hat,

gegebenenfalls in Gegenwart einer Base und gegebenenfalls in Gegenwart eines Verdünnungsmittels umsetzt;
oder
d) substituierte Acetyl-aminosäureester der allgemeinen Formel (VII),

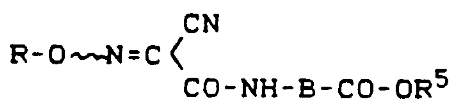 (VII)

in welcher
B, R und $R^5$ die oben angegebene Bedeutung haben,
mit Aminen der Formel (VIII),

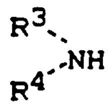 (VIII)

in welcher
$R^3$ und $R^4$ die oben angegebene Bedeutung haben,
gegebenenfalls in Gegenwart eines Verdünnungsmittels und gegebenenfalls in Gegenwart einer Base umsetzt;
oder
e) carboxy-akivierte Acetyl-aminosäuren der allgemeinen Formel (IX),

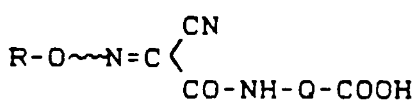 (IX)

in welcher
R die oben angegebene Bedeutung hat und
Q für A oder B steht, wobei diese die oben angegebene Bedeutung haben,
mit Verbindungen der Formel (X),

Y-H (X)

in welcher
Y für die Gruppierungen $R^2O$- oder $R^3R^4N$- steht, wobei $R^2$, $R^3$ und $R^4$ die oben angegebene Bedeutung haben,
gegebenenfalls in Gegenwart eines Katalysators und gegebenenfalls in Gegenwart eines Säurebindemittels sowie gegebenenfalls in Gegenwart eines Verdünnungsmittels umsetzt;
oder
f) Ammoniumsalze von 2-Cyan-2-oximino-essigsäuren der allgemeinen Formel (XI),

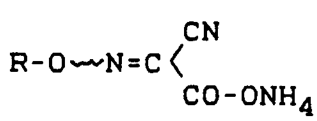 (XI)

in welcher
R die oben angegebene Bedeutung hat,

mit Aldehyden der Formel (XII),

$R^6$-CH = O (XII)

in welcher
die Gruppierung $R^6$-CH = für die oben genannten Bedeutungen von A oder B steht,
und mit Isonitrilen der Formel (XIII),

$$|\overset{\ominus}{C}\equiv\overset{\oplus}{N}-R^4 \qquad (XIII)$$

in welcher
$R^4$ die oben angegebene Bedeutung hat,
gegebenenfalls in Gegenwart eines Verdünnungsmittels umsetzt;
oder
g) 2-Cyan-2-oximino-essigsäuren der allgemeinen Formel (IV),

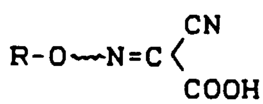
(IV)

in welcher
R die oben angegebene Bedeutung hat,
mit Isocyanaten der Formel (XIV),

$R^1$-NCO (XIV)

in welcher
$R^1$ die oben angegebene Bedeutung hat,
gegebenenfalls in Gegenwart eines Katalysators und gegebenenfalls in Gegenwart eines Verdünnungsmittels umsetzt;
oder
h) 2-Cyano-2-oximino-acetamide der allgemeinen Formel (XV),

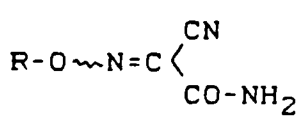
(XV)

in welcher
R die oben angegebene Bedeutung hat,
mit einer Base, gegebenenfalls in Gegenwart eines Verdünnungsmittels, umsetzt und die entstehenden Salze direkt oder gegebenenfalls nach Zwischenisolierung mit Verbindungen der Formel (XVI),

$R^1$-X (XVI)

in welcher
$R^1$ und X die oben angegebene Bedeutung haben,
gegebenenfalls in Gegenwart eines Verdünnungsmittels umsetzt.

Schließlich wurde gefunden, daß die neuen (2-Cyan-2-oximinoacetyl)-aminosäure-Derivate u.a. starke fungizide Eigenschaften aufweisen. Dabei zeigen überraschenderweise die erfindungsgemäßen Verbindungen eine höhere Wirkung als die aus dem Stand der Technik bekannten substituierten 2-Cyan-2-methoximino-acetamide, welche konstitutionell und/oder wirkungsmäßig naheliegende Verbindungen sind.

Die erfindungsgemäßen Stoffe stellen somit eine Bereicherung der Technik dar.

Im folgenden können alle aliphatischen Reste, wie Alkyl, Alkoxy usw., allein oder in Zusammensetzungen, wie Alkoxyalkyl, geradkettig oder verzweigt sein, ebenso sind alle Ringsysteme gegebenenfalls ein- bis fünffach, besonders bevorzugt ein- bis dreifach oder ganz besonders bevorzugt ein- oder zweifach, gleich oder verschieden substituiert, auch wenn es nicht ausdrücklich beschrieben ist.

Die erfindungsgemäßen (2-Cyan-2-oximinoacetyl)-aminosäure-Derivate sind durch die Formel (I) allgemein definiert. In dieser Formel (I) stehen vorzugsweise

$R^1$ für die Gruppierungen $-A-COOR^2$ oder $-B-CONR^3R^4$,

A für eine geradkettige oder verzweigte Alkylenkette mit 1 bis 4 Kohlenstoffatomen,

B für eine geradkettige oder verzweigte Alkylenkette mit 1 bis 4 Kohlenstoffatomen,

$R^2$ für gegebenenfalls substituiertes, geradkettiges oder verzweigtes Alkyl mit 1 bis 6 Kohlenstoffatomen, wobei als Substituenten vorzugsweise genannt seien: Halogen, gegebenenfalls ein- bis fünffach, gleich oder verschieden durch Halogen, Alkyl und Alkoxy mit jeweils 1 bis 4 Kohlenstoffatomen substituiertes Phenyl und Cycloalkyl mit 3 bis 6 Kohlenstoffatomen; ferner steht $R^2$ vorzugsweise für gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder für gegebenenfalls ein- bis fünffach, gleich oder verschieden durch Alkyl mit 1 bis 4 Kohlenstoffatomen substituiertes Cycloalkenyl mit 5 bis 7 Kohlenstoffatomen;

$R^3$ für Wasserstoff oder für gegebenenfalls ein- oder zweifach substituiertes, geradkettiges oder verzweigtes Alkyl mit 1 bis 4 Kohlenstoffatomen, wobei als Substituenten vorzugsweise genannt seien: Halogen, Cyano, $-COOR^{I}$, $-CONR^{II}R^{III}$, $-NR^{II}R^{III}$, $-OR^{IV}$, $-S(O)_nR^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes Phenyl, gegebenenfalls ein- bis fünffach, gleich oder verschieden durch Halogen sowie Alkyl und Alkoxy mit jeweils 1 bis 4 Kohlenstoffatomen substituierter 5- oder 6-gliedriger Heterocyclus mit 1 bis 3 Heteroatomen, insbesondere Stickstoff, Sauerstoff oder Schwefel, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen; $R^3$ steht ferner vorzugsweise für geradkettiges oder verzweigtes Alkenyl oder Alkinyl mit jeweils 2 bis 6 Kohlenstoffatomen; für gegebenenfalls ein- bis fünffach, gleich oder verschieden durch Alkyl mit 1 bis 4 Kohlenstoffatomen substituiertes Cycloalkenyl mit 5 bis 7 Kohlenstoffatomen oder für gegebenenfalls ein- bis fünffach, gleich oder verschieden substituiertes Cycloalkyl mit 3 bis 6 Kohlenstoffatomen, wobei als Substituenten vorzugsweise genannt seien: Halogen, Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im Alkoxyteil, Cycloalkyl und Cycloalkylalkyl mit 5 oder 6 Kohlenstoffatomen im Cycloalkylteil und 1 bis 4 Kohlenstoffatomen im geradkettigen oder verzweigten Alkylteil, gegebenenfalls ein- bis fünffach, gleich oder verschieden durch Halogen und Alkyl mit 1 bis 4 Kohlenstoffatomen substituiertes Phenyl und Pyrrolidon; $R^3$ steht weiterhin vorzugsweise für gegebenenfalls ein- bis fünffach, gleich oder verschieden durch Halogen, Alkyl und Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis fünffach, gleich oder verschieden substituierten 5- oder 6-gliedrigen Heterocyclus mit 1 bis 3 gleichen oder verschiedenen Heteroatomen, wie insbesondere Stickstoff-, Sauerstoff- und Schwefelatomen; als Substituenten seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil;

$R^4$ für die Bedeutungen von $R^3$, für polycyclische Carbocyclen oder die Gruppierung $-OR^{IV}$,

oder

A und $R^2$ gemeinsam mit den Atomen, an die sie gebunden sind, für einen Ring der Formel

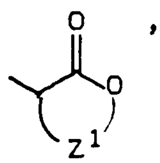

in welcher $Z^1$ für eine geradkettige oder verzweigte Alkylenkette mit 1 bis 4 Kohlenstoffatomen, steht,
oder

B und $R^3$ gemeinsam mit den Atomen, an die sie gebunden sind, für einen Ring der Formel

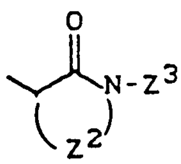

in welcher $Z^2$ für eine geradkettige oder verzweigte Alkylenkette mit 1 bis 4 Kohlenstoffatomen, und $Z^3$ für Wasserstoff oder Alkyl mit 1 bis 4 Kohlenstoffatomen steht,
oder

$R^3$ und $R^4$ gemeinsam mit dem Stickstoffatom, an das sie gebunden sind, für einen gegebenenfalls ein-bis fünffach, gleich oder verschieden substituierten mono-, bi- oder tricyclischen Heterocyclus oder Spiroheterocyclus mit ein bis drei weiteren, gleichen oder verschiedenen Heteroatomen, wie Sauerstoff-, Stickstoff- oder Schwefelatome, wobei als Substituenten genannt seien:
geradkettiges oder verzweigtes Alkyl oder Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl-oder Dialkylcarbamoyl mit jeweils 1 bis 4 Kohlenstoffatomen in jedem Alkylteil, sowie jeweils gegebenenfalls ein- oder zweifach, gleich oder verschieden durch Halogen oder geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

R für ein- oder zweifach substituiertes, geradkettiges oder verzweigtes Alkyl mit 1 bis 6 Kohlenstoffatomen, wobei als Substituenten vorzugsweise genannt seien: Halogen, -$COOR^I$, -$CONR^{II}R^{III}$, -$OR^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 Heteroatomen, wie insbesondere Stickstoff-, Sauerstoff- und Schwefelatomen, ausgenommen Azolyl; ferner steht R vorzugsweise für gegebenenfalls ein-bis fünffach, gleich oder verschieden durch Alkyl mit 1 bis 4 Kohlenstoffatomen substituiertes Cycloalkenyl mit 5 bis 7 Kohlenstoffatomen,

$R^I$ für Wasserstoff oder geradkettiges oder verzweigtes Alkyl mit 1 bis 6 Kohlenstoffatomen,

$R^{II}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil, wobei als Phenylsubstituenten genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen; $R^{II}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen,

$R^{III}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil, wobei als Phenylsubstituenten vorzugsweise die bei $R^{II}$ genannten Phenylsubstituenten infrage kommen; $R^{III}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen;

$R^{IV}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil, wobei als Phenylsubstituenten vorzugsweise die bei $R^{II}$ genannten Phenylsubstituenten infrage kommen; $R^{IV}$ steht ferner für Acyl mit 2 bis 9 Kohlenstoffatomen und

n für die Zahlen 0, 1 oder 2.

Besonders bevorzugt sind (2-Cyan-2-oximinoacetyl)-aminosäure-Derivate der allgemeinen Formel (I), bei denen

$R^1$ für die Gruppierungen $-A-COOR^2$ oder $-B-CONR^3R^4$ steht,

A für die Gruppierungen $-CH_2-$, $-CH(CH_3)-$ oder $-CH_2CH_2-$ steht,

B für die Gruppierungen $-CH_2-$, $-CH(CH_3)-$ oder $-CH_2CH_2-$ steht,

$R^2$ für geradkettiges oder verzweigtes Alkyl mit 1 bis 4 Kohlenstoffatomen steht,

$R^3$ für Wasserstoff oder für gegebenenfalls ein- oder zweifach, gleich oder verschieden substituiertes geradkettiges oder verzweigtes Alkyl mit 1 bis 4 Kohlenstoffatomen steht, wobei als Substituenten vorzugsweise genannt seien: Halogen, Cyano, $-COOR^I$, $-CONR^{II}R^{III}$, $NR^{II}R^{III}$, $-OR^{IV}$, $-S(O)_nR^{IV}$, Acyl mit 2 bis 9 Kohlenstoffatomen, gegebenenfalls ein- bis dreifach, gleich oder verschieden durch Halogen und Alkyl mit 1 bis 4 Kohlenstoffatomen substituiertes Phenyl, gegebenenfalls ein- bis dreifach, gleich oder verschieden durch Halogen, geradkettiges oder verzweigtes Alkyl und Alkoxy mit jeweils 1 bis 4 Kohlenstoffatomen substituierter 5-oder 6-gliedrigen Heterocyclus mit 1 bis 3 gleichen oder verschiedenen Heteroatomen, insbesondere Stickstoff, Sauerstoff und Schwefel, und jeweils gegebenenfalls durch geradkettiges oder verzweigtes Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen; $R^3$ steht ferner besonders bevorzugt für geradkettiges oder verzweigtes Alkenyl oder Alkinyl mit jeweils 2 bis 6 Kohlenstoffatomen, für gegebenenfalls durch geradkettiges oder verzweigtes Alkyl mit 1 bis 4 Kohlenstoffatomen substituiertes Cycloalkenyl mit 5 bis 7 Kohlenstoffatomen, für gegebenenfalls substituiertes Cycloalkyl mit 3 bis 6 Kohlenstoffatomen, wobei als Substituenten vorzugsweise genannt seien: Halogen, geradkettiges oder verzweigtes Alkyl und Alkoxy mit jeweils 1 bis 4 Kohlenstoffatomen, Cyano, Amino, Carbamoyl, jeweils geradkettiges oder verzweigtes Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im geradkettigen oder verzweigten Alkoxyteil, Cycloalkyl und Cycloalkylalkyl mit jeweils 5 oder 6 Kohlenstoffatomen im Cycloalkylteil und 1 bis 4 Kohlenstoffatomen im geradkettigen oder verzweigten Alkylteil, gegebenenfalls ein- bis dreifach, gleich oder verschieden durch Halogen oder Alkyl mit 1 bis 4 Kohlenstoffatomen substituiertes Phenyl und Pyrrolidon; $R^3$ steht weiterhin besonders bevorzugt für gegebenenfalls ein- bis dreifach, gleich oder verschieden durch Halogen, geradkettiges oder verzweigtes Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen substituiertes Phenyl oder für einen gegebenenfalls ein - bis dreifach, gleich oder verschieden substituierten 5- oder 6-gliedrigen Heterocyclus mit 1 bis 3 Heteroatomen, wie insbesondere Stickstoff, Sauerstoff oder Schwefel; als Substituenten für die Heterocyclen seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkyl;

$R^4$ für die Bedeutungen von $R^3$, für polycyclische Carbocyclen oder die Gruppierung $-OR^{IV}$ steht,

oder

A und $R^2$ gemeinsam mit den Atomen, an die sie gebunden sind, für gegebenenfalls durch Alkyl mit 1 bis 4, vorzugsweise 1 oder 2 Kohlenstoffatomen substituierte 5- oder 6-gliedrige Ringe der

Formeln 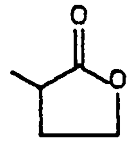 und 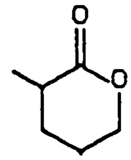 stehen,

oder

B und $R^3$ gemeinsam mit den Atomen, an die sie gebunden sind, für gegebenenfalls durch Alkyl mit 1 bis 4, vorzugsweise 1 oder 2 Kohlenstoffatomen substituierte 5- bis 7-gliedrige Ringe der Formeln

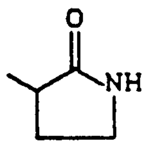 , 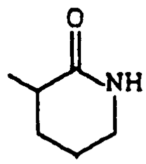 und 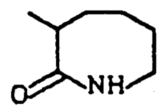 stehen,

oder

$R^3$ und $R^4$ gemeinsam mit dem Stickstoffatom, an das sie gebunden sind, für einen gegebenenfalls ein-bis fünffach, gleich oder verschieden substituierten mono-, bi- oder tricyclischen Heterocyclus oder Spiroheterocyclus stehen, wobei als Heterocyclen genannt seien: Oxazolidin, Pyrrolidin, Imidazolidin, Piperidin, Piperazin, Morpholin, Thiomorpholin, 1,3-Oxazan oder 1,3-Diazon; diese Heterocyclen können jeweils gegebenenfalls mit 1 oder 2 Benzol- oder Cyclohexanringen anelliert sein oder gegebenenfalls mit Methylen oder Ethylen überbrückt sein.
Als Substituenten für alle Heterosysteme seien genannt:
geradkettiges oder verzweigtes Alkyl oder Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl-oder Dialkylcarbamoyl mit jeweils 1 bis 4 Kohlenstoffatomen in jedem Alkylteil, sowie jeweils gegebenenfals ein- oder zweifach, gleich oder verschieden durch Halogen oder geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

R für substituiertes, geradkettiges oder verzweigtes Alkyl mit 1 bis 4 Kohlenstoffatomen steht, wobei als Substituenten vorzugsweise genannt seien: Halogen, $-COOR^{I}$, $-CONR^{II}R^{III}$, $-OR^{IV}$, Acyl mit 2 bis 9 Kohlenstoffatomen, gegebenenfalls ein- bis dreifach, gleich oder verschieden durch Alkyl mit 1 oder 2 Kohlenstoffatomen substituierte Heterocyclen der Formeln

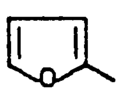 , 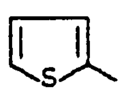 , 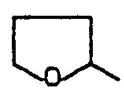 , 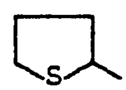 ,

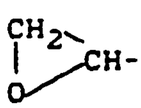 , 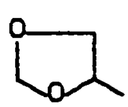 und 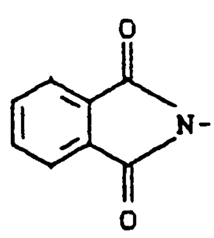 ,

R steht ferner für gegebenenfalls ein- bis dreifach durch Methyl substituiertes Cyclohexenyl oder Cyclopentenyl,

$R^I$ für geradkettiges oder verzweigtes Alkyl mit 1 bis 4 Kohlenstoffatomen steht,

$R^{II}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl und Phenethyl steht, wobei als Phenylsubstituenten jeweils genannt seien: Fluor, Chlor, Methyl, Methoxy und Trifluormethyl; $R^{II}$ ferner für Alkoxycarbonylalkyl mit 1 bis 4 Kohlenstoffatomen im Alkoxyteil und 1 oder 2 Kohlenstoffatomen im Alkylteil, Carbamoylalkyl mit 1 oder 2 Kohlenstoffatomen im Alkylteil, Alkylcarbamoylalkyl und Dialkylcarbamoylalkyl mit jeweils 1 oder 2 Kohlenstoffatomen in jedem Alkylteil, oder für gegebenenfalls ein - bis dreifach, gleich oder verschieden durch Methyl und Ethyl substituiertes Cycloalkyl mit 3 bis 6 Kohlenstoffatomen steht,

$R^{III}$ für die Bedeutungen von $R^{II}$ steht,

$R^{IV}$ für Wasserstoff, geradkettiges oder verzweigtes Alkyl mit 1 bis 4 Kohlenstoffatomen, jeweils gegebenenfalls ein - bis dreifach, gleich oder verschieden substituiertes Benzyl oder Phenethyl steht, wobei als Phenylsubstituenten jeweils genannt seien: Fluor, Chlor, Methyl, Methoxy und Trifluormethyl; $R^{IV}$ steht ferner für Acyl mit 2 bis 9 Kohlenstoffatomen und

n für die Zahlen 0, 1 oder 2 steht.

Ganz besonders bevorzugt sind (2-Cyan-2-oximinoacetyl)-aminosäure-Derivateder allgemeinen Formel (I), bei denen

(A)

$R^1$ für die Gruppierung $-B-CO-NR^3R^4$ steht,

B für die Gruppierungen $-CH_2-$, $-CH(CH_3)-$ oder $-CH_2CH_2-$ steht,

$R^3$ für Wasserstoff oder geradkettiges oder verzweigtes Alkyl mit 1 bis 4 Kohlenstoffatomen steht,

$R^4$ für Wasserstoff oder geradkettiges oder verzweigtes Alkyl mit 1 bis 4 Kohlenstoffatomen steht,

R für substituiertes Alkyl mit 1 oder 2 Kohlenstoffatomen steht, wobei als Substituenten genannt seien: Halogen, Alkylcarbonyl mit 1 bis 4 Kohlenstoffatomen im geradkettigen oder verzweigen Alkylteil, die Gruppierungen $-COOR^I$, $-CONR^{II}R^{III}$, $-OR^{IV}$ und die folgenden Heterocyclen:

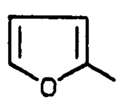 , 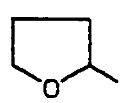 , 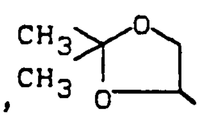 , 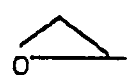 und

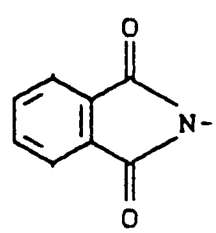 ;

R steht ferner ganz besonders bevorzugt für gegebenenfalls ein- bis dreifach durch Methyl substituiertes 1-Cyclohexenyl;

$R^I$ für Methyl, Ethyl, n- oder i-Propyl oder n-, i-, s-oder t-Butyl steht,

$R^{II}$ und $R^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor und Methyl als Substituenten, für Alkoxycarbonylalkyl mit 1 oder 2 Kohlenstoffatomen in jedem Alkylteil, für Carbamoylalkyl, Alkylcarbamoylalkyl oder Dialkylcarbamoylalkyl mit jeweils 1 oder 2 Kohlenstoffatomen in jedem Alkylteil oder für gegebenenfalls ein- bis dreifach durch Methyl substituiertes Cyclohexyl stehen und

$R^{IV}$ für Wasserstoff, Alkyl mit 1 oder 2 Kohlenstoffatomen oder gegebenenfalls ein- bis dreifach, gleich oder verschieden, substituiertes Benzyl steht, wobei als Phenylsubstituenten Fluor, Chlor und Methyl genannt seien;

oder

(B)

$R^1$ für die Gruppierung -B-CO-$NR^3R^4$ steht,

B für die Gruppierungen -$CH_2$-, -$CH(CH_3)$- oder -$CH_2CH_2$- steht,

$R^3$ für Wasserstoff, geradkettiges oder verzweigtes Alky mit 1 bis 4 Kohlenstoffatomen oder ein- oder zweifach substituiertes Alkyl mit 1 bis 3 Kohlenstoffatomen steht, wobei als Substituenten genannt seien: Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl; $R^3$ steht ferner ganz besonders bevorzugt für Allyl oder Propargyl, für 1-Cycloexenyl oder für ein- bis dreifach, gleich oder verschieden substituiertes Cyclohexyl, wobei als Substituenten genannt seien: Chlor, Methyl, Ethyl, Methoxy, Cyano, Amino, Alkyl-oder Dialkylamino mit jeweils 1 oder 2 Kohlenstoffatomen in jedem Alkylteil, Carbamoyl, Alkyl- oder Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil oder 1-Pyrrolidin-2-on;

$R^3$ steht außerdem ganz besonders bevorzugt für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein-bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadiazol-5-yl oder für die Gruppierung

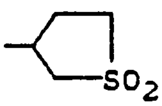 ,

$R^4$ für ein- oder zweifach substituiertes Alkyl mit 1 bis 3 Kohlenstoffatomen steht, wobei als Substituenten genannt seien: Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl; $R^4$ steht ferner ganz besonders bevorzugt für Allyl oder Propargyl, für 1-Cyclohexenyl oder für ein- bis dreifach, gleich oder verschieden substituiertes Cyclohexyl, wobei als Substituenten genannt seien: Chlor, Methyl, Ethyl, Methoxy, Cyano, Amino, Alkyl-oder Dialkylamino mit jeweils 1 oder 2 Kohlenstoffatomen in jedem Alkylteil, Carbamoyl, Alkyl- oder Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil und 1-Pyrrolidin-2-on;

$R^4$ steht außerdem ganz besonders bevorzugt für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein-bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadiazol-5-yl oder für die Gruppierung

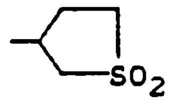 ,

$R^4$ steht weiterhin ganz besonders bevorzugt für Hydroxy, Alkoxy mit 1 oder 2 Kohlenstoffatomen, für gegebenenfalls ein- bis dreifach, gleich oder verschieden durch Chlor und Methyl substituiertes Benzyloxy sowie für 1-Adamantyl, 2-Norbornyl, 1- oder 2-Decalyl oder 1- oder 2-Tetralyl;

R für substituiertes Alkyl mit 1 oder 2 Kohlenstoffatomen steht, wobei als Substituenten genannt seien: Halogen, Alkylcarbonyl mit 1 bis 4 Kohlenstoffatomen im Alkylteil, die Gruppierungen -COOR$^I$, -CONR$^{II}$R$^{III}$, OR$^{IV}$ und die folgenden Heterocyclen:

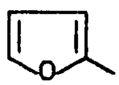 , 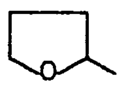 , 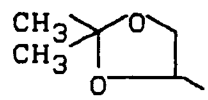 , 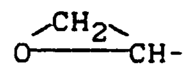 und

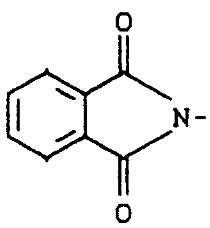 ;

R steht ferner ganz besonders bevorzugt für gegebenenfalls ein- bis dreifach durch Methyl substituiertes 1-Cyclohexenyl,

R$^I$ für Methyl, Ethyl, n- oder i-Propyl oder n-, i- s-oder t-Butyl steht,

R$^{II}$ und R$^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor oder Methyl als Substituenten, für Alkoxycarbonylalkyl mit 1 oder 2 Kohlenstoffatomen in jedem Alkylteil, für Carbamoylalkyl, Alkylcarbamoylalkyl oder Dialkylcarbamoylalkyl mit jeweils 1 oder 2 Kohlenstoffatomen in jedem Alkylteil oder für gegebenenfalls ein- bis dreifach durch Methyl substituiertes Cyclohexyl stehen und

R$^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien,

oder

(C)

$R^1$ für die Grupierung -B-CO-NR$^3$R$^3$ steht,

B für die Gruppierungen, $-CH_2-$, $-CH(CH_3)-$ oder $-CH_2CH_2-$ steht,

$R^3$ und $R^4$ gemeinsam mit dem Stickstoffatom, an das sie gebunden sind, für folgende mono-, bi- oder tricyclischen Heterocyclen oder Spiroheterocyclen stehen:

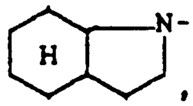 , 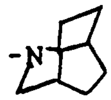 , 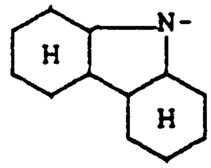 ,

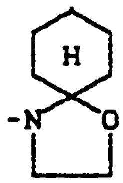 , 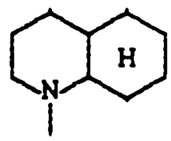 ,

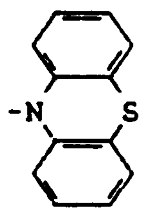 , 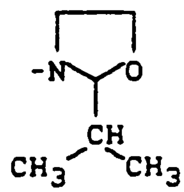 , 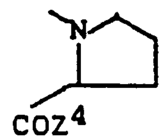

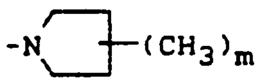 , 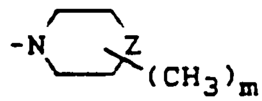

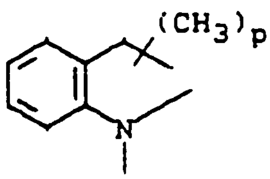 , 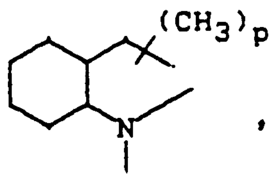 ,

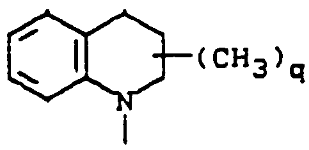 , 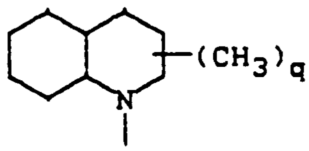 ,

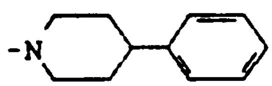 , 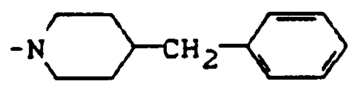 ,

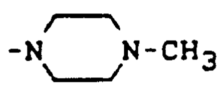 , 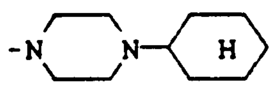 , 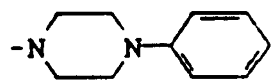 ,

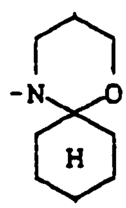 , 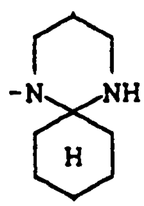 , 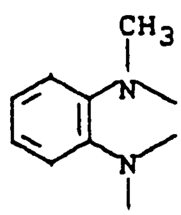 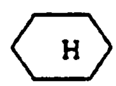 ,

wobei

| | |
|---|---|
| Z | für Sauerstoff oder die $CH_2$-Gruppe steht, |
| $Z^4$ | für Hydroxy, Methoxy, Ethoxy, Amino, Methylamino, Ethylamino, Dimethylamino oder Ethylmethylamino steht, |
| m | für die Zahlen 0, 1, 2, 3 oder 4 steht, |
| p | für die Zahlen 0, 1 oder 2 steht, und |
| q | für die Zahlen 0, 1, 2 oder 3 steht und |
| R | für substituiertes Alkyl mit 1 oder 2 Kohlenstoffatomen steht, wobei als Substituenten genannt seien: Halogen, Alkylcarbonyl mit 1 bis 4 Kohlenstoffatomen im geradkettigen oder verzweigten Alkylteil, $-COOR^{I}$, $-CONR^{II}R^{III}$, $OR^{IV}$ und die folgenden Heterocyclen: |

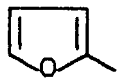 , 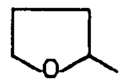 , 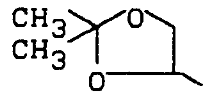 , 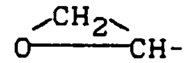 und

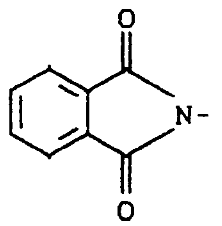 ;

R steht ferner ganz besonders bevorzugt für gegebenenfalls ein- bis dreifach durch Methyl substituiertes 1-Cyclohexenyl

R$^{I}$ für Methyl, Ethyl, n- oder i-Propyl oder n-, i-, s-oder t-Butyl steht,

R$^{II}$ und R$^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor und Methyl als Substituenten, für Alkoxycarbonylalkyl mit 1 oder 2 Kohlenstoffatomen in jedem Alkylteil, für Carbamoylalkyl, Alkylcarbamoylalkyl oder Dialkylcarbamoylalkyl mit jeweils 1 oder 2 Kohlenstoffatomen in jedem Alkylteil oder für gegebenenfalls ein- bis dreifach durch Methyl substituiertes Cyclohexyl stehen und

R$^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien,

oder

(D)

R$^{1}$ für die Gruppierung -A-COOR$^{2}$ steht,

A für die Gruppierungen $-CH_2-$, $-CH(CH_3)-$ oder $-CH_2CH_2-$ steht,

R$^{2}$ für Methyl oder Ethyl steht und

R für ein- oder zweifach substituiertes Alkyl mit 1 oder 2 Kohlenstoffatomen steht, wobei als Substituenten genannt seien: Halogen, Alkylcarbonyl mit 1 bis 4 Kohlenstoffatomen im geradkettigen oder verzweigten Alkylteil, -COOR$^{I}$, -CONR$^{II}$R$^{III}$, OR$^{IV}$ und die folgenden Heterocyclen:

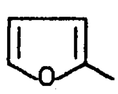 , 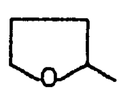 , 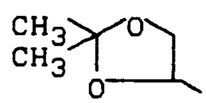 , 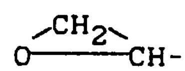 und

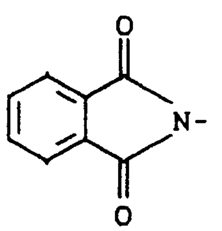 ;

R steht ferner ganz besonders bevorzugt für gegebenenfalls ein- bis dreifach durch Methyl substituiertes 1-Cyclohexenyl,

R$^{I}$ für Methyl, Ethyl, n- oder i-Propyl, n-, i-, s-oder t-Butyl steht,

R$^{II}$ und R$^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor und Methyl als Substituenten, für Alkoxycarbonylalkyl mit 1 oder 2

Kohlenstoffatomen in jedem Alkylteil, für Carbamoylalkyl, Alkylcarbamoylalkyl oder Dialkylcarbamoylalkyl mit jeweils 1 oder 2 Kohlenstoffatomen in jedem Alkylteil oder für gegebenenfalls ein- bis dreifach durch Methyl substituiertes Cyclohexyl stehen, und

$R^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien.

Verwendet man beispielsweise (E)-2-Cyan-2-(tert.-butyloxycarbonylmethoximino)-essigsäureethylester und $N^{\alpha}$-Glycyl-prolinamid-hydrobromid als Ausgangsstoffe, so kann der Ablauf des erfindungsgemäßen Verfahrens (a) durch das folgende Formelschema wiedergegeben werden:

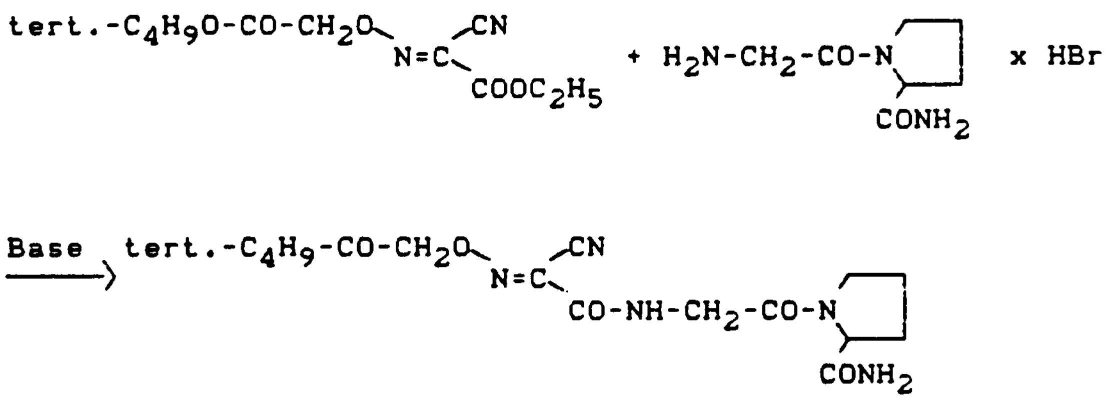

Verwendet man beispielsweise (E)-2-Cyan-2-(2-furylmethoximino)-acetylchlorid und N-Glycyl-aminomethylcyclo-hexan-hydrobromid als Ausgangsstoffe, so kann der Ablauf des erfindungsgemäßen Verfahrens (b) durch das folgende Formelschema wiedergegeben werden:

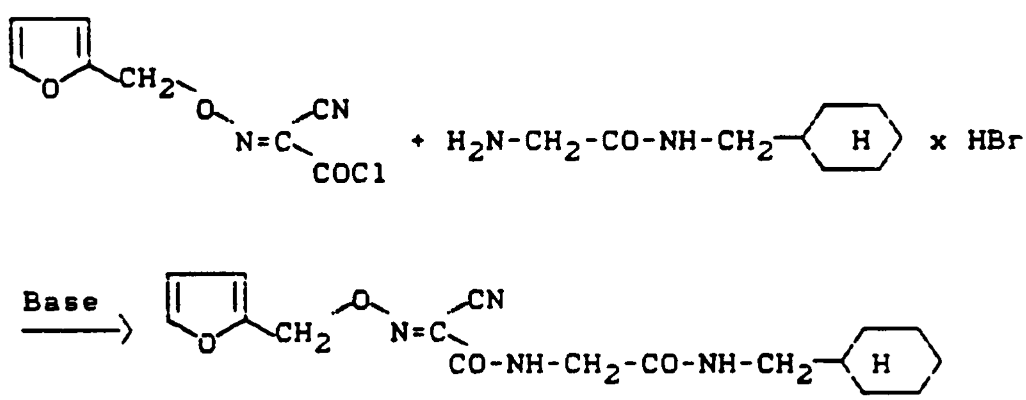

Verwendet man beispielsweise das Natriumsalz von N-(2-Cyan-2-hydroximino-acetyl)-glycinethylester und Chloressigsäure-tert.-butylester als Ausgangsstoffe, so kann der Ablauf des erfindungsgemäßen Verfahrens (c) durch das folgende Formelschema wiedergegeben werden:

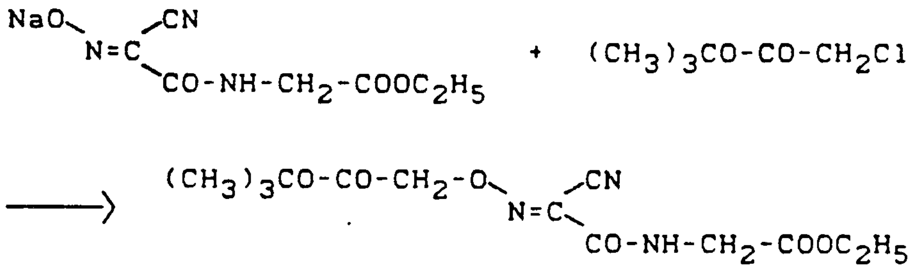

Verwendet man beispielsweise N-(2-Cyan-2-methyloxy-methoximino-acetyl)-glycinethylester und Ethanolamin als Ausgangsstoffe, so kann der Ablauf des erfindungsgemäßen Verfahrens (d) durch das folgende Formelschema wiedergegeben werden:

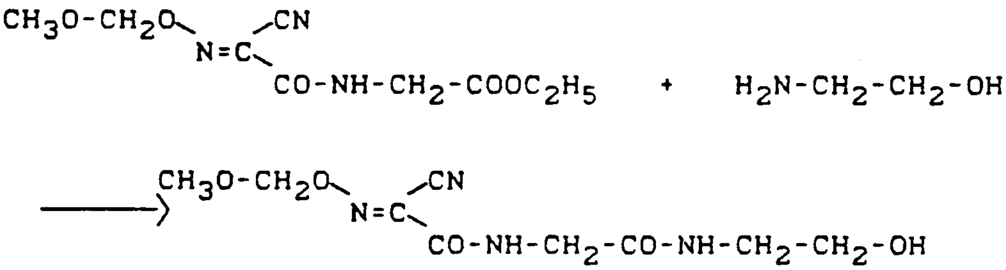

Verwendet man beispielsweise N-(2-Cyan-2-methyloxy-methoximinoacetyl)-glycin und 4-Ethoxycyclohexylamin als Ausgangsstoffe sowie Dicyclohexylcarbodiimid und N-Hydroxysuccinimid als Hilfsstoffe, so kann der Ablauf des erfindungsgemäßen Verfahrens (e) durch das folgende Formelschema wiedergegeben werden:

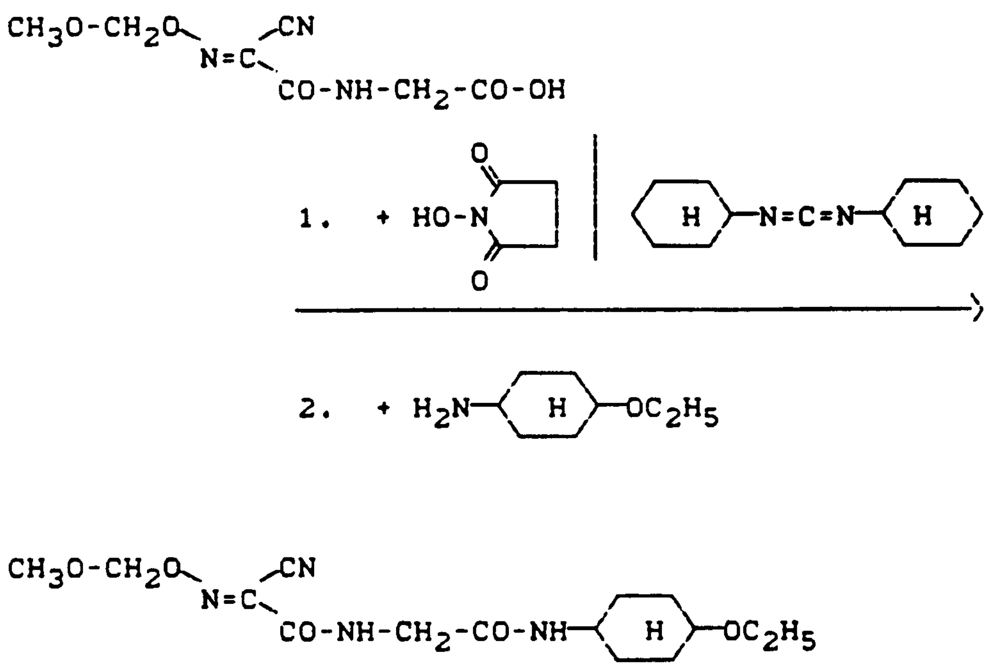

Verwendet man beispielsweise das Ammoniumsalz der 2-Cyan-2-(tert.-butyloxycarbonylmethoximino)-essigsäure, Isobutyraldehyd und Cyclohexylisonitril als Ausgangsstoffe, so kann der Ablauf des erfindungsgemäßen Verfahrens (f) durch das folgende Formelschema wiedergegeben werden:

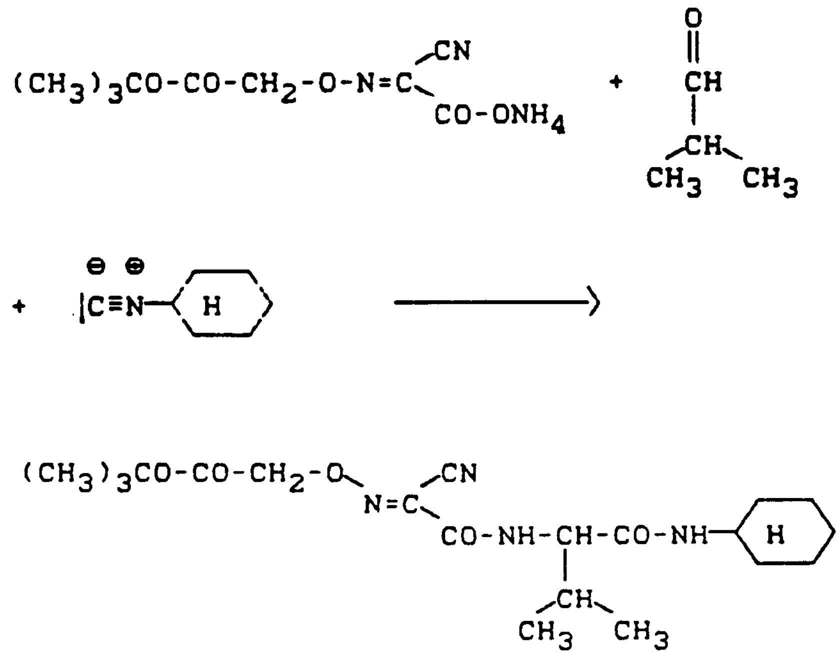

Verwendet man beispielsweise 2-Cyan-2-(tert.-butoxycarbonylmethoximino)-essigsäure und Ethoxycarbonyl-methylisocyanat als Ausgangsstoffe, so kann der Ablauf des erfindungsgemäßen Verfahrens (g) durch das folgende Formelschema wiedergegeben werden:

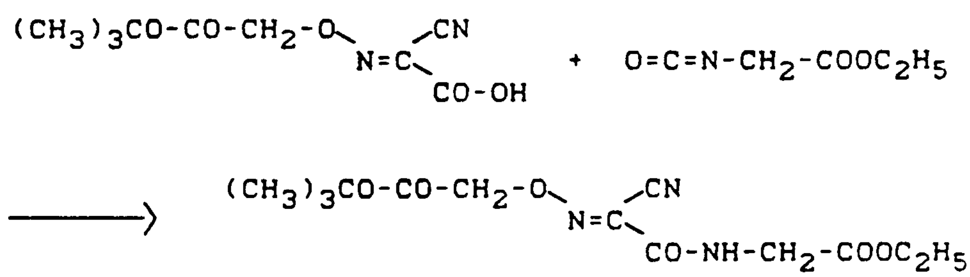

Verwendet man beispielsweise 2-Cyan-2-(tert.-butoxycarbonylmethoximino)-acetamid und Bromessigsäurediethylamid als Ausgangsstoffe, so kann der Ablauf des erfindungsgemäßen Verfahrens (h) durch das folgende Formelschema wiedergegeben werden:

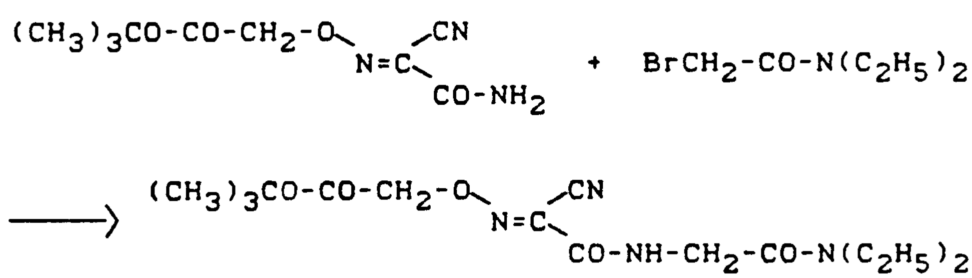

Die für die Durchführung des erfindungsgemäßen Verfahrens (a) als Ausgangsstoffe zu verwendenden 2-Cyan-2-oximino-essigsäureester sind durch die Formel (II) allgemein definiert. In dieser Formel hat R vorzugsweise die Bedeutung, die bereits im Zusammenhang mit der Beschreibung der erfindungsgemäßen Stoffe der Formel (I) für diesen Substituenten vorzugsweise genannt wurde. $R^5$ steht vorzugsweise für Methyl oder Ethyl.

Die 2-Cyan-2-oximino-essigsäureester der Formel (II) sind nicht bekannt. Sie können jedoch in bekannter Art und Weise hergestellt werden (vgl. hierzu z.B. J. Antibiot. 37, 557 (1984); Pesticide Science 12, 27 (1981) und EP-OS 0 201 999; sowie die deutschen Patentanmeldungen P 3 625 460 vom 28.07.86 und P 3 625 497 vom 28.07.1986).

Die außerdem für die Durchführung des erfindungsgemäßen Verfahrens (a) und ebenfalls des Verfahrens (b) als Ausgangsstoffe zu verwendenden Amine sind durch die Formel (III) allgemein definiert. In dieser Formel hat $R^1$ vorzugsweise die Bedeutung, die bereits im Zusammenhang mit der Beschreibung der erfindungsgemäßen Stoffe der Formel (I) für diesen Substituenten vorzugsweise genannt wurde.

Die Amine der Formel (III) sind bekannt (vgl. z.B. Houben-Weyl, Methoden der organischen Chemie, Band XV, Teil 1 und 2, Georg Thieme Verlag, Stuttgart 1974; bzw. R. C. Sheppard, A Specialist Periodical Report, Aminoacids, Peptides and Proteins, The Royal Society of Chemistry, Burlington House, London 1978, bzw. I.P. Greenstein and M. Winitz, Chemistry of Amino Acids, I. Wiley Sons Inc., New York, London 1961; bzw. E. Schröder and K. Lübke, The Peptides Vol. I, Academic Press, New York, London 1965 sowie EP-OS 0 201 999); bzw. sind sie Gegenstand einer eigenen, noch nicht veröffentlichten Patentanmeldung (vgl. die deutsche Patentanmeldung P 3 625 497 vom 28.07.1986); bzw. können sie nach den dort angegebenen Verfahren in allgemein bekannter Art und Weise erhalten werden.

Die für die Durchführung des erfindungsgemäßen Verfahrens (b) bzw. des erfindungsgemäßen Verfahrens (g) als Ausgangsstoffe zu verwendenden Carbonsäuren sind durch die Formel (IV) allgemein definiert. In dieser Formel hat R vorzugsweise die Bedeutung, die bereits im Zusammenhang mit der Beschreibung der erfindungsgemäßen Stoffe der Formel (I) vorzugsweise für diesen Substituenten genannt wurden.

Als carboxy-aktivierte Derivate der Carbonsäuren der Formel (IV) kommen alle carboxyaktivierten Derivate infrage, wie Säurehalogenide, wie z.B. Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxisuccinimidester sowie mit Kondensationsmitteln, wie z.B. Dicyclohexylcarbodiimid oder Carbonyldiimidazol, in situ erzeugte aktivierte Formen der Carbonsäuren.

Vorzugsweise werden die den Carbonsäuren der Formel (IV) entsprechenden Säurechloride eingesetzt. Sie können hergestellt werden, indem man die Carbonsäuren der Formel (IV) oder deren Salze mit einem Halogenierungsmittel, wie beispielsweise Phosphorpentachlorid, Thionylchlorid oder Oxalylchlorid, in allgemein bekannter Art und Weise umsetzt. Bevorzugt ist der Einsatz von Oxalylchlorid zusammen mit dem Natrium- oder Kaliumsalz der Carbonsäure der Formel (IV).

Die Carbonsäuren der Formel (IV) und deren carboxyaktivierten Derivate sind noch nicht bekannt. Sie können jedoch in bekannter Art und Weise hergestellt werden (vgl. hierzu z.B. Pesticide Science 12, 27 (1981) und DE-OS 3 521 131 sowie die deutschen Patentanmeldungen P 3 602 243 vom 25.01.1986 und P 3 625 497 vom 28.07.86).

Die für die Durchführung des erfindungsgemäßen Verfahrens (c) als Ausgangsstoffe zu verwendenden 2-Cyan-2-oximino-acetamide sind durch die Formel (V) allgemein definiert. In dieser Formel hat $R^1$ vorzugsweise die Bedeutung, die bereits im Zusammenhang mit der Beschreibung der erfindungsgemäßen Stoffe der Formel (I) vorzugsweise für diesen Substituenten genannt wurde. M steht vorzugsweise für Wasserstoff oder für ein Kalium-bzw. Natriumäquivalent.

Die 2-Cyan-2-oximino-acetamide der Formel (V) sind noch nicht bekannt. Sie sind teilweise Gegenstand einer eigenen, noch nicht veröffentlichten Patentanmeldung (vgl. die deutsche Patentanmeldung P 3 702 282 vom 27.01.1987) bzw. können sie nach den dort angegebenen Verfahren erhalten werden, indem man beispielsweise Cyanessigester der Formel (XVII),

$NC-CH_2-CO-OR^7$ (XVII)

in welcher
$R^7$ für Methyl oder Ethyl steht,
mit Aminosäurederivaten der Formel (III),

$H_2N-R^1$ (III)

in welcher
$R^1$ die oben angegebene Bedeutung hat,
gegebenenfalls in Gegenwart eines Lösungsmittels, wie beispielsweise Methanol, Ethanol, Diethylether, 1,2-Dimethoxyethan, Dimethylformamid oder deren Mischungen mit Wasser bei 0 °C bis 60 °C, vorzugsweise bei Raumtemperatur umsetzt und anschließend die so erhaltenen Verbindungen der Formel (XVIII),

$NC-CH_2-CO-NH-R^1$ (XVIII)

in welcher

$R^1$ die oben angegebene Bedeutung hat,

mit salpetriger Säure oder Derivaten der salpetrigen Säure, gegebenenfalls in Gegenwart eines Verdünnungsmittels, wie beispielsweise Alkohole, und gegebenenfalls in Gegenwart einer Säure oder einer Base, wie beispielsweise Chlorwasserstoff oder Alkalimetallalkoholate, bei Temperaturen zwischen - 20 °C und 120 °C umsetzt.

Die Verbindungen der Formeln (XVII) und (III) sind bekannt; bzw. können sie in allgemein bekannter Art und Weise erhalten werden.

Die außerdem für die Durchführung des erfindungsgemäßen Verfahrens (c) als Ausgangsstoffe zu verwendenden Verbindungen sind durch die Formel (VI) allgemein definiert. In dieser Formel hat R vorzugsweise die Bedeutung, die bereits im Zusammenhang mit der Beschreibung der erfindungsgemäßen Stoffe der Formel (I) vorzugsweise für diesen Substituenten genannt wurde. X steht vorzugsweise für Chlor, Brom, Methylsulfonyloxy oder p-Toluolsulfonyloxy.

Die Verbindungen der Formel (VI) sind allgemein bekannte Verbindungen der organischen Chemie; bzw. können sie in allgemein bekannter Art und Weise erhalten werden.

Die für die Durchführung des erfindungsgemäßen Verfahrens (d) als Ausgangsstoffe zu verwendenden substituierten Acetyl-aminosäureester sind durch die Formel (VII) allgemein definiert. In dieser Formel haben B und R vorzugsweise die Bedeutungen, die bereits im Zusammenhang mit der Beschreibung der erfindungsgemäßen Stoffe der Formel (I) für diese Substituenten vorzugsweise genannt wurden. $R^5$ steht vorzugsweise für Methyl oder Ethyl.

Die substituierten Acetyl-aminosäureester der allgemeinen Formel (VII) sind teilweise erfindungsgemäße Verbindungen bzw. können sie in bekannter Art und Weise hergestellt werden (vgl. hierzu EP-OS 0 201 999 und DE-OS 3 521 131 sowie die deutschen Patentanmeldungen P 3 602 243 vom 25.0.1.1986 und P 3 625 497 vom 28.07.1986).

Die außerdem für die Durchführung des erfindungsgemäßen Verfahrens (d) als Ausgangsstoffe zu verwendenden Amine sind durch die Formel (VIII) allgemein definiert. In dieser Formel haben $R^3$ und $R^4$ vorzugsweise die Bedeutungen, die bereits im Zusammenhang mit der Beschreibung der erfindungsgemäßen Stoffe der Formel (I) für diese Substituenten vorzugsweise genannt wurden.

Die Amine der Formel (VIII) sind allgemein bekannte Verbindungen der organischen Chemie, bzw. können sie in allgemein bekannter Art und Weise erhalten werden.

Die für die Durchführung des erfindungsgemäßen Verfahrens (e) als Ausgangsstoffe zu verwendenden Acetyl-aminosäuren sind durch die Formel (IX) allgemein definiert. In dieser Formel hat R vorzugsweise die Bedeutung, die bereits im Zusammenhang mit der Beschreibung der erfindungsgemäßen Stoffe der Formel (I) vorzugsweise für diesen Substituenten genannt wurde. Q steht vorzugsweise für die bereits im Zusammenhang mit der Beschreibung der erfindungsgemäßen Stoffe der Formel (I) vorzugsweise für A und B genannten Bedeutungen.

Als carboxy-aktivierte Derivate kommen vorzugsweise die bei den Carbonsäuren der Formel (IV) bereits vorzugsweise genannten Derivate infrage.

Die Acetyl-aminosäuren der allgemeinen Formel (IX) sind teilweise erfindungsgemäße Verbindungen bzw. können sie in bekannter Art und Weise hergestellt werden (vgl. hierzu EP-OS 0 201 999 und DE-OS 3 521 131 sowie die deutschen Patentanmeldungen P 3 602 243 vom 25.01.1986 und P 3 625 497 vom 28.07.1986).

Die außerdem für die Durchführung des erfindungsgemäßen Verfahrens (e) als Ausgangsstoffe zu verwendenden Verbindungen sind durch die Formel (X) allgemein definiert.

In dieser Formel steht Y vorzugsweise für die Gruppierungen $R^2O$- und $R^3R^4N$-, wobei $R^2$, $R^3$ und $R^4$ vorzugsweise die Bedeutungen haben, die bereits im Zusammenhang mit der Beschreibung der erfindungsgemäßen Stoffe der Formel (I) für diese Substituenten vorzugsweise genannt wurden.

Die Verbindungen der Formel (X) sind allgemein bekannte Verbindungen der organischen Chemie, bzw. können sie in allgemein bekannter Art und Weise erhalten werden.

Die für die Durchführung des erfindungsgemäßen Verfahrens (f) als Ausgangsstoffe zu verwendenden Ammoniumsalze von 2-Cyan-2-oximino-essigsäuren sind durch die Formel (XI) allgemein definiert. In dieser Formel hat R vorzugsweise die Bedeutung, die bereits im Zusammenhang mit der Beschreibung der erfindungsgemäßen Stoffe der Formel (I) vorzugsweise für diesen Substituenten genannt wurde.

Die Ammoniumsalze von 2-Cyan-2-oximino-essigsäuren werden erhalten, indem man die Carbonsäuren der Formel (IV) in üblicher Weise mit Ammoniak umsetzt.

Die außerdem für die Durchführung des erfindungsgemäßen Verfahrens (f) als Ausgangsstoffe zu verwendenden Aldehyde sind durch die Formel (XII) und die Isonitrile durch die Formel (XIII) allgemein definiert. In der Formel (XII) steht die Gruppierung $R^6$-CH= für die Bedeutungen von A und B, wobei diese vorzugsweise die Bedeutungen haben, die bereits im Zusammenhang mit der Beschreibung der erfindungsgemäßen Stoffe der Formel (I) für diese Substituenten vorzugsweise genannt wurden. In der Formel (XIII) hat $R^4$ vorzugsweise die Bedeutung, die bereits im Zusammenhang mit der Beschreibung der erfindungsgemäßen Stoffe der Formel (I) vorzugsweise für diesen Substituenten genannt wurde.

Die Aldehyde der Formel (XII) und die Isonitrile der Formel (XIII) sind allgemein bekannte Verbindungen der organischen Chemie, bzw. können sie in allgemein bekannter Art und Weise erhalten werden.

Die für die Durchführung des erfindungsgemäßen Verfahrens (g) als Ausgangsstoffe zu verwendenden Isocyanate sind durch die Formel (XIV) allgemein definiert. In dieser Formel hat $R^1$ vorzugsweise die Bedeutung, die bereits im Zusammenhang mit der Beschreibung der erfindungsgemäßen Stoffe der Formel (I) vorzugsweise für diesen Substituenten genannt wurde.

Die Isocyanate der Formel (XIV) sind allgemein bekannte Verbindungen der organischen Chemie, bzw. können sie in allgemein bekannter Art und Weise erhalten werden.

Die für die Durchführung des erfindungsgemäßen Verfahrens (h) als Ausgangsstoffe zu verwendenden 2-Cyano-2-oximino-acetamide sind durch die Formel (XV) allgemein definiert. In dieser Formel hat R vorzugsweise die Bedeutung, die bereits im Zusammenhang mit der Beschreibung der erfindungsgemäßen Stoffe der Formel (I) vorzugsweise für diesen Substituenten genannt wurde.

Die 2-Cyano-2-oximino-acetamide der Formel (XV) sind noch nicht bekannt. Sie können jedoch in bekannter Art und Weise hergestellt werden (vgl. hierzu z.B. Chem. Ber. 54, 1342 (1921), DE-OS 2 623 847 und DE-OS 2 657 145) sowie die deutsche Patentanmeldung P 3 702 283 vom 27.01.1987).

Zu den weiterhin für die Durchführung des erfindungsgemäßen Verfahrens (h) als Ausgangsstoffe zu verwendenden Basen gehören alle üblichen anorganischen und organischen Basen, wie insbesondere Alkalimetallalkoholate, wie beispielsweise Kalium-tert.-butylat; Alkalimetallhydroxide und -carbonate, wie beispielsweise Natriumhydroxid, Kaliumhydroxid und Kaliumcarbonat sowie Tetraalkylammonium- bzw. Benzyltrialkylammonium-hydroxide und -alkoholate, wie beispielsweise Tetramethylammonium-hydroxid und -alkoholat, Tetrabutylammoniumhydroxid und -alkoholat oder Benzyltriethylammoniumhydroxid und -alkoholat.

Die Basen sind allgemein bekannte Verbindungen der organischen Chemie.

Die außerdem für die Durchführung des erfindungsgemäßen Verfahrens (h) als Ausgangsstoffe zu verwendenden Verbindungen sind durch die Formel (XVI) allgemein definiert. In dieser Formel hat $R^1$ vorzugsweise die Bedeutung, die bereits im Zusammenhang mit der Beschreibung der erfindungsgemäßen Stoffe der Formel (I) vorzugsweise für diesen Substituenten genannt wurde. X steht vorzugsweise für Chlor, Brom, Methylsulfonyloxy oder p-Toluolsulfonyloxy.

Die Verbindungen der Formel (XVI) sind allgemein bekannte Verbindungen der organischen Chemie; bzw. können sie in allgemein bekannter Art und Weise erhalten werden.

Als Verdünnungsmittel kommen für die erfindungsgemäßen Verfahren (a) und (d) Wasser sowie organische Lösungsmittel infrage. Hierzu gehören vorzugsweise Alkohole, wie Methanol, Ethanol oder Isopropanol; Ether, wie Tetrahydrofuran oder 1,2-Dimethoxyethan; Amide, wie Dimethylformamid; Nitrile, wie Acetonitril sowie tertiäre Amine, wie Pyridin.

Die erfindungsgemäßen Verfahren (a) und (d) werden gegebenenfalls in Gegenwart einer Base durchgeführt, je nachdem ob die jeweiligen Amine in Form von Säureadditionssalzen eingesetzt werden. Hierbei kommen übliche organische und anorganische Basen infrage. Vorzugsweise genannt seien tert. Amine, wie Triethylamin; Alkoholate, wie Natriummethylat sowie Alkalicarbonate, wie Kaliumcarbonat.

Die Temperaturen können bei der Durchführung der erfindungsgemäßen Verfahren (a) und (d) in einem größeren Bereich variiert werden. Im allgemeinen abeitet man zwischen - 20 °C und der Rückflußtemperatur, vorzugsweise bei Raumtemperatur.

Bei der Durchführung der erfindungsgemäßen Verfahren (a) und (d) können alle Reaktionspartner im Überschuß eingesetzt werden; vorzugsweise arbeitet man mit äquimolaren Mengen. Die Reaktionsdurchführung, Aufarbeitung und Isolierung der Reaktionsprodukte der Formel (I) erfolgt in allgemein üblicher Weise.

Als Verdünnungsmittel kommen für die erfindungsgemäßen Verfahren (b) und (e) inerte organische Lösungsmittel infrage. Hierzu gehören Ketone, wie Aceton oder Ethylmethylketon; Ester, wie Ethyl- oder Methylacetat; Amide, wie Dimethylformamid; Nitrile, wie Acetonitril; Chlorkohlenwasserstoffe, wie Methylenchlorid oder Tetrachlorkohlenstoff; Kohlenwasserstoffe, wie Toluol; oder Ether, wie Tetrahydrofuran; bzw. deren Mischungen.

Als Säurebindemittel kommen für die erfindungsgemäßen Verfahren (b) und (e) übliche anorganische und organische Säurebinder infrage. Hierzu gehören vorzugsweise tertiäre Amine, wie Triethylamin, Pyridin

oder N-Methylmorpholin sowie anorganische Basen, wie Natriumcarbonat oder Calciumcarbonat.

Die erfindungsgemäßen Verfahren (b) und (e) werden gegebenenfalls in Gegenwart eines Katalysators durchgeführt. Beispielsweise genannt seien 4-Dimethylaminopyridin, 1-Hydroxy-benzotriazol oder Dimethylformamid.

Die Temperaturen können bei der Durchführung der Verfahren (b) und (e) in einem größeren Bereich variiert werden. Im allgemeinen arbeitet man zwischen - 60 °C bis + 120 °C, vorzugsweise bei - 20 °C bis + 40 °C.

Bei der Durchführung des erfindungsgemäßen Verfahrens (b) und (e) arbeitet man vorzugsweise in äquimolaren Mengen. Die Reaktionsdurchführung, Aufarbeitung und Isolierung der Reaktionsprodukte der Formel (I) erfolgt in allgemein üblicher Weise.

Als Verdünnungsmittel kommen für das erfindungsgemäße Verfahren (c) organische Lösungsmittel infrage. Hierzu gehören Alkohole, wie Methanol und Ethanol; Ketone, wie Aceton und Methylisobutylketon; Nitrile, wie Acetonitril; Ester, wie Essigester; Ether, wie Tetrahydrofuran; Amide, wie Dimethylformamid und N-Methylpyrrolidon sowie Dimethylsulfoxid.

Als Basen kommen für das erfindungsgemäße Verfahren (c) alle üblichen organischen und anorganischen Basen infrage. Hierzu gehören vorzugsweise tertiäre Amine, wie Triethylamin, Pyridin und 1,8-Diazabicyclo[5,4,0-undec-7-en (DBU), sowie anorganische Basen, wie Natrium-, Kalium- oder Calciumcarbonat.

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 150 °C, vorzugsweise bei Temperaturen zwischen 20 °C und 120 °C.

Bei der Durchführung des erfindungsgemäßen Verfahrens (c) setzt man pro Mol 2-Cyan-2-oximino-acetamid der Formel (V) im allgemeinen 1 bis 3 Mol, vorzugsweise 1 bis 2 Mol der Verbindung der Formel (VI) ein. Die Reaktionsdurchführung, Aufarbeitung und Isolierung der Reaktionsprodukte der Formel (I) erfolgt in allgemein üblicher Weise. Hervorzuheben ist, daß die 2-Cyan-2-oximino-acetamide der Formel (V) nach ihrer Herstellung ohne Isolierung direkt weiter umgesetzt werden können.

Als Verdünnungsmittel kommen für das erfindungsgemäße Verfahren (f) alle üblichen organischen Lösungsmittel infrage. Hierzu gehören vorzugsweise Alkohole, wie Methanol oder Ethanol; Ether, wie Diethylether; chlorierte Kohlenwasserstoffe, wie Chloroform oder Tetrachlorkohlenstoff; sowie Ketone, wie Aceton.

Die Temperaturen können bei der Durchführung des erfindungsgemäßen Verfahrens (f) in einem größeren Bereich variiert werden. Im allgemeinen arbeitet man zwischen - 20 °C bis + 40 °C, vorzugsweise bei 0 bis 20 °C.

Bei der Durchführung des erfindungsgemäßen Verfahrens (f) arbeitet man vorzugsweise in äquimolaren Mengen. Die Reaktionsdurchführung, Aufarbeitung und Isolierung der Reaktionsprodukte der Formel (I) erfolgt in allgemein üblicher Weise.

Als Verdünnungsmittel kommen für das erfindungsgemäße Verfahren (g) inerte organische Lösungsmittel infrage.

Hierzu gehören aromatische Kohlenwasserstoffe, wie Toluol; Chlorkohlenwasserstoffe, wie Methylenchlorid; Ether, wie Tetrahydrofuran oder 1,2-Dimethoxyethan; Ester, wie Essigester; und Nirile, wie Acetonitril.

Das erfindungsgemäße Verfahren (g) wird gegebenenfalls in Gegenwart eines Katalysators durchgeführt. Beispielsweise genannt seien Triethylamin, 1,4-Diazabicyclo[2,2,2]octan (DABCO) und 1,8-Diazabicyclo[5,4,0]undec-7-en (DBU).

Die Temperaturen können bei der Durchführung des erfindungsgemäßen Verfahrens (g) in einem größeren Bereich variiert werden. Im allgemeinen arbeitet man zwischen - 20 °C und Rückflußtemperatur, vorzugsweise bei Raumtemperatur.

Bei der Durchführung des erfindungsgemäßen Verfahrens (g) können alle Reaktionspartner in geringen Überschüssen eingesetzt werden; vorzugsweise arbeitet man mit äquimolaren Mengen. Die Reaktionsdurchführung, Aufarbeitung und Isolierung der Reaktionprodukte der Formel (I) erfolgt in allgemein üblicher Weise.

Als Verdünnungsmittel kommen für das erfindungsgemäße Verfahren (h) inerte organische Lösungsmittel infrage. Vorzugsweise verwendbar sind Ether, wie Diethylether, Diisopropylether, Tetrahydrofuran oder Dioxan; Ketone, wie Aceton oder Methylethylketon; ferner Acetonitril und Dimethylformamid.

Die Reaktionstemperaturen können bei der Durchführung des erfindungsgemäßen Verfahrens (h) in einem größeren Bereich variiert werden. Bei der Salzbildung arbeitet man allgemein zwischen 0 °C und 100 °C, vorzugsweise zwischen 0 °C und 30 °C. Bei der anschließenden Alkylierung arbeitet man allgemein zwischen 0 °C und 150 °C, vorzugsweise zwischen 60 °C und 120 °C.

Bei der Durchführung des erfindungsgemäßen Verfahrens (h) setzt man vorzugsweise äquimolare Mengen ein, gegebenenfalls einen Überschuß bis zu 1 Mol an Alkylierungsmittel der Formel (XVI). Die Salze der Verbindungen der Formel (XV) können gegebenenfalls isoliert werden, es kann aber auch ohne Isolierung gearbeitet werden. In manchen Fällen erweist es sich als vorteilhaft, die Acetamide der Formel (XV) zunächst zu einem Alkalimetallsalz umzusetzen und dieses dann mit Hilfe von Tetraalkylammonium- bzw. Benzyltrialkylammoniumchloriden oder - bromiden in das entsprechende Ammoniumsalz zu überführen. Die Reaktionsdurchführung, Aufarbeitung und Isolierung der Reaktionsprodukte der Formel (I) erfolgt in allgemein üblicher Weise.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Schädlingsbekämpfungsmittel geeignet.

Zum Beispiel werden fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt.

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Pseudocercosporella-Arten, wie beispielsweise Pseudocerco sporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Schädlingsbekämpfungsmittel können die erfindungsgemäßen Wirkstoffe mit besonders gutem Erfolg zur Bekämpfung von Phytophthora-Arten, wie beispielsweise Phytophthora infestans, an Tomaten eingesetzt werden; sowie auch zur Bekämpfung von Plasmopara-Arten, wie beispielsweise Plasmopara viticola, an Reben.

Besonders hervorzuheben ist, daß die erfindungsgemäßen Wirkstoffe nicht nur eine protektive Wirkung entfalten, sondern auch kurativ wirksam sind, also bei Anwendung nach der Kontamination mit den Sporen des Pilzes.

Ferner eignen sich die erfindungsgemäßen Wirkstoffe sehr gut zur Bekämpfung von Pyricularia-Arten wie Pyricularia oryzae am Reis.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen

Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage; Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage; z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Beispiel 1

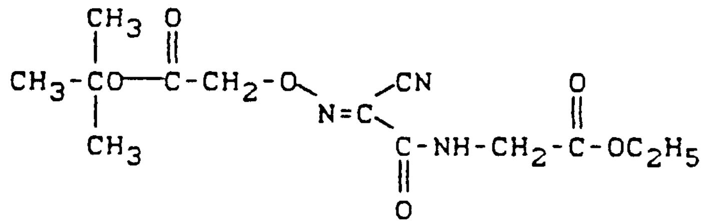

(Verfahren c)

Eine Mischung aus 11 g (0,05 Mol) N-[(E)-2-Cyan-2-hydroximino-acetyl]-glycinethylester-Natriumsalz, 50 ml Dimethylformamid und 8,3 g (0,055 Mol) Chloressigsäure-tert.-butylester wird 15 Stunden bei Raumtemperatur gerührt. Nach Abdestillieren des Lösungsmittels im Vakuum löst man den Rückstand in 150 ml Essigester, wäscht die Lösung mit Wasser (3 x 50 ml), trocknet und dampft ein. Das Rohprodukt wird über 100 g Kieselgel filtriert.

Man erhält 10,2 g (65 % der Theorie) N[(E)-2-(tert.-Butoxycarbonylmethoximino)-2-cyan-acetyl]-glycinethylester als gelbes Öl.

NMR ($CDCl_3$) $\delta$: 1,3 (t, 3H), 1,5 (s, 9H), 4,13 (d, 2H), 4,24 (q, 2H), 4,84 (s, 2H), 7,1 (t, NH).

Herstellung des Ausgangsproduktes

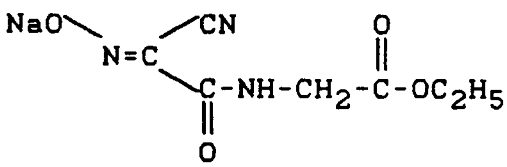

Eine Mischung von 9,0 g (0,05 Mol) 88prozentigem N-Cyan-acetyl-glycinethylester, 5,9 g (0,05 Mol) Isoamylnitrit und 20 ml Ethanol (absolut) wird bei 0 °C tropfenweise mit einer aus 1,2 g (0,05 Mol) Natrium und 20 ml absolutem Ethanol hergestellten Natriumethylatlösung versetzt und 15 Sunden bei Raumtemperatur gerührt. Die Lösung wird eingedampft, der Rückstand mit 20 ml Ether versetzt, der Niederschlag abgesaugt und im Vakuumexsikkator getrocknet.

Man erhält 11 g (99 % der Theorie) N-[(E)-2-Cyan-2-hydroximino-acetyl]-glycinethylester-Natriumsalz als stark hygroskopischen Feststoff.

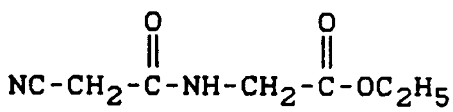

Eine Lösung von 297 g (3 Mol) Cyanessigsäuremethylester und 420 g (3 Mol) Glycinethylester-hydrochlorid in 750 ml Isopropanol wird bei 20 °C mit 630 g (3 Mol) einer 26prozentigen Natriummethylatlösung in Methanol versetzt und 15 Stunden bei Raumtemperatur gerührt. Nach Abdestillieren des Lösungsmittels wird der Rückstand zwischen 200 ml Wasser und 750 ml Methylenchlorid verteilt, die wässrige Phase mit Methylenchlorid (2 x 100 ml) extrahiert und die vereinigten organischen Phasen getrocknet und eingedampft. Das Rohprodukt kristalliersiert man aus 500 ml Ether.

Man erhält 350 g (60 % der Theorie) N-Cyanacetylglycinethylester vom Schmelzpunkt 95 °C - 97 °C; Gehalt: 88 % (GC).

In analoger Weise und gemäß den erfindungsgemäßen Verfahren (a) bis (h) werden die nachfolgenden (2-Cyan-2-oximinoacetyl)-aminosäure-Derivate der allgemeinen Formel (I) erhalten:

$$R-O\sim N=C\begin{matrix} CN \\ CO-NH-R^1 \end{matrix} \qquad (I)$$

| Bsp. Nr. | R | $R^1$ | Physikal. Daten |
|---|---|---|---|
| 2 | $C_2H_5O-CO-CH_2-NH-CO-CH_2-$ | $-CH_2-CO-OC_2H_5$ | Fp. 76-77 °C (E-Isomer) |
| 3 | $CH_3NH-CO-CH_2-NH-CO-CH_2-$ | $-CH_2-CO-NHCH_3$ | Fp:180-183° C (E-Isomer) |
| 4 | $C_2H_5O-CO-CH_2-$ | $-CH_2-CO-NHCH_3$ | Fp:122-124° C (E-Isomer) |
| 5 | $CH_3O-CO-\overset{\overset{CH_3}{\vert}}{CH}-$ (R,S) | $-CH_2-CO-NHCH_3$ | Fp: 60-63° C (E-Isomer) |

| Bsp. Nr. | R | $R^1$ | Physikal. Daten |
|---|---|---|---|
| 6 | $(C_2H_5O)_2CH-CH_2-$ | $-CH_2-CO-NHCH_3$ | Fp. 125-128° C (E-Isomer) |
| 7 | 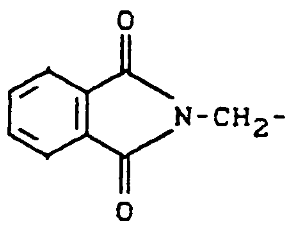 | $-CH_2-CO-NHCH_3$ | Fp: 180-183° C (E-Isomer) |
| 8 | $C_2H_5O-CO-CH_2-$ | $-CH_2-CO-N(C_2H_5)_2$ | Fp: 90-92° C (E-Isomer) |
| 9 | $CH_3O-CO-CH(CH_3)-$ (R, S) | $-CH_2-CO-N(C_2H_5)_2$ | Fp: 80-81° C (E-Isomer) |
| 10 | $(C_2H_5O)_2CH-CH_2-$ | $-CH_2-CO-N(C_2H_5)_2$ | zähes Öl (E-Isomer) |
| 11 | $(CH_3)_3CO-CO-CH_2-$ | $-CH_2-CO-N(C_2H_5)_2$ | Fp: 112-114° C (E-Isomer) |
| 12 | $(CH_3)_3CO-CO-CH_2-$ | $-CH_2-CO-NHCH_3$ | Fp: 127-130° C (E-Isomer) |
| 13 | 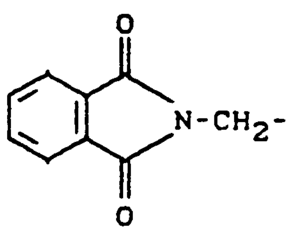 | $-CH_2-CO-N(C_2H_5)_2$ | Fp: 150-153° C (E-Isomer) |

Anwendungsbeispiele

In den folgenden Anwendungsbeispielen werden die nachstehend aufgeführten Verbindungen als Vergleichssubstanzen eingesetzt:

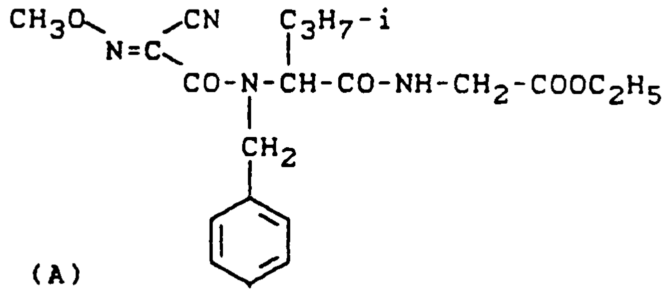

(A)

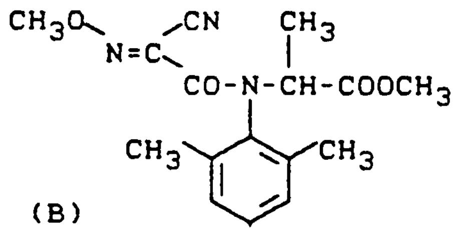

(B)

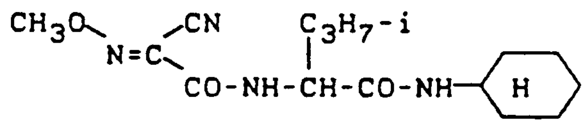

(C)

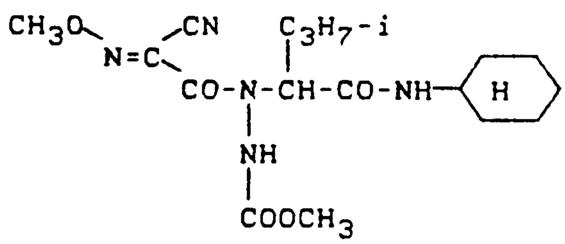

(D)

(bekannt aus DE-OS 3 521 131)

Beispiel A

Phytophthora-Test (Tomate) /kurativ

| | |
|---|---|
| Lösungsmittel: | 4,7 Gewichtsteile Aceton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20 °C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100% relativer Luftfeuchtigkeit und ca. 20 °C aufgestellt.

3 Tage nach der Inokulation erfolgt die Auswertung.

Eine deutliche Überlegenheit in der Wirksamkeit gegenüber dem Stand der Technik zeigen in diesem Test z.B. die Verbindungen gemäß den Herstellungsbeispielen: 4, 6, 8, 9, 11, 12 und 13.

Beispiel B

Plasmopara-Test (Reben) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spriztbelages werden die Pflanzen mit einer wäßrigen Sporensuspension von Plasmopara viticola inokuliert und verbleiben dann 1 Tag in einer Feuchtkammer bei 20 bis 22 °C und 100 % relativer Luftfeuchtigkeit. Anschließend werden die Pflanzen 5 Tage im Gewächshaus bei 22 °C und ca. 80 % Luftfeuchtigkeit aufgestellt. Die Pflanzen werden dann angefeuchtet und 1 Tag in eine Feuchtkammer gestellt.

7 Tage nach der Inokulation erfolgt die Auswertung.

Eine deutliche Überlegenheit in der Wirksamkeit gegenüber dem Stand der Technik zeigen in diesem Test z.B. die Verbindungen gemäß dem Herstellungsbeispiel: 11.

**Patentansprüche**
**Patentansprüche für folgende Vertragsstaaten : AT, BE, CH, DE, FR, GB, GR, IT, LI, NL**

**1.** (2-Cyan-2-oximinoacetyl-aminosäure-Derivate der allgemeinen Formel (I)

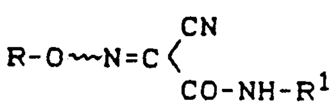

(I)

in welcher

| | |
|---|---|
| $R^1$ | für die Gruppierungen -A-COOR$^2$ oder -B-CONR$^3$R$^4$ steht, |
| A | für eine gegebenenfalls substituierte, geradkettige oder verzweigte Alkylenkette steht, |
| B | für eine gegebenenfalls substituierte, geradkettige oder verzweigte Alkylenkette steht, |
| $R^2$ | für gegebenenfalls substituiertes Alkyl steht mit folgenden Substituenten: Halogen, Hydroxy, Alkoxy, Acyloxy, gegebenenfalls substituiertes Aryl, gegebenenfalls substituiertes Cycloalkyl und gegebenenfalls substituiertes Heterocyclyl; $R^2$ ferner für gegebenenfalls substituiertes Cycloalkyl oder gegebenenfalls substituiertes Cycloalkenyl steht, |
| $R^3$ | für Wasserstoff oder für gegebenenfalls substituiertes Alkyl steht mit folgenden Substituenten : Halogen, Cyano, -COOR$^I$, -CONR$^{II}$R$^{III}$, -NR$^{II}$R$^{III}$, -OR$^{IV}$, -S(O)$_n$R$^{IV}$,Acyl, jeweils gegebenenfalls substituiertes Aryl, Heterocyclyl, Cycloalkyl und Cycloalkenyl; $R^3$ ferner für jeweils gegebenenfalls substituiertes Alkenyl, Alkinyl, Cycloalkyl, Cycloalkenyl, Aryl oder Heterocyclyl steht, |
| $R^4$ | für Wasserstoff oder für gegebenenfalls substituiertes Alkyl steht mit folgenden |

Substituenten: Halogen, Cyano, $-COOR^{I}$, $-CONR^{II}R^{III}$, $-NR^{II}R^{III}$, $-OR^{IV}$, $-S(O)_nR^{IV}$,Acyl, jeweils gegebenenfalls substituiertes Aryl, Heterocyclyl, Cycloalkyl und Cycloalkenyl; $R^4$ ferner für jeweils gegebenenfalls substituiertes Alkenyl, Alkinyl, Cycloalkyl, Cycloalkenyl, Aryl, Heterocyclyl oder polycyclische Carbocyclen oder die Gruppierung $-OR^{IV}$ steht,

oder

A und $R^2$ gemeinsam mit den Atomen, an die sie gebunden sind, für einen gegebenenfalls substituierten Ring stehen,

oder

B und $R^3$ gemeinsam mit den Atomen, an die sie gebunden sind, für einen gegebenenfalls substituierten Ring stehen,

oder

$R^3$ und $R^4$ gemeinsam mit dem Stickstoffatom, an das sie gebunden sind, für einen gegebenenfalls substituierten Heterocyclus stehen, der weitere Heteroatome enthalten kann,

R für substituiertes Alkyl steht mit folgenden Substituenten: Halogen, $-COOR^{I}$, $-CONR^{II}R^{III}$, $-OR^{IV}$, Acyl oder gegebenenfalls substituiertes Heterocyclyl, ausgenommen Azolyl; R ferner für gegebenenfalls substituiertes Cycloalkenyl steht;

$R^{I}$ für Wasserstoff oder gegebenenfalls substituiertes Alkyl steht mit folgenden Substituenten: Halogen, jeweils gegebenenfalls substituiertes Aryl, Cycloalklyl und Cycloalkenyl,

$R^{II}$ und $R^{III}$ gleich oder verschieden sind und für Wasserstoff oder gegebenenfall substituiertes Alkyl stehen mit folgenden Substituenten: Halogen, $-COOR^{IV}$, $-CONR^{I}R^{IV}$, jeweils gegebenenfalls substituiertes Aryl, Cycloalkyl und Cycloalkenyl; $R^{II}$ und $R^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann,

$R^{IV}$ für Wasserstoff, Alkyl, Aralkyl oder Acyl steht und

n für die Zahlen 0, 1 oder 2 steht,

deren geometrische und optische Isomeren und Isomerengemische.

**2.** (2-Cyan-2-oximinoacetyl)-aminosäure-Derivate gemäß Anspruch 1, worin in der Formel (I)

$R^1$ für die Gruppierungen $-A-COOR^2$ oder $-B-CONR^3R^4$ steht,

A für eine geradkettige oder verzweigte Alkylenkette mit 1 bis 4 Kohlenstoffatomen steht,

B für eine geradkettige oder verzweigte Alkylenkette mit 1 bis 4 Kohlenstoffatomen steht,

$R^2$ für gegebenenfalls substituiertes, geradkettiges oder verzweigtes Alkyl mit 1 bis 6 Kohlenstoffatomen steht, wobei als Substituenten genannt seien: Halogen, gegebenenfalls ein-bis fünffach, gleich oder verschieden durch Halogen, Alkyl und Alkoxy mit jeweils 1 bis 4 Kohlenstoffatomen substituiertes Phenyl und Cycloalkyl mit 3 bis 6 Kohlenstoffatomen; ferner steht $R^2$ für gegebenenfalls ein-bis fünffach, gleich oder verschieden durch Alkyl mit 1 bis 4 Kohlenstoffatomen substituiertes Cycloalkyl mit 3 bis 6 Kohlenstoffatomen oder für gegebenenfalls ein- bis fünffach, gleich oder verschieden durch Alkyl mit 1 bis 4 Kohlenstoffatomen substituiertes Cycloalkenyl mit 5 bis 7 Kohlenstoffatomen;

$R^3$ für Wasserstoff oder für gegebenenfalls ein-oder zweifach substituiertes, geradkettiges oder verzweigtes Alkyl mit 1 bis 4 Kohlenstoffatomen steht, wobei als Substituenten genannt seien: Halogen, Cyano, $-COOR^{I}$, $-CONR^{II}R^{III}$, $-NR^{II}R^{III}$, $-OR^{IV}$, $-S(O)_nR^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes Phenyl, gegebenenfalls ein- bis fünffach, gleich oder verschieden durch Halogen sowie Alkyl and Alkoxy mit jeweils 1 bis 4 Kohlenstoffatomen substituierter 5- oder 6-gliedriger Heterocyclus mit 1 bis 3 Heteroatomen, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen; $R^3$ steht ferner für geradkettiges oder verzweigtes Alkenyl oder Alkinyl mit jeweils 2 bis 6 Kohlenstoffatomen; für gegebenenfalls ein- bis fünffach, gleich oder

verschieden durch Alkyl mit 1 bis 4 Kohlenstoffatomen substituiertes Cycloalkenyl mit 5 bis 7 Kohlenstoffatomen oder für gegebenenfalls ein- bis fünffach, gleich oder verschieden substituiertes Cycloalkyl mit 3 bis 6 Kohlenstoffatomen, wobei als Substituenten genannt seien: Halogen, Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im Alkoxyteil, Cycloalkyl und Cycloalkylalkyl mit 5 oder 6 Kohlenstoffatomen im Cycloalkylteil und 1 bis 4 Kohlenstoffatomen im geradkettigen oder verzweigten Alkylteil, gegebenenfalls ein- bis fünffach, gleich oder verschieden durch Halogen und Alkyl mit 1 bis 4 Kohlenstoffatomen substituiertes Phenyl und Pyrrolidon; $R^3$ steht weiterhin für gegebenenfalls ein- bis fünffach, gleich oder verschieden durch Halogen, Alkyl und Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis fünffach, gleich oder verschieden substituierten 5- oder 6-gliedrigen Heterocyclus mit 1 bis 3 gleichen oder verschiedenen Heteroatomen, als Substituenten seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil;

$R^4$ für die Bedeutungen von $R^3$, für polycyclische Carbocyclen oder die Gruppierung $-OR^{IV}$ steht,

oder

A und $R^2$ gemeinsam mit den Atomen, an die sie gebunden sind, für einen Ring der Formel

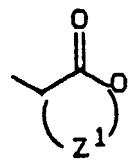

stehen, in welcher $Z^1$ für eine geradkettige oder verzweigte Alkylenkette mit 1 bis 4 Kohlenstoffatomen, steht,

oder

B und $R^3$ gemeinsam mit den Atomen, an die sie gebunden sind, für einen Ring der Formel

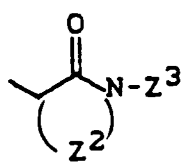

stehen, in welcher $Z^2$ für eine geradkettige oder verzweigte Alkylenkette mit 1 bis 4 Kohlenstoffatomen, und $Z^3$ für Wasserstoff oder Alkyl mit 1 bis 4 Kohlenstoffatomen stehen,

oder

$R^3$ und $R^4$ gemeinsam mit dem Stickstoffatom, an das sie gebunden sind, für einen gegebenenfalls ein- bis fünffach, gleich oder verschieden, substituierten mono-, bi- oder tricyclischen Heterocyclus oder Spiroheterocyclus mit ein bis drei weiteren, gleichen oder verschiedenen Heteroatomen stehen, und wobei als Substituenten genannt seien:
geradkettiges oder verzweigtes Alkyl oder Alkloxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyloder Dialkylcarbamoyl mit jeweils 1 bis 4 Kohlenstoffatomen in jedem Alkylteil, sowie jeweils gegebenenfalls ein- oder zweifach, gleich oder verschieden durch Halogen, geradkettiges oder verzweigtes Alkyl und Alkoxy mit

jeweils 1 bis 4 Kohlenstoffatomen substituiertes Phenyl oder Phenylalkyl mit 1 bis 4 Kohlenstoffatomen im geradkettigen oder verzweigten Alkylteil,

R für ein- oder zweifach substituiertes, geradkettiges oder verzweigtes Alkyl mit 1 bis 6 Kohlenstoffatomen steht, wobei als Substituenten genannt seien: Halogen, $-COOR^{I}$, $-CONR^{II}R^{III}$, $-OR^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 Heteroatomen, ausgenommen Azolyl; ferner steht R vorzugsweise für gegebenenfalls ein-bis fünffach, gleich oder verschieden durch Alkyl mit 1 bis 4 Kohlenstoffatomen substituiertes Cycloalkenyl mit 5 bis 7 Kohlenstoffatomen,

$R^{I}$ für Wasserstoff oder geradkettiges oder verzweigtes Alkyl mit 1 bis 6 Kohlenstoffatomen steht,

$R^{II}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil, wobei als Phenylsubstituenten genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen; $R^{II}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen,

$R^{III}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten die bei $R^{II}$ genannten Phenylsubstituenten infrage kommen; $R^{III}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen;

$R^{IV}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten die bei $R^{II}$ genannten Phenylsubstituenten infrage kommen; $R^{IV}$ steht ferner für Acyl mit 2 bis 9 Kohlenstoffatomen und

n für die Zahlen 0, 1 oder 2 steht, deren geometrische und optische Isomeren und Isomerengemische.

**3.** (2-Cyan-2-oximinoacetyl)-aminosäure-Derivate gemäß Anspruch 1, wobei in der allgemeinen Formel (I)

$R^1$ für die Gruppierungen $-A-COOR^2$ oder $-B-CONR^3R^4$ steht,

A für die Gruppierungen $-CH_2-$, $-CH(CH_3)-$ oder $-CH_2CH_2-$ steht,

B für die Gruppierungen $-CH_2-$, $-CH(CH_3)-$ oder $-CH_2CH_2-$ steht,

$R^2$ für geradkettiges oder verzweigtes Alkyl mit 1 bis 4 Kohlenstoffatomen steht,

$R^3$ für Wasserstoff oder für gegebenenfalls ein-oder zweifach, gleich oder verschieden substituiertes geradkettiges oder verzweigtes Alkyl mit 1 bis 4 Kohlenstoffatomen steht, wobei als Substituenten genannt seien: Halogen, Cyano, $-COOR^{I}$, $-CONR^{II}R^{III}$, $NR^{II}R^{III}$, $-OR^{IV}$, $-S(O)_nR^{IV}$, Acyl mit 2 bis 9 Kohlenstoffatomen, gegenbenenfalls ein- bis dreifach, gleich oder verschieden durch Halogen und Alkyl mit 1 bis 4 Kohlenstoffatomen substituiertes Phenyl, gegebenenfalls ein- bis dreifach, gleich oder verschieden durch Halogen, geradkettiges oder verzweigtes Alkyl und Alkoxy mit jeweils 1 bis 4 Kohlenstoffatomen substituierter 5- oder 6-gliedrigen Heterocyclus mit 1 bis 3 gleichen oder verschiedenen Heteroatomen, und jeweils gegebenenfalls durch geradkettiges oder verzweigtes Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen; $R^3$ steht ferner für geradkettiges oder verzweigtes Alkenyl oder Alkinyl mit jeweils 2 bis 6 Kohlenstoffatomen, für gegebenenfalls durch geradkettiges oder verzweigtes Alkyl mit 1 bis 4 Kohlenstoffatomen substituiertes Cycloalkenyl mit 5 bis 7 Kohlenstoffatomen, für gegebenenfalls substituiertes Cycloalkyl mit 3 bis 6 Kohlenstoffatomen, wobei als

Substituenten genannt seien: Halogen, geradkettiges oder verzweigtes Alkyl und Alkoxy mit jeweils 1 bis 4 Kohlenstoffatomen, Cyano, Amino, Carbamoyl, jeweils geradkettiges oder verzweigtes Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im geradkettigen oder verzweigten Alkoxyteil, Cycloalkyl und Cycloalkylalkyl mit jeweils 5 oder 6 Kohlenstoffatomen im Cycloalkylteil und 1 bis 4 Kohlenstoffatomen im geradkettigen oder verzweigten Alkylteil, gegebenenfalls ein- bis dreifach, gleich oder verschieden durch Halogen und Alkyl mit 1 bis 4 Kohlenstoffatomen substituiertes Phenyl und Pyrrolidon; $R^3$ steht weiterhin für gegebenenfalls ein- bis dreifach, gleich oder verschieden durch Halogen, geradkettiges oder verzweigtes Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen substituiertes Phenyl oder für einen gegebenenfalls ein - bis dreifach, gleich oder verschieden substituierten 5- oder 6-gliedrigen Heterocyclus mit 1 bis 3 gleichen oder verschiedenen Heteroatomen, als Substituenten für die Heterocyclen seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkyl;

$R^4$ für die Bedeutungen von $R^3$, für polycyclische Carbocyclen oder die Gruppierung $-OR^{IV}$ steht,

oder

A und $R^2$ gemeinsam mit den Atomen, an die sie gebunden sind, für gegebenenfalls durch Alkyl mit 1 bis 4 Kohlenstoffatomen substituierte 5- oder 6-gliedrige Ringe der Formeln

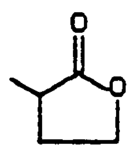 oder 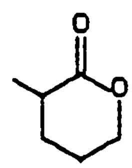 stehen,

oder

B und $R^3$ gemeinsam mit den Atomen, an die sie gebunden sind, für gegebenenfalls durch Alkyl mit 1 bis 4 Kohlenstoffatomen substituierte 5- bis 7-gliedrige Ringe der Formeln

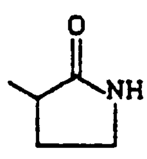, 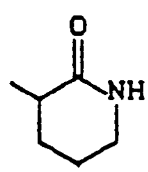 und 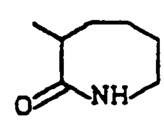

stehen,

oder

$R^3$ und $R^4$ gemeinsam mit dem Stickstoffatom, an das sie gebunden sind, für einen gegebenenfalls ein- bis fünffach, gleich oder verschieden substituierten mono-, bi- oder tricyclischen Heterocyclus oder Spiroheterocyclus stehen, wobei als Heterocyclen genannt seien: Oxazolidin, Pyrrolidin, Imidazolidin, Piperidin, Piperazin, Morpholin, Thiomorpholin, 1,3-Oxazan oder 1,3-Diazan; diese Heterocyclen können jeweils gegebenenfalls mit 1 oder 2 Benzol- oder Cyclohexanringen anelliert sein oder gegebenenfalls mit Methylen oder Ethylen überbrückt sein.

Als Substituenten für alle Heterosysteme seien genannt:

geradkettiges oder verzweigtes Alkyl oder Akoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mmit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyloder Dialkylcarbamoyl mit jeweils 1 bis 4 Kohlenstoffatomen in jedem Alkylteil, sowie jeweils gegebenenfals ein- oder zweifach, gleich oder verschieden durch Halogen oder geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

R für substituiertes, geradkettiges oder verzweigtes Alkyl mit 1 bis 4 Kohlenstoffatomen steht, wobei als Substituenten genannt seien: Halogen, $-COOR^{I}$, - $CONR^{II}R^{III}$, $-OR^{IV}$, Acyl mit 2 bis 9 Kohlenstoffatomen, gegebenenfalls einbis dreifach, gleich oder verschieden durch Alkyl mit 1 oder 2 Kohlenstoffatomen substituierte Heterocyclen der Formeln

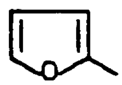 , 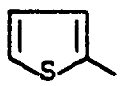 , 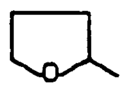 , 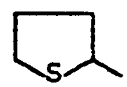 ,

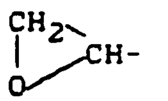 , 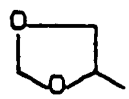 und 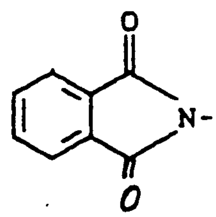 ,

R steht ferner für gegebenenfalls ein- bis dreifach durch Methyl substituiertes Cyclohexenyl oder Cyclopentenyl,

$R^{I}$ für geradkettiges oder verzweigtes Alkyl mit 1 bis 4 Kohlenstoffatomen steht,

$R^{II}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl und Phenethyl steht, wobei als Phenylsubstituenten jeweils genannt seien: Fluor, Chlor, Methyl, Methoxy und Trifluormethyl; $R^{II}$ ferner für Alkoxycarbonylalkyl mit 1 bis 4 Kohlenstoffatomen im Alkoxyteil und 1 oder 2 Kohlenstoffatomen im Alkylteil, Carbamoylalkyl mit 1 oder 2 Kohlenstoffatomen im Alkylteil, Alkylcarbamoylalkyl und Dialkylcarbamoylalkyl mit jeweils 1 oder 2 Kohlenstoffatomen in jedem Alkylteil, oder für gegebenenfalls ein - bis dreifach, gleich oder verschieden durch Methyl und Ethyl substituiertes Cycloalkyl mit 3 bis 6 Kohlenstoffatomen steht,

$R^{III}$ für die Bedeutungen von $R^{II}$ steht,

$R^{IV}$ für Wasserstoff, geradkettiges oder verzweigtes Alkyl mit 1 bis 4 Kohlenstoffatomen, jeweils gegebenenfalls ein - bis dreifach, gleich oder verschieden substituiertes Benzyl oder Phenethyl steht, wobei als Phenylsubstituenten jeweils genannt seien: Fluor, Chlor, Methyl, Methoxy und Trifluromethyl; $R^{IV}$ steht ferner für Acyl mit 2 bis 9 Kohlenstoffatomen und

n für die Zahlen 0, 1 oder 2 steht,

deren geometrische und optische Isomeren und Isomerengemische.

**4.** (2-Cyan-2-oximinoacetyl)-aminosäure-Derivate gemäß Anspruch 1, wobei in der Formel (I)

$R^1$ für die Gruppierung $-B-CO-NR^3R^4$ steht,

B für die Gruppierungen $-CH_2-$, $-CH(CH_3)-$ oder $-CH_2CH_2-$ steht,

$R^3$ für Wasserstoff oder geradkettiges oder verzweigtes Alkyl mit 1 bis 4 Kohlenstoffatomen steht,

$R^4$ für Wasserstoff oder geradkettiges oder verzweigtes Alkyl mit 1 bis 4 Kohlenstoffatomen steht,

R für substituiertes Alkyl mit 1 oder 2 Kohlenstoffatomen steht, wobei als Substituenten genannt seien: Halogen, Alkylcarbonyl mit 1 bis 4 Kohlenstoffatomen im geradkettigen oder verzweigten Alkylteil, die Gruppierungen $-COOR^{I}$, $-CONR^{II}R^{III}$, $-OR^{IV}$ und die folgenden Heterocyclen:

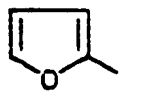 , 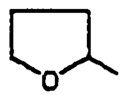 , 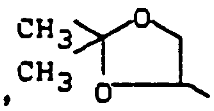 , 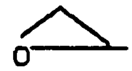 und

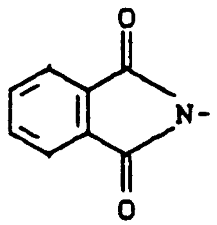 ;

R steht ferner für gegebenenfalls ein-bis dreifach durch Methyl substituiertes 1-Cyclohexenyl;

$R^I$ für Methyl, Ethyl, n- oder i-Propyl oder n-, i-, s-oder t-Butyl steht,

$R^{II}$ und $R^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor und Methyl als Substituenten, für Alkoxycarbonylalkyl mit 1 oder 2 Kohlenstoffatomen in jedem Alkylteil, für Carbamoylalkyl, Alkylcarbamoylalkyl oder Dialkylcarbamoylalkyl mit jeweils 1 odr 2 Kohlenstoffatomen in jedem Alkylteil oder für gegebenenfalls ein-bis dreifach durch Methyl substituiertes Cyclohexyl stehen, und

$R^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien, deren geometrische und optische Isomeren und Isomerengemische.

**5.** (2-Cyan-2-oximinoacetyl)-aminosäure-Derivate gemäß Anspruch 1, wobei in der Formel (I)

$R^1$ für die Gruppierung -B-CO-$NR^3R^4$ steht,

B für die Gruppierungen -$CH_2$-, -$CH(CH_3)$- oder -$CH_2CH_2$- steht,

$R^3$ für Wasserstoff, geradkettiges oder verzweigtes Alky mit 1 bis 4 Kohlenstoffatomen oder ein- oder zweifach substituiertes Alkyl mit 1 bis 3 Kohlenstoffatomen steht, wobei als Substituenten genannt seien; Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl;

$R^3$ steht ferner für Allyl oder Propargyl, für 1-Cyclohexenyl oder für ein- bis dreifach, gleich oder verschieden substituiertes Cyclohexyl, wobei als Substituenten genannt seien: Chlor, Methyl, Ethyl, Methoxy, Cyano, Amino, Alkyl- oder Dialkylamino mit jeweils 1 oder 2 Kohlenstoffatomen in jedem Alkylteil, Carbamoyl, Alkyl- oder Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil oder 1-Pyrrolidin-2-on;

$R^3$ steht außerdem für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4,-Thiadiazol-5-yl, für gegebenenfalls ein- bis dreifach, gleich oder verschieden durch Mercapto geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadazol-5-yl oder für die Gruppierung

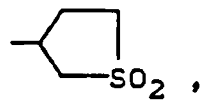

$R^4$ für ein- oder zweifach substituiertes Alkyl mit 1 bis 3 Kohlenstoffatomen steht, wobei als Substituenten genannt seien: Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl;
$R^4$ steht ferner für Allyl oder Propargyl, für 1-Cyclohexenyl oder für ein- bis dreifach, gleich oder verschieden substituiertes Cyclohexyl, wobei als Substituenten genannt seien: Chlor, Methyl, Ethyl, Methoxy, Cyano, Amino, Alkyl-oder Dialkylamino mit jeweils 1 oder 2 Kohlenstoffatomen in jedem Alkylteil, Carbamoyl, Alkyl- oder Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil und 1-Pyrrolidin-2-on;
$R^4$ steht außerdem für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein-bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadazol-5-yl oder für die Gruppierung

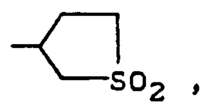

$R^4$ steht weiterhin für Hydroxy, Alkoxy mit 1 oder 2 Kohlenstoffatomen, für gegebenenfalls ein- bis dreifach, gleich oder verschieden durch Chlor und Methyl substituiertes Benzyloxy sowie für 1-Adamantyl, 2-Norbornyl, 1- oder 2-Decalyl oder 1- oder 2-Tetralyl;

R für substituiertes Alkyl mit 1 oder 2 Kohlenstoffatomen steht, wobei als Substituenten genannt seien: Halogen, Alkylcarbonyl mit 1 bis 4 Kohlenstoffatomen im Alkylteil, die Gruppierungen -COOR$^I$, -CONR$^{II}$R$^{III}$, OR$^{IV}$ oder die folgenden Heterocyclen:

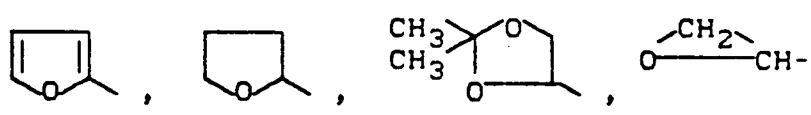

und

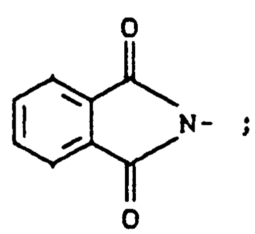

R steht ferner für jeweils gegebenenfalls ein-bis dreifach durch Methyl substituiertes 1-Cyclohexenyl,

$R^I$ für Methyl, Ethyl, n- oder i-Propyl oder n-, i-, s-oder t-Butyl steht,

$R^{II}$ und $R^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor oder Methyl als Substituenten, für Alkoxycarbonylalkyl mit 1 oder 2 Kohlenstoffatomen in jedem Alkylteil, für Carbamoylalkyl, Alkylcarbamoylalkyl oder Dialkylcarbamoylalkyl mit jeweils 1 oder 2 Kohlenstoffatomen in jedem Alkylteil oder für gegebenenfalls ein-bis dreifach durch Methyl substituiertes Cyclohexyl stehen, und

$R^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien,

deren geometrische und optische Isomeren und Isomerengemische.

**6.** (2-Cyan-2-oximinoacetyl)-aminosäure-Derivate gemäß Anspruch 1, wobei in der Formel (I)

$R^1$ für die Grupierung $-B-CO-NR^3R^3$ steht,

B für die Gruppierungen $-CH_2-$, $-CH(CH_3)-$ oder $-CH_2CH_2-$ steht,

$R^3$ und $R^4$ gemeinsam mit dem Stickstoffatom, an das sie gebunden sind, für folgende mono-, bi-oder tricyclischen Heterocyclen oder Spiroheterocyclen stehen:

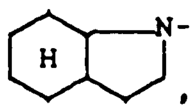 , 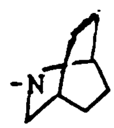 , 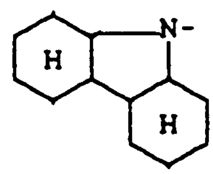 ,

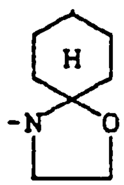 , 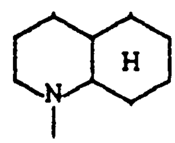 ,

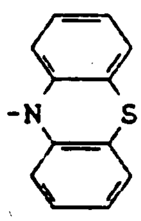 , 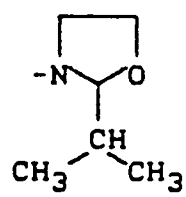 , 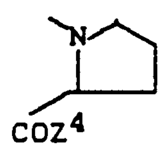

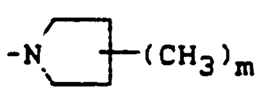 , 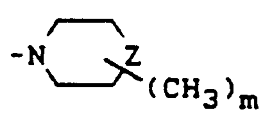

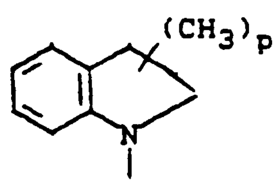 , 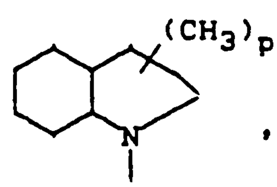 ,

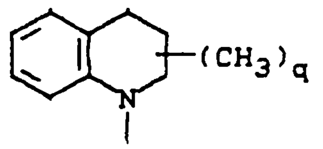 , 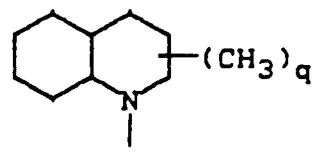 ,

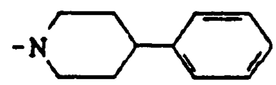 , 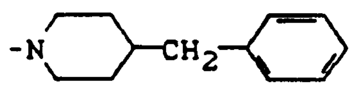 ,

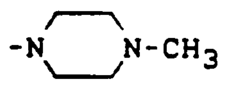 , 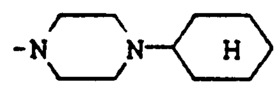 , 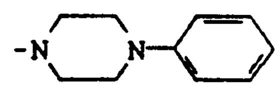 ,

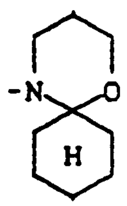 , 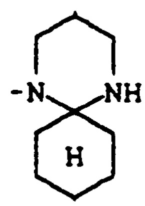 , 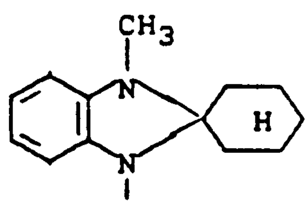 ,

wobei

Z für Sauerstoff oder die $CH_2$-Gruppe steht,

$Z^4$ für Hydroxy, Methoxy, Ethoxy, Amino, Methylamino, Ethylamino, Dimethylamino oder Ethylmethylamino steht,

m für die Zahlen 0, 1, 2, 3 oder 4 steht,

p für die Zahlen 0, 1 oder 2 steht, und

q für die Zahlen 0, 1, 2 oder 3 steht und

R für substituiertes Alkyl mit 1 oder 2 Kohlenstoffatomen steht, wobei als Substituenten genannt seien: Halogen, Alkylcarbonyl mit 1 bis 4 Kohlenstoffatomen im geradkettigen oder verzweigten Alkylteil, $-COOR^I$, $-CONR^{II}R^{III}$, $OR^{IV}$ und die folgenden Heterocyclen:

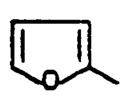 , 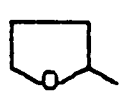 , 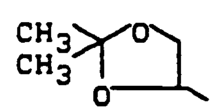 , 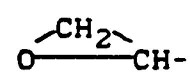

und 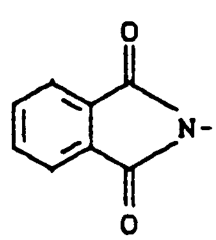 ;

R steht ferner für jeweils gegebenenfalls ein-bis dreifach durch Methyl substituiertes 1-Cyclohexenyl

$R^I$ für Methyl, Ethyl, n- oder i-Propyl oder n-, i-, s-oder t-Butyl steht,

$R^{II}$ und $R^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor und Methyl als Substituenten, für Alkoxycarbonylalkyl mit 1 oder 2 Kohlenstoffatomen in jedem Alkylteil, für Carbamoylalkykl, Alkylcarbamoylalkyl oder

Dialkylcarbamoylalkyl mit jeweils 1 oder 2 Kohlenstoffatomen in jedem Alkylteil oder für gegebenenfalls ein-bis dreifach durch Methyl substituiertes Cyclohexyl stehen und

$R^{IV}$ für Wasserstoff, Alkyl mit 1 oder 2 Kohlenstoffatomen oder gegebenenfall ein- bis dreifach, gleich oder verschieden substituiertes Benzyl steht, wobei als Phenylsubstituenten Fluor, Chlor und Methyl genannt seien,

deren geometrische und optische Isomeren und Isomerengemische.

**7.** (2-Cyan-2-oximinoacetyl)-aminosäure-Derivate gemäß Anspruch 1, worin in der Formel (I)

$R^1$ für die Gruppierung -A-COOR$^2$ steht,

A für die Gruppierungen $-CH_2-$, $-CH(CH_3)-$ oder $-CH_2CH_2-$ steht,

$R^2$ für Methyl oder Ethyl steht,

R für ein- oder zweifach substituiertes Alkyl mit 1 oder 2 Kohlenstoffatomen steht, wobei als Substituenten genannt seien: Halogen, Alkylcarbonyl mit 1 bis 4 Kohlenstoffatomen im geradkettigen oder verzweigten Alkylteil, -COOR$^I$, -CONR$^{II}$R$^{III}$, OR$^{IV}$ und die folgenden Heterocyclen:

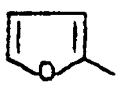 , 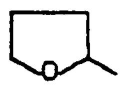 , 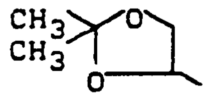 , 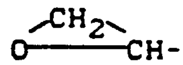

und 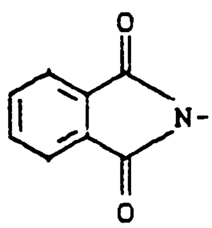 ;

R steht ferner für jeweils gegebenenfalls ein- bis dreifach durch Methyl substituiertes 1-Cyclohexenyl,

$R^I$ für Methyl, Ethyl, n- oder i-Propyl, n-, i-, s-oder t-Butyl steht,

$R^{II}$ und $R^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor und Methyl als Substituenten, für Alkoxycarbonylalkyl mit 1 oder 2 Kohlenstoffatomen in jedem Alkylteil, für Carbamoylalkyl, Alkylcarbamoylalkyl oder Dialkylcarbamoylalkyl mit jeweils 1 oder 2 Kohlenstoffatomen in jedem Alkylteil oder für gegebenenfalls ein-bis dreifach durch Methyl substituiertes Cyclohexyl stehen und

$R^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien,

deren geometrische und optische Isomeren und Isomerengemische.

**8.** Verfahren zur Herstellung von (2-Cyan-2-oximinoacetyl)-aminosäure-Derivaten der allgemeinen Formel (I),

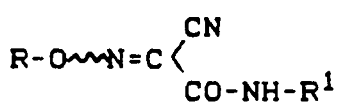 (I)

in welcher

$R^1$ für die Gruppierungen -A-COOR$^2$ oder -B-CONR$^3$R$^4$ steht,

A für eine gegebenenfalls substituierte, geradkettige oder verzweigte Alkylenkette steht,

B für eine gegebenenfalls substituierte, geradkettige oder verzweigte Alkylenkette steht,

$R^2$ für gegebenenfalls substituiertes Alkyl steht mit folgenden Substituenten: Halogen, Hydroxy, Alkoxy, Acyloxy, gegebenenfalls substituiertes Aryl, gegebenenfalls substituiertes Cycloalkyl und gegebenenfalls substituiertes Heterocyclyl; $R^2$ ferner für gegebenenfalls substituiertes Cycloalkyl oder gegebenenfalls substituiertes Cycloalkenyl steht,

$R^3$ — für Wasserstoff oder für gegebenenfalls substituiertes Alkyl steht mit folgenden Substituenten : Halogen, Cyano, $-COOR^I$, $-CONR^{II}R^{III}$, $-NR^{II}R^{III}$, $-OR^{IV}$, $-S(O)_nR^{IV}$,Acyl, jeweils gegebenenfalls substituiertes Aryl, Heterocyclyl, Cycloalkyl und Cycloalkenyl; $R^3$ ferner für jeweils gegebenenfalls substituiertes Alkenyl, Alkinyl, Cycloalkl, Cycloalkenyl, Aryl oder Heterocyclyl steht,

$R^4$ — für Wasserstoff oder für gegebenenfalls substituiertes Alkyl steht mit folgenden Substituenten: Halogen, Cyano, $-COOR^I$, $-CONR^{II}R^{III}$, $-NR^{II}R^{III}$, $-OR^{IV}$, $-S(O)_nR^{IV}$,Acyl, jeweils gegebenenfalls substituiertes Aryl, Heterocyclyl, Cycloalkyl und Cycloalkenyl; $R^4$ ferner für jeweils gegebenenfalls substituiertes Alkenyl, Alkinyl, Cycloalkyl, Cycloalkenyl, Aryl, Heterocyclyl oder polycyclische Carbocyclen oder die Gruppierung $-OR^{IV}$ steht,

oder

A und $R^2$ — gemeinsam mit den Atomen, an die sie gebunden sind, für einen gegebenenfalls substituierten Ring stehen,

oder

B und $R^3$ — gemeinsam mit den Atomen, an die sie gebunden sind, für einen gegebenenfalls substituierten Ring stehen,

oder

$R^3$ und $R^4$ — gemeinsam mit dem Stickstoffatom, an das sie gebunden sind, für einen gegebenenfalls substituierten Heterocyclus stehen, der weitere Heteroatome enthalten kann,

R — für substituiertes Alkyl steht mit folgenden Substituenten: Halogen, $-COOR^I$, $-CONR^{II}R^{III}$, $-OR^{IV}$, Acyl und gegebenenfalls substituiertes Heterocyclyl, ausgenommen Azolyl; R ferner für gegebenenfalls substituiertes Cycloalkenyl steht;

$R^I$ — für Wasserstoff oder gegebenenfalls substituiertes Alkyl steht mit folgenden Substituenten: Halogen, jeweils gegebenenfalls substituiertes Aryl, Cycloalkyl und Cycloalkenyl,

$R^{II}$ und $R^{III}$ — gleich oder verschieden sind und für Wasserstoff oder gegebenenfalls substituiertes Alkyl stehen mit folgenden Substituenten: Halogen, $-COOR^{IV}$, $-CONR^IR^{IV}$, jeweils gegebenenfalls substituiertes Aryl, Cycloalkyl und Cycloalkenyl; $R^{II}$ und $R^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann,

$R^{IV}$ — für Wasserstoff, Alkyl, Aralkyl oder Acyl steht und

n — für die Zahlen 0, 1 oder 2 steht,

deren geometrische und optische Isomeren und Isomerengemische, dadurch gekennzeichnet, daß man

a) 2-Cyan-2-oximino-essigsäureester der allgemeinen Formel (II),

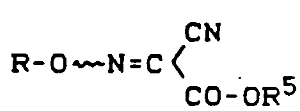

(II)

in welcher

R — die oben angegebene Bedeutung hat und

$R^5$ — für Alkyl steht,

mit Aminen der Formel (III),

$R^1-NH_2$ (III)

in welcher

$R^1$ — die oben angegebene Bedeutung hat,

gegebenenfalls in Gegenwart eines Verdünnungsmittels und gegebenenfalls in Gegenwart einer

Base umsetzt;
oder
b) carboxy-aktivierte Derivate von Carbonsäuren der allgemeinen Formel (IV),

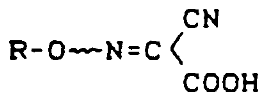
(IV)

in welcher
R die oben angegebene Bedeutung hat,
mit Aminen der Formel (III),

$R^1$-$NH_2$ (III)

in welcher
$R^1$ die oben angegebene Bedeutung hat,
gegebenenfalls in Gegenwart eines Katalysators und gegebenenfalls in Gegenwart eines Säurebindemittels sowie gegebenenfalls in Gegenwart eines Verdünnungsmittels umsetzt;
oder
c) 2-Cyan-2-oximino-acetamide der allgemeinen Formel (V),

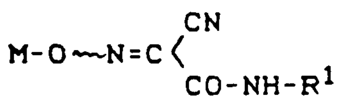
(V)

in welcher
M für Wasserstoff, ein Alkalimetallion, eine protonierte tertiäre Base oder ein quarternäres Ammoniumion steht und
$R^1$ die oben angegebene Bedeutung hat,
mit Verbindungen der Formel (VI),

R-X (VI)

in welcher
X für Halogen oder einen Sulfonyloxy-Rest steht und
R die oben angegebene Bedeutung hat,
gegebenenfalls in Gegenwart einer Base und gegebenenfalls in Gegenwart eines Verdünnungsmittels umsetzt;
oder
d) substituierte Acetyl-aminosäureester der allgemeinen Formel (VII),

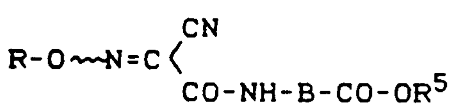
(VII)

in welcher
B, R und $R^5$ die oben angegebene Bedeutung haben,
mit Aminen der Formel (VIII),

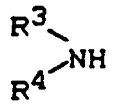

(VIII)

in welcher

$R^3$ und $R^4$ die oben angegebene Bedeutung haben,

gegebenenfalls in Gegenwart eines Verdünnungsmittels und gegebenenfalls in Gegenwart einer Base umsetzt;

oder

e) carboxy-akivierte Acetyl-aminosäuren der allgemeinen Formel (IX),

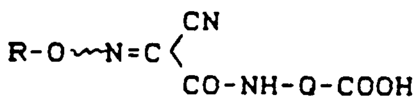

(IX)

in welcher

R die oben angegebene Bedeutung hat und

Q für A oder B steht, wobei diese die oben angegebene Bedeutung haben,

mit Verbindungen der Formel (X),

Y-H (X)

in welcher

Y für die Gruppierungen $R^2O$- oder $R^3R^4N$-steht, wobei $R^2$, $R^3$ und $R^4$ die oben angegebene Bedeutung haben,

gegenbenenfalls in Gegenwart eines Katalysators und gegebenenfalls in Gegenwart eines Säurebindemittels sowie gegebenenfalls in Gegenwart eines Verdünnungsmittels umsetzt;

oder

f) Ammoniumsalze von 2-Cyan-2-oximino-essigsäuren der allgemeinen Formel (XI),

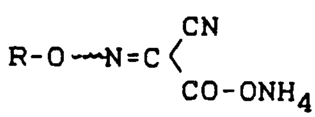

(XI)

in welcher

R die oben angegebene Bedeutung hat,

mit Aldehyden der Formel (XII),

$R^6$-CH=O (XII)

in welcher

die Gruppierung $R^6$-CH= für die oben genannten Bedeutungen von A oder B steht,

und mit Isonitrilen der Formel (XIII),

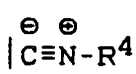

(XIII)

in welcher

$R^4$ die oben angegebene Bedeutung hat,

gegebenenfalls in Gegenwart eines Verdünnungsmittels umsetzt;

oder
g) 2-Cyan-2-oximino-essigsäuren der allgemeinen Formel (IV),

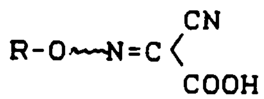
(IV)

in welcher
R die oben angegebene Bedeutung hat,
mit Isocyanaten der Formel (XIV),

$R^1$-NCO (XIV)

in welcher
$R^1$ die oben angegebene Bedeutung hat,
gegebenenfalls in Gegenwart eines Katalysators und gegebenenfalls in Gegenwart eines Verdünnungsmittels umsetzt;
oder
h) 2-Cyano-2-oximino-acetamide der allgemeinen Formel (XV),

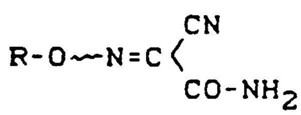
(XV)

in welcher
R die oben angegebene Bedeutung hat,
mit einer Base, gegebenenfalls in Gegenwart eines Verdünnungsmittels, umsetzt und die entstehenden Salze direkt oder gegebenenfalls nach Zwischenisolierung mit Verbindungen der Formel (XVI),

$R^1$-X (XVI)

in welcher
$R^1$ und X die oben angegebene Bedeutung haben,
gegebenenfalls in Gegenwart eines Verdünnungsmittels umsetzt.

**9.** Schädlingsbekämpfungsmittel, gekennzeichnet durch einen Gehalt an mindestens einem (2-Cyano-2-oximino-acetyl)-aminosäure-Derivat der Formel (I) nach den Ansprüchen 1 und 8.

**10.** Verwendung von (2-Cyano-2-oximino-acetyl)-aminosäure-Derivaten der Formel (I) nach den Ansprüchen 1 und 8 zur Bekämpfung von Schädlingen.

**11.** Verfahren zur Bekämpfung von Schädlingen, dadurch gekennzeichnet, daß man (2-Cyano-2-oximino-acetyl)-aminosäure-Derivate der Formel (I) nach den Ansprüchen 1 und 8 auf Schädlinge und/oder ihren Lebensraum einwirken läßt.

**12.** Verfahren zur Herstellung von Schädlingsbekämpfungsmitteln, dadurch gekennzeichnet, daß man (2-Cyano-2-oximino-acetyl)-aminosäure-Derivate der Formel (I) nach den Ansprüchen 1 und 8 mit Streckmitteln und/oder oberflächenaktiven Mitteln vermischt.

**Patentansprüche für folgenden Vertragsstaat : ES**

**1.** Verfahren zur Herstellung von (2-Cyan-2-oximinoacetyl)-aminosäure-Derivaten der allgemeinen Formel (I),

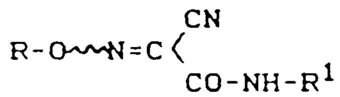

(I)

in welcher

- $R^1$ für die Gruppierungen $-A-COOR^2$ oder $-B-CONR^3R^4$ steht,
- A für eine gegebenenfalls substituierte, geradkettige oder verzweigte Alkylenkette steht,
- B für eine gegebenenfalls substituierte, geradkettige oder verzweigte Alkylenkette steht,
- $R^2$ für gegebenenfalls substituiertes Alkyl steht mit folgenden Substituenten: Halogen, Hydroxy, Alkoxy, Acyloxy, gegebenenfalls substituiertes Aryl, gegebenenfalls substituiertes Cycloalkyl und gegebenenfalls substituiertes Heterocyclyl; $R^2$ ferner für gegebenenfalls substituiertes Cycloalkyl oder gegebenenfalls substituiertes Cycloalkenyl steht,
- $R^3$ für Wasserstoff oder für gegebenenfalls substituiertes Alkyl steht mit folgenden Substituenten : Halogen, Cyano, $-COOR^I$, $-CONR^{II}R^{III}$, $-NR^{II}R^{III}$, $-OR^{IV}$, $-S(O)_nR^{IV}$,Acyl, jeweils gegebenenfalls substituiertes Aryl, Heterocyclyl, Cycloalkyl und Cycloalkenyl; $R^3$ ferner für jeweils gegebenenfalls substituiertes Alkenyl, Alkinyl, Cycloalkyl, Cycloalkenyl, Aryl oder Heterocyclyl steht,
- $R^4$ für Wasserstoff oder für gegebenenfalls substituiertes Alkyl steht mit folgenden Substituenten: Halogen, Cyano, $-COOR^1$, $-CONR^{II}R^{III}$, $-NR^{II}R^{III}$, $-OR^{IV}$, $-S(O)_nR^{IV}$,Acyl, jeweils gegebenenfalls substituiertes Aryl, Heterocyclyl, Cycloalkyl und Cycloalkenyl; $R^4$ ferner für jeweils gegebenenfalls substituiertes Alkenyl, Alkinyl, Cycloalkyl, Cycloalkenyl, Aryl, Heterocyclyl oder polycyclische Carbocyclen oder die Gruppierung $-OR^{IV}$ steht,

  oder
- A und $R^2$ gemeinsam mit den Atomen, an die sie gebunden sind, für einen gegebenenfalls substituierten Ring stehen,

  oder
- B und $R^3$ gemeinsam mit den Atomen, an die sie gebunden sind, für einen gegebenenfalls substituierten Ring stehen,

  oder
- $R^3$ und $R^4$ gemeinsam mit dem Stickstoffatom, an das sie gebunden sind, für einen gegebenenfalls substituierten Heterocyclus stehen, der weitere Heteroatome enthalten kann,
- R für substituiertes Alkyl steht mit folgenden Substituenten: Halogen, $-COOR^I$, $-CONR^{II}R^{III}$, $-OR^{IV}$, Acyl und gegebenenfalls substituiertes Heterocyclyl, ausgenommen Azolyl; R ferner für gegebenenfalls substituiertes Cycloalkenyl steht;
- $R^I$ für Wasserstoff oder gegebenenfalls substituiertes Alkyl steht mit folgenden Substituenten: Halogen, jeweils gegebenenfalls substituiertes Aryl, Cycloalkyl und Cycloalkenyl,
- $R^{II}$ und $R^{III}$ gleich oder verschieden sind und für Wasserstoff oder gegebenenfalls substituiertes Alkyl stehen mit folgenden Substituenten: Halogen, $-COOR^{IV}$, $-CONR^IR^{IV}$, jeweils gegebenenfalls substituiertes Aryl, Cycloalkyl und Cycloalkenyl; $R^{II}$ und $R^{III}$ ferner für Alkenyl, Alkinyl oder jeweils gegebenenfalls substituiertes Cycloalkyl, Cycloalkenyl, Aryl oder Heterocyclyl stehen oder gemeinsam mit dem Stickstoffatom, an das sie gebunden sind, für einen gegebenenfalls substituierten Heterocyclus stehen, der weitere Heteroatome enthalten kann,
- $R^{IV}$ für Wasserstoff, Alkyl, Aralkyl oder Acyl steht und
- n für die Zahlen 0, 1 oder 2 steht,

deren geometrische und optische Isomeren und Isomerengemische, dadurch gekennzeichnet, daß man

a) 2-Cyan-2-oximino-essigsäureester der allgemeinen Formel (II),

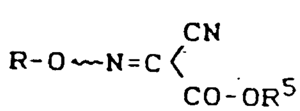

(II)

in welcher

R die oben angegebene Bedeutung hat und

$R^5$ für Alkyl steht,

mit Aminen der Formel (III),

$R^1$-$NH_2$ (III)

in welcher

$R^1$ die oben angegebene Bedeutung hat,

gegebenenfalls in Gegenwart eines Verdünnungsmittels und gegebenenfalls in Gegenwart einer Base umsetzt;

oder

b) carboxy-aktivierte Derivate von Carbonsäuren der allgemeinen Formel (IV),

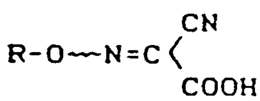

(IV)

in welcher

R die oben angegebene Bedeutung hat,

mit Aminen der Formel (III),

$R^1$-$NH_2$ (III)

in welcher

$R^1$ die oben angegebene Bedeutung hat,

gegebenenfalls in Gegenwart eines Katalysators und gegebenenfalls in Gegenwart eines Säurebindemittels sowie gegebenenfalls in Gegenwart eines Verdünnungsmittels umsetzt;

oder

c) 2-Cyan-2-oximino-acetamide der allgemeinen Formel (V),

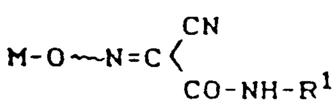

(V)

in welcher

M für Wasserstoff, ein Alkalimetallion, eine protonierte tertiäre Base oder ein quarternäres Ammoniumion steht und

$R^1$ die oben angegebene Bedeutung hat,

mit Verbindungen der Formel (VI),

R-X (VI)

in welcher

X für Halogen oder einen Sulfonyloxy-Rest steht und

R die oben angegebene Bedeutung hat,

gegebenenfalls in Gegenwart einer Base und gegebenenfalls in Gegenwart eines Verdünnungsmittels umsetzt;

oder

d) substituierte Acetyl-aminosäureester der allgemeinen Formel (VII),

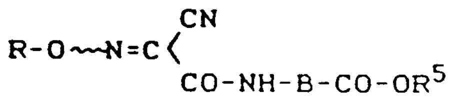

(VII)

in welcher

B, R und $R^5$ die oben angegebene Bedeutung haben,

mit Aminen der Formel (VIII),

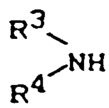

(VIII)

in welcher

$R^3$ und $R^4$ die oben angegebene Bedeutung haben,

gegebenenfalls in Gegenwart eines Verdünnungsmittels und gegebenenfalls in Gegenwart einer Base umsetzt;

oder

e) carboxy-akivierte Acetyl-aminosäuren der allgemeinen Formel (IX),

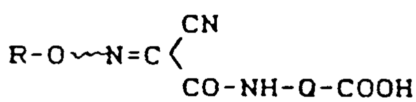

(IX)

in welcher

R die oben angegebene Bedeutung hat und

Q für A oder B steht, wobei diese die oben angegebene Bedeutung haben,

mit Verbindungen der Formel (X),

Y-H (X)

in welcher

Y für die Gruppierungen $R^2O$- oder $R^3R^4N$-steht, wobei $R^2$, $R^3$ und $R^4$ die oben angegebene Bedeutung haben,

gegebenenfalls in Gegenwart eines Katalysators und gegebenenfalls in Gegenwart eines Säurebindemittels sowie gegebenenfalls in Gegenwart eines Verdünnungsmittels umsetzt;

oder

f) Ammoniumsalze von 2-Cyan-2-oximino-essigsäuren der allgemeinen Formel (XI),

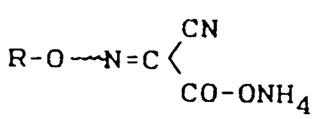

(XI)

in welcher

R die oben angegebene Bedeutung hat,

mit Aldehyden der Formel (XII),

$R^6$-CH = O (XII)

in welcher

die Gruppierung $R^6$-CH = für die oben genannten Bedeutungen von A oder B steht,

und mit Isonitrilen der Formel (XIII),

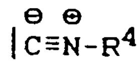 (XIII)

in welcher

$R^4$ die oben angegebene Bedeutung hat,

gegebenenfalls in Gegenwart eines Verdünnungsmittels umsetzt;

oder

g) 2-Cyan-2-oximino-essigsäuren der allgemeinen Formel (IV),

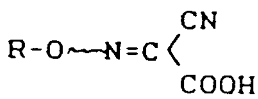 (IV)

in welcher

R die oben angegebene Bedeutung hat,

mit Isocyanaten der Formel (XIV),

$R^1$-NCO (XIV)

in welcher

$R^1$ die oben angegebene Bedeutung hat,

gegebenenfalls in Gegenwart eines Katalysators und gegebenenfalls in Gegenwart eines Verdünnungsmittels umsetzt;

oder

h) 2-Cyano-2-oximino-acetamide der allgemeinen Formel (XV),

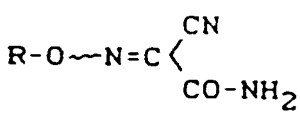 (XV)

in welcher

R die oben angegebene Bedeutung hat,

mit einer Base, gegebenenfalls in Gegenwart eines Verdünnungsmittels, umsetzt und die entstehenden Salze direkt oder gegebenenfalls nach Zwischenisolierung mit Verbindungen der Formel (XVI),

$R^1$-X (XVI)

in welcher

$R^1$ und X die oben angegebene Bedeutung haben,

gegebenenfalls in Gegenwart eines Verdünnungsmittels umsetzt.

**2.** Verfahren gemäß Anspruch 1, worin in der Formel (I)

$R^1$ für die Gruppierungen -A-COOR$^2$ oder -B-CONR$^3$R$^4$ steht,

A für eine geradkettige oder verzweigte Alkylenkette mit 1 bis 4 Kohlenstoffatomen steht,

B für eine geradkettige oder verzweigte Alkylenkette mit 1 bis 4 Kohlenstoffatomen steht,

$R^2$ für gegebenenfalls substituiertes, geradkettiges oder verzweigtes Alkyl mit 1 bis 6 Kohlenstoffatomen steht, wobei als Substituenten genannt seien: Halogen, gegebenenfalls ein-bis fünffach, gleich oder verschieden durch Halogen, Alkyl und Alkoxy mit

jeweils 1 bis 4 Kohlenstoffatomen substituiertes Phenyl und Cycloalkyl mit 3 bis 6 Kohlenstoffatomen; ferner steht $R^2$ für gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder für gegebenenfalls ein- bis fünffach, gleich oder verschieden durch Alkyl mit 1 bis 4 Kohlenstoffatomen substituiertes Cycloalkenyl mit 5 bis 7 Kohlenstoffatomen;

$R^3$ für Wasserstoff oder für gegebenenfalls ein- oder zweifach substituiertes, geradkettiges oder verzweigtes Alkyl mit 1 bis 4 Kohlenstoffatomen steht, wobei als Substituenten genannt seien: Halogen, Cyano, $-COOR^I$, $-CONR^{II}R^{III}$, $-NR^{II}R^{III}$, $-OR^{IV}$, $-S(O)_nR^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes Phenyl, gegebenenfalls ein- bis fünffach, gleich oder verschieden durch Halogen sowie Alkyl und Alkoxy mit jeweils 1 bis 4 Kohlenstoffatomen substituierter 5- oder 6-gliedriger Heterocyclus mit 1 bis 3 Heteroatomen, jeweils gegebenenfalls ein- bis fünffach, gleich oder verschieden durch Alkyl mit 1 bis 4 Kohlenstoffatomen substituiertes Cycloalkyl mit 3 bis 6 Kohlenstoffatomen oder Cycloalkenyl mit 5 bis 7 Kohlenstoffatomen; $R^3$ steht ferner für geradkettiges oder verzweigtes Alkenyl oder Alkinyl mit jeweils 2 bis 6 Kohlenstoffatomen; für gegebenenfalls ein- bis fünffach, gleich oder verschieden durch Alkyl mit 1 bis 4 Kohlenstoffatomen substituiertes Cycloalkenyl mit 5 bis 7 Kohlenstoffatomen oder für gegebenenfalls ein- bis fünffach, gleich oder verschieden substituiertes Cycloalkyl mit 3 bis 6 Kohlenstoffatomen, wobei als Substituenten genannt seien: Halogen, Alkyl und Alkoxy mit jeweils 1 bis 4 Kohlenstoffatomen, Cyano, Amino, Carbamoyl, Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im Alkoxyteil, Cycloalkyl und Cycloalkylalkyl mit 5 oder 6 Kohlenstoffatomen im Cycloalkylteil und 1 bis 4 Kohlenstoffatomen im geradkettigen oder verzweigten Alkylteil, gegebenenfalls ein- bis fünffach, gleich oder verschieden durch Halogen und Alkyl mit 1 bis 4 Kohlenstoffatomen substituiertes Phenyl und Pyrrolidon; $R^3$ steht weiterhin für gegebenenfalls ein- bis fünffach, gleich oder verschieden durch Halogen, Alkyl und Alkoxy mit jeweils 1 bis 4 Kohlenstoffatomen, Halogenalkyl oder Halogenalkoxy mit jeweils 1 oder 2 Kohlenstoffatomen und 1 bis 5 gleichen oder verschiedenen Halogenatomen substituiertes Phenyl oder für einen gegebenenfalls ein- bis fünffach, gleich oder verschieden substituierten 5- oder 6-gliedrigen Heterocyclus mit 1 bis 3 gleichen oder verschiedenen Heteroatomen, als Substituenten seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkylteil;

$R^4$ für die Bedeutungen von $R^3$, für polycyclische Carbocyclen oder die Gruppierung $-OR^{IV}$ steht,

oder

A und $R^2$ gemeinsam mit den Atomen, an die sie gebunden sind, für einen Ring der Formel

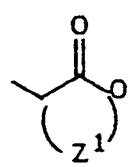

stehen, in welcher $Z^1$ für eine geradkettige oder verzweigte Alkylenkette mit 1 bis 4 Kohlenstoffatomen, steht,

oder

B und $R^3$ gemeinsam mit den Atomen, an die sie gebunden sind, für einen Ring der Formel

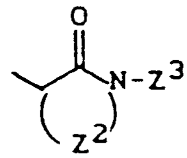

stehen, in welcher $Z^2$ für eine geradkettige oder verzweigte Alkylenkette mit 1 bis 4 Kohlenstoffatomen, und $Z^3$ für Wasserstoff oder Alkyl mit 1 bis 4 Kohlenstoffatomen stehen,
oder

$R^3$ und $R^4$ gemeinsam mit dem Stickstoffatom, an das sie gebunden sind, für einen gegebenenfalls ein- bis fünffach, gleich oder verschieden, substituierten mono-, bi- oder tricyclischen Heterocyclus oder Spiroheterocyclus mit ein bis drei weiteren, gleichen oder verschiedenen Heteroatomen stehen, und wobei als Substituenten genannt seien: geradkettiges oder verzweigtes Alkyl oder Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen in jedem Alkylteil, sowie jeweils gegebenenfalls ein- oder zweifach, gleich oder verschieden durch Halogen, geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

R für ein- oder zweifach substituiertes, geradkettiges oder verzweigtes Alkyl mit 1 bis 6 Kohlenstoffatomen steht, wobei als Substituenten genannt seien: Halogen, $-COOR^{I}$, $-CONR^{II}R^{III}$, $-OR^{IV}$, Acyl mit 2 bis 9 Kohlenstoffatomen, gegebenenfalls ein- bis fünffach, gleich oder verschieden durch Halogen und Alkyl mit 1 bis 4 Kohlenstoffatomen substituiertes 3- bis 6-gliedriges Heterocyclyl mit 1 bis 3 Heteroatomen, ausgenommen Azolyl; ferner steht R vorzugsweise für gegebenenfalls ein-bis fünffach, gleich oder verschieden durch Alkyl mit 1 bis 4 Kohlenstoffatomen substituiertes Cycloalkenyl mit 5 bis 7 Kohlenstoffatomen,

$R^{I}$ für Wasserstoff oder geradkettiges oder verzweigtes Alkyl mit 1 bis 6 Kohlenstoffatomen steht,

$R^{II}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil, wobei als Phenylsubstituenten genannt seien: Halogen, Alkyl und Alkoxy mit jeweils 1 bis 4 Kohlenstoffatomen, Halogenalkyl und Halogenalkoxy mit jeweils 1 oder 2 Kohlenstoffatomen und 2 bis 5 gleichen oder verschiedenen Halogenatomen; $R^{II}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen,

$R^{III}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten die bei $R^{II}$ genannten Phenylsubstituenten infrage kommen; $R^{III}$ steht ferner für Alkoxycarbonylalkyl, Carbamoylalkyl, Alkylcarbamoylalkyl oder Dialkylcarbamoylalkyl mit jeweils 1 bis 4 Kohlenstoffatomen in jedem Alkylteil oder für gegebenenfalls durch geradkettiges oder verzweigtes Alkyl mit 1 bis 4 Kohlenstoffatomen substituiertes Cycloalkyl mit 3 bis 6 Kohlenstoffatomen;

$R^{IV}$ für Wasserstoff, geradkettiges oder verzweigtes Alkyl mit 1 bis 6 Kohlenstoffatomen, gegebenenfalls ein- bis fünffach, gleich oder verschieden substituiertes Phenylalkyl mit 1 bis 4 Kohlenstoffatomen im geradkettigen oder verzweigten Alkylteil steht, wobei als Phenylsubstituenten die bei $R^{II}$ genannten Phenylsubstituenten infrage kommen; $R^{IV}$ steht ferner für Acyl mit 2 bis 9 Kohlenstoffatomen und

n für die Zahlen 0, 1 oder 2 steht,

deren geometrische und optische Isomeren und Isomerengemische.

**3.** Verfahren gemäß Anspruch 1, wobei in der allgemeinen Formel (I)

$R^1$ für die Gruppierungen $-A-COOR^2$ oder $-B-CONR^3R^4$ steht,

A für die Gruppierungen $-CH_2-$, $-CH(CH_3)-$ oder $-CH_2CH_2-$ steht,

B für die Gruppierungen $-CH_2-$, $-CH(CH_3)-$ oder $-CH_2CH_2-$ steht,

$R^2$ für geradkettiges oder verzweigtes Alkyl mit 1 bis 4 Kohlenstoffatomen steht,

$R^3$ für Wasserstoff oder für gegebenenfalls einoder zweifach, gleich oder verschieden substituiertes geradkettiges oder verzweigtes Alkyl mit 1 bis 4 Kohlenstoffatomen steht, wobei als Substituenten genannt seien: Halogen, Cyano, $-COOR^{I}$, $-CONR^{II}R^{III}$, $NR^{II}R^{III}$, $-OR^{IV}$, $-S(O)_nR^{IV}$, Acyl mit 2 bis 9 Kohlenstoffatomen, gegebenenfalls ein- bis dreifach, gleich oder verschieden durch Halogen und Alkyl mit 1 bis 4 Kohlenstoffatomen substituiertes Phenyl, gegebenenfalls ein- bis dreifach, gleich oder verschieden durch Halogen, geradkettiges oder verzweigtes Alkyl und Alkoxy mit jeweils 1 bis 4 Kohlenstoffatomen substituierter 5- oder 6-gliedrigen Heterocyclus mit 1 bis 3 gleichen oder verschiedenen Heteroatomen, und jeweils gegebenenfalls durch geradkettiges oder verzweigtes Alkyl mit 1 bis 4 Kohlenstoffatomen substituiertes Cycloalkyl mit 3 bis 6 Kohlenstoffatomen und Cycloalkenyl mit 5 bis 7 Kohlenstoffatomen; $R^3$ steht ferner für geradkettiges oder verzweigtes Alkenyl oder Alkinyl mit jeweils 2 bis 6 Kohlenstoffatomen, für gegebenenfalls durch geradkettiges oder verzweigtes Alkyl mit 1 bis 4 Kohlenstoffatomen substituiertes Cycloalkenyl mit 5 bis 7 Kohlenstoffatomen, für gegebenenfalls substituiertes Cycloalkyl mit 3 bis 6 Kohlenstoffatomen, wobei als Substituenten genannt seien: Halogen, geradkettiges oder verzweigtes Alkyl und Alkoxy mit jeweils 1 bis 4 Kohlenstoffatomen, Cyano, Amino, Carbamoyl, jeweils geradkettiges oder verzweigtes Alkylamino, Dialkylamino, Alkylcarbamoyl und Dialkylcarbamoyl mit jeweils 1 bis 4 Kohlenstoffatomen in jedem Alkylteil, Alkoxycarbonylamino mit 1 bis 4 Kohlenstoffatomen im geradkettigen oder verzweigten Alkoxyteil, Cycloalkyl und Cycloalkylalkyl mit jeweils 5 oder 6 Kohlenstoffatomen im Cycloalkylteil und 1 bis 4 Kohlenstoffatomen im geradkettigen oder verzweigten Alkylteil, gegebenenfalls ein- bis dreifach, gleich oder verschieden durch Halogen und Alkyl mit 1 bis 4 Kohlenstoffatomen substituiertes Phenyl und Pyrrolidon; $R^3$ steht weiterhin für gegebenenfalls ein- bis dreifach, gleich oder verschieden durch Halogen, geradkettiges oder verzweigtes Alkyl und Alkoxy mit jeweils 1 bis 4 Kohlenstoffatomen, Halogenalkyl und Halogenalkoxy mit jeweils 1 oder 2 Kohlenstoffatomen und 1 bis 5 gleichen oder verschiedenen Halogenatomen substituiertes Phenyl oder für einen gegebenenfalls ein - bis dreifach, gleich oder verschieden substituierten 5- oder 6-gliedrigen Heterocyclus mit 1 bis 3 gleichen oder verschiedenen Heteroatomen, als Substituenten für die Heterocyclen seien genannt: Halogen, Mercapto, Phenyl, geradkettiges oder verzweigtes Alkyl, Alkoxy, Alkylthio, Alkylsulfinyl und Alkylsulfonyl mit 1 bis 4 Kohlenstoffatomen je Alkyl;

$R^4$ für die Bedeutungen von $R^3$, für polycyclische Carbocyclen oder die Gruppierung $-OR^{IV}$ steht,

oder

A und $R^2$ gemeinsam mit den Atomen, an die sie gebunden sind, für gegebenenfalls durch Alkyl mit 1 bis 4 Kohlenstoffatomen substituierte 5- oder 6-gliedrige Ringe der Formeln

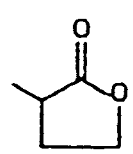 oder 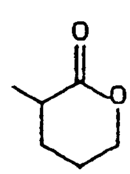 stehen,

oder

B und $R^3$ gemeinsam mit den Atomen, an die sie gebunden sind, für gegebenenfalls durch Alkyl mit 1 bis 4 Kohlenstoffatomen substituierte 5- bis 7-gliedrige Ringe der Formeln

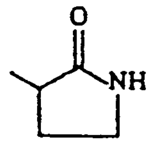, 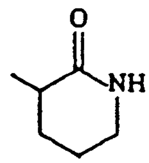 und 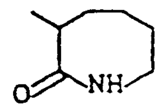

stehen,

oder

$R^3$ und $R^4$ gemeinsam mit dem Stickstoffatom, an das sie gebunden sind, für einen gegebenenfalls ein- bis fünffach, gleich oder verschieden substituierten mono-, bi- oder tricyclischen Heterocyclus oder Spiroheterocyclus stehen, wobei als Heterocyclen genannt seien: Oxazolidin, Pyrrolidin, Imidazolidin, Piperidin, Piperazin, Morpholin, Thiomorpholin, 1,3-Oxazan oder 1,3-Diazan; diese Heterocyclen können jeweils gegebenenfalls mit 1 oder 2 Benzol- oder Cyclohexanringen anelliert sein oder gegebenenfalls mit Methylen oder Ethylen überbrückt sein.

Als Substituenten für alle Heterosysteme seien genannt:

geradkettiges oder verzweigtes Alkyl oder Alkoxy mit jeweils 1 bis 4 Kohlenstoffatomen, Cycloalkyl mit 3 bis 6 Kohlenstoffatomen, die Hydroxy- oder die Oxo-Gruppe, geradkettiges oder verzweigtes Alkenyl mit 2 bis 4 Kohlenstoffatomen, Hydroxycarbonyl, Alkoxycarbonyl mit 1 bis 4 Kohlenstoffatomen im geradkettigen oder verzweigten Alkylteil, Carbamoyl, Alkyl- oder Dialkylcarbamoyl mit jeweils 1 bis 4 Kohlenstoffatomen in jedem Alkylteil, sowie jeweils gegebenenfals ein- oder zweifach, gleich oder verschieden durch Halogen oder geradkettiges oder verzweigtes Alkyl und Alkoxy mit jeweils 1 bis 4 Kohlenstoffatomen substituiertes Phenyl oder Phenylalkyl mit 1 bis 4 Kohlenstoffatomen im geradkettigen oder verzweigten Alkylteil,

R für substituiertes, geradkettiges oder verzweigtes Alkyl mit 1 bis 4 Kohlenstoffatomen steht, wobei als Substituenten genannt seien: Halogen, $-COOR^{I}$, $-CONR^{II}R^{III}$, $-OR^{IV}$, Acyl mit 2 bis 9 Kohlenstoffatomen, gegebenenfalls ein- bis dreifach, gleich oder verschieden durch Alkyl mit 1 oder 2 Kohlenstoffatomen substituierte Heterocyclen der Formeln

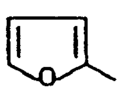, 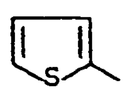, 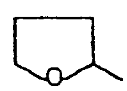, 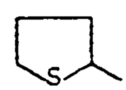,

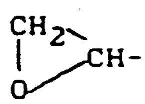, 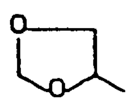 und 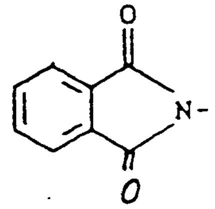,

R steht ferner für gegebenenfalls ein- bis dreifach durch Methyl substituiertes Cyclohexenyl oder Cyclopentenyl,

$R^{I}$ für geradkettiges oder verzweigtes Alkyl mit 1 bis 4 Kohlenstoffatomen steht,

$R^{II}$ für Wasserstoff, geradkettiges oder verzweigtes Alkyl mit 1 bis 4 Kohlenstoffatomen, jeweils gegebenenfalls ein- bis dreifach, gleich oder verschieden substituiertes Benzyl und Phenethyl steht, wobei als Phenylsubstituenten jeweils genannt seien: Fluor, Chlor, Methyl, Methoxy und Trifluormethyl; $R^{II}$ ferner für Alkoxycarbonylalkyl mit 1 bis 4 Kohlenstoffatomen im Alkoxyteil und 1 oder 2 Kohlenstoffatomen im Alkylteil, Carbamoylalkyl mit 1 oder 2 Kohlenstoffatomen im Alkylteil, Alkylcarbamoylalkyl und Dialkylcarbamoylalkyl mit jeweils 1 oder 2 Kohlenstoffatomen in jedem Alkylteil, oder für gegebenenfalls ein - bis dreifach, gleich oder verschieden durch Methyl und Ethyl

substituiertes Cycloalkyl mit 3 bis 6 Kohlenstoffatomen steht,

$R^{III}$ für die Bedeutungen von $R^{II}$ steht,

$R^{IV}$ für Wasserstoff, geradkettiges oder verzweigtes Alkyl mit 1 bis 4 Kohlenstoffatomen, jeweils gegebenenfalls ein - bis dreifach, gleich oder verschieden substituiertes Benzyl oder Phenethyl steht, wobei als Phenylsubstituenten jeweils genannt seien: Fluor, Chlor, Methyl, Methoxy und Trifluormethyl; $R^{IV}$ steht ferner für Acyl mit 2 bis 9 Kohlenstoffatomen und

n für die Zahlen 0, 1 oder 2 steht,

deren geometrische und optische Isomeren und Isomerengemische.

**4.** Verfahren gemäß Anspruch 1, wobei in der Formel (I)

$R^1$ für die Gruppierung -B-CO-$NR^3R^4$ steht,

B für die Gruppierungen -$CH_2$-, -$CH(CH_3)$- oder -$CH_2CH_2$- steht,

$R^3$ für Wasserstoff oder geradkettiges oder verzweigtes Alkyl mit 1 bis 4 Kohlenstoffatomen steht,

$R^4$ für Wasserstoff oder geradkettiges oder verzweigtes Alkyl mit 1 bis 4 Kohlenstoffatomen steht,

R für substituiertes Alkyl mit 1 oder 2 Kohlenstoffatomen steht, wobei als Substituenten genannt seien: Halogen, Alkylcarbonyl mit 1 bis 4 Kohlenstoffatomen im geradkettigen oder verzweigten Alkylteil, die Gruppierungen -$COOR^I$, -$CONR^{II}R^{III}$, -$OR^{IV}$ und die folgenden Heterocyclen:

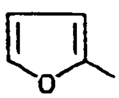 , 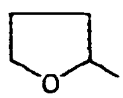 , 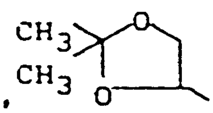 , 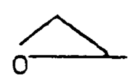 und

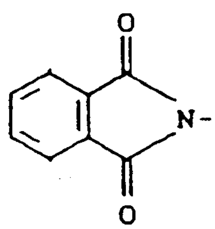 ;

R steht ferner für gegebenenfalls ein-bis dreifach durch Methyl substituiertes 1-Cyclohexenyl;

$R^I$ für Methyl, Ethyl, n- oder i-Propyl oder n-, i-, s-oder t-Butyl steht,

$R^{II}$ und $R^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor und Methyl als Substituenten, für Alkoxycarbonylalkyl mit 1 oder 2 Kohlenstoffatomen in jedem Alkylteil, für Carbamoylalkyl, Alkylcarbamoylalkyl oder Dialkylcarbamoylalkyl mit jeweils 1 oder 2 Kohlenstoffatomen in jedem Alkylteil oder für gegebenenfalls ein- bis dreifach durch Methyl substituiertes Cyclohexyl stehen, und

$R^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien, deren geometrische und optische Isomeren und Isomerengemische.

**5.** Verfahren gemäß Anspruch 1, wobei in der Formel (I)

$R^1$ für die Gruppierung -B-CO-$NR^3R^4$ steht,

B für die Gruppierungen -$CH_2$-, -$CH(CH_3)$- oder -$CH_2CH_2$- steht,

$R^3$ für Wasserstoff, geradkettiges oder verzweigtes Alky mit 1 bis 4 Kohlenstoffatomen oder ein- oder zweifach substituiertes Alkyl mit 1 bis 3 Kohlenstoffatomen steht, wobei als Substituenten genannt seien: Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl;

$R^3$ steht ferner für Allyl oder Propargyl, für 1-Cyclohexenyl oder für ein- bis dreifach, gleich oder verschieden substituiertes Cyclohexyl, wobei als Substituenten genannt seien: Chlor, Methyl, Ethyl, Methoxy, Cyano, Amino, Alkyl- oder Dialkylamino mit jeweils 1 oder 2 Kohlenstoffatomen in jedem Alkylteil, Carbamoyl, Alkyl- oder Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil oder 1-Pyrrolidin-2-on;
$R^3$ steht außerdem für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein- bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadiazol-5-yl oder für die Gruppierung

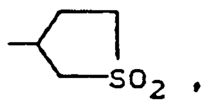

$R^4$ für ein- oder zweifach substituiertes Alkyl mit 1 bis 3 Kohlenstoffatomen steht, wobei als Substituenten genannt seien: Chlor, Hydroxy, Alkylcarbonyloxy mit 1 bis 4 Kohlenstoffatomen im geradkettigen oder verzweigten Alkylteil, Alkylcarbonyl mit 1 bis 4 Kohlenstoffatomen im geradkettigen oder verzweigten Alkylteil, Alkoxy und Alkylthio mit jeweils 1 oder 2 Kohlenstoffatomen, gegebenenfalls ein- bis dreifach, gleich oder verschieden durch Chlor und Methyl substituiertes Phenyl, 2-Furyl, 2-Pyridyl, 1-Morpholino, Cyclopropyl und Cyclohexyl;
$R^4$ steht ferner für Allyl oder Propargyl, für 1-Cyclohexenyl oder für ein- bis dreifach, gleich oder verschieden substituiertes Cyclohexyl, wobei als Substituenten genannt seien: Chlor, Methyl, Ethyl, Methoxy, Cyano, Amino, Alkyl-oder Dialkylamino mit jeweils 1 oder 2 Kohlenstoffatomen in jedem Alkylteil, Carbamoyl, Alkyl- oder Dialkylcarbamoyl mit jeweils 1 oder 2 Kohlenstoffatomen in jedem Alkylteil, Cyclohexyl, Cyclohexylalkyl mit 1 oder 2 Kohlenstoffatomen im Alkylteil und 1-Pyrrolidin-2-on;
$R^4$ steht außerdem für gegebenenfalls ein- bis dreifach, gleich oder verschieden durch Chlor und Methyl substituiertes Phenyl, für jeweils gegebenenfalls ein- bis dreifach durch Methyl substituiertes 2-Pyridyl oder 2-Pyrimidinyl, für gegebenenfalls durch Phenyl substituiertes 2-Thiazolyl, für gegebenenfalls ein- bis dreifach, gleich oder verschieden durch Chlor, Methyl und Methoxy substituiertes 2-Benzothiazolyl, für 1,2,4-Triazol-3-yl, für gegebenenfalls durch Phenyl substituiertes 1,2,4-Thiadiazol-5-yl, für gegebenenfalls ein-bis dreifach, gleich oder verschieden durch Mercapto, geradkettiges oder verzweigtes Alkyl mit 1 bis 4 Kohlenstoffatomen, jeweils geradkettiges oder verzweigtes Alkythio, Alkylsulfinyl und Alkylsulfonyl mit jeweils 1 bis 4 Kohlenstoffatomen substituiertes 1,3,4-Thiadiazol-5-yl oder für die Gruppierung

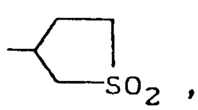

$R^4$ steht weiterhin für Hydroxy, Alkoxy mit 1 oder 2 Kohlenstoffatomen, für gegebenenfalls ein- bis dreifach, gleich oder verschieden durch Chlor und Methyl substituiertes Benzyloxy sowie für 1-Adamantyl, 2-Norbornyl, 1- oder 2-Decalyl oder 1- oder 2-Tetralyl;

R für substituiertes Alkyl mit 1 oder 2 Kohlenstoffatomen steht, wobei als Substituenten genannt seien: Halogen, Alkylcarbonyl mit 1 bis 4 Kohlenstoffatomen im Alkylteil, die Gruppierungen $-COOR^{I}$, $-CONR^{II}R^{III}$, $OR^{IV}$ oder die folgenden Heterocyclen:

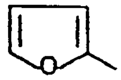 , 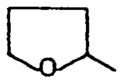 , 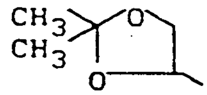 , 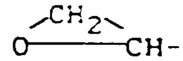

und 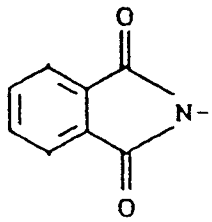 ;

R steht ferner für jeweils gegebenenfalls ein- bis dreifach durch Methyl substituiertes 1-Cyclohexenyl,

$R^I$ für Methyl, Ethyl, n- oder i-Propyl oder n-, i-, s-oder t-Butyl steht,

$R^{II}$ und $R^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor oder Methyl als Substituenten, für Alkoxycarbonylalkyl mit 1 oder 2 Kohlenstoffatomen in jedem Alkylteil, für Carbamoylalkyl, Alkylcarbamoylalkyl oder Dialkylcarbamoylalkyl mit jeweils 1 oder 2 Kohlenstoffatomen in jedem Alkylteil oder für gegebenenfalls ein- bis dreifach durch Methyl substituiertes Cyclohexyl stehen, und

$R^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien,

deren geometrische und optische Isomeren und Isomerengemische.

**6.** Verfahren gemäß Anspruch 1, wobei in der Formel (I)

$R^1$ für die Grupierung $-B-CO-NR^3R^3$ steht,

B für die Gruppierungen $-CH_2-$, $-CH(CH_3)-$ oder $-CH_2CH_2-$ steht,

$R^3$ und $R^4$ gemeinsam mit dem Stickstoffatom, an das sie gebunden sind, für folgende mono-, bi- oder tricyclischen Heterocyclen oder Spiroheterocyclen stehen:

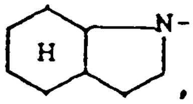 ,
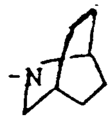 ,
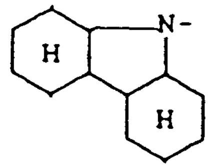 ,

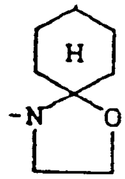 ,
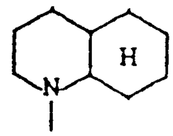 ,

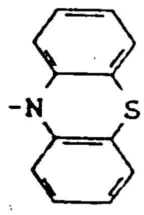 ,
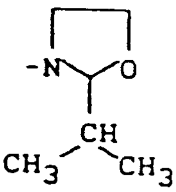 ,
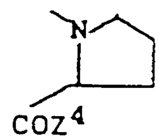

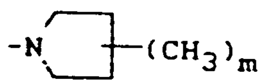 ,
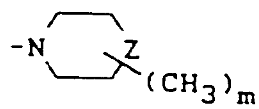

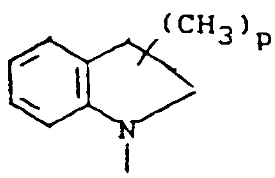 ,
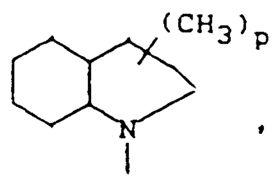 ,

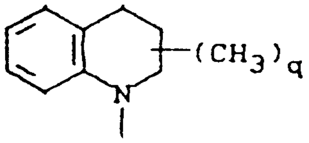 , 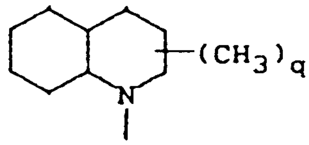 ,

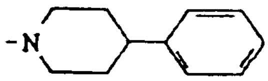 , 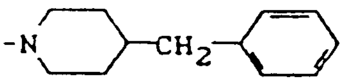 ,

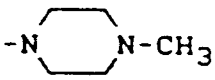 , 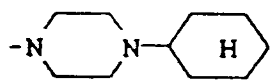 , 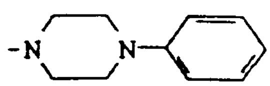 ,

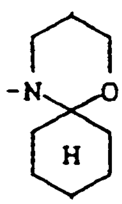 , 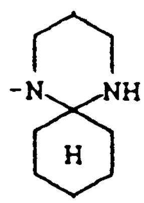 , 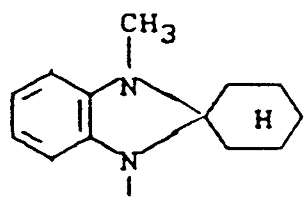 ,

wobei

- Z für Sauerstoff oder die $CH_2$-Gruppe steht,
- $Z^4$ für Hydroxy, Methoxy, Ethoxy, Amino, Methylamino, Ethylamino, Dimethylamino oder Ethylmethylamino steht,
- m für die Zahlen 0, 1, 2, 3 oder 4 steht,
- p für die Zahlen 0, 1 oder 2 steht, und
- q für die Zahlen 0, 1, 2 oder 3 steht und
- R für substituiertes Alkyl mit 1 oder 2 Kohlenstoffatomen steht, wobei als Substituenten genannt seien: Halogen, Alkylcarbonyl mit 1 bis 4 Kohlenstoffatomen im geradkettigen oder verzweigten Alkylteil, $-COOR^I$, $-CONR^{II}R^{III}$, $OR^{IV}$ und die folgenden Heterocyclen:

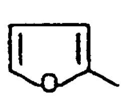 , 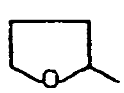 , 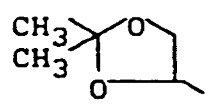 , 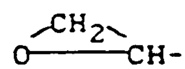

und 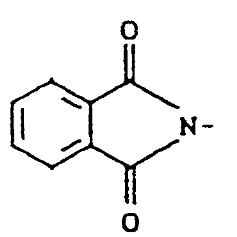 ;

R steht ferner für jeweils gegebenenfalls ein- bis dreifach durch Methyl substituiertes 1-Cyclohexenyl

- $R^I$ für Methyl, Ethyl, n- oder i-Propyl oder n-, i-, s-oder t-Butyl steht,
- $R^{II}$ und $R^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor und Methyl als Substituenten, für Alkoxycarbonylalkyl mit 1 oder 2 Kohlenstoffatomen in jedem Alkylteil, für Carbamoylalkyl, Alkylcarbamoylalkyl oder

Dialkylcarbamoylalkyl mit jeweils 1 oder 2 Kohlenstoffatomen in jedem Alkylteil oder für gegebenenfalls ein- bis dreifach durch Methyl substituiertes Cyclohexyl stehen und

$R^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien,

deren geometrische und optische Isomeren und Isomerengemische.

**7.** Verfahren gemäß Anspruch 1, worin in der Formel (I)

$R^1$ für die Gruppierung -A-COOR$^2$ steht,

A für die Gruppierungen $-CH_2-$, $-CH(CH_3)-$ oder $-CH_2CH_2-$ steht,

$R^2$ für Methyl oder Ethyl steht,

R für ein- oder zweifach substituiertes Alkyl mit 1 oder 2 Kohlenstoffatomen steht, wobei als Substituenten genannt seien: Halogen, Alkylcarbonyl mit 1 bis 4 Kohlenstoffatomen im geradkettigen oder verzweigten Alkylteil, -COOR$^I$, -CONR$^{II}$R$^{III}$, OR$^{IV}$ und die folgenden Heterocyclen:

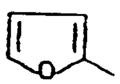 , 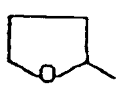 , 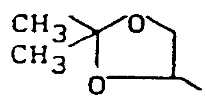 , 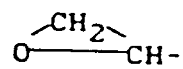

und 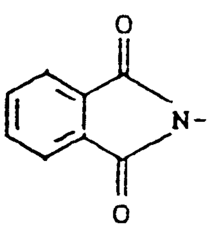 ;

R steht ferner für jeweils gegebenenfalls ein- bis dreifach durch Methyl substituiertes 1-Cyclohexenyl,

$R^I$ für Methyl, Ethyl, n- oder i-Propyl, n-, i-, s-oder t-Butyl steht,

$R^{II}$ und $R^{III}$ gleich oder verschieden sind und für Wasserstoff, Methyl, Ethyl, n- oder i-Propyl stehen, für gegebenenfalls ein- bis dreifach, gleich oder verschieden substituiertes Benzyl mit Chlor und Methyl als Substituenten, für Alkoxycarbonylalkyl mit 1 oder 2 Kohlenstoffatomen in jedem Alkylteil, für Carbamoylalkyl, Alkylcarbamoylalkyl oder Dialkylcarbamoylalkyl mit jeweils 1 oder 2 Kohlenstoffatomen in jedem Alkylteil oder für gegebenenfalls ein- bis dreifach durch Methyl substituiertes Cyclohexyl stehen und

$R^{IV}$ für Wasserstoff, Alkyl mit 1 oder 2 Kohlenstoffatomen oder gegebenenfalls ein- bis dreifach, gleich oder verschieden substituiertes Benzyl steht, wobei als Phenylsubstituenten Fluor, Chlor und Methyl genannt seien,

deren geometrische und optische Isomeren und Isomerengemische.

**8.** Schädlingsbekämpfungsmittel, gekennzeichnet durch einen Gehalt an mindestens einem (2-Cyano-2-oximino-acetyl)-aminosäure-Derivat der Formel (I) nach den Ansprüchen 1 bis 7.

**9.** Verwendung von (2-Cyano-2-oximino-acetyl)-aminosäure-Derivaten der Formel (I) nach den Ansprüchen 1 bis 7 zur Bekämpfung von Schädlingen.

**10.** Verfahren zur Bekämpfung von Schädlingen, dadurch gekennzeichnet, daß man (2-Cyano-2-oximino-acetyl)-aminosäure-Derivate der Formel (I) nach den Ansprüchen 1 bis 7 auf Schädlinge und/oder ihren Lebensraum einwirken läßt.

**11.** Verfahren zur Herstellung von Schädlingsbekämpfungsmitteln, dadurch gekennzeichnet, daß man (2-Cyano-2-oximino-acetyl)-aminosäure-Derivate der Formel (I) nach den Ansprüchen 1 bis 7 mit Streckmitteln und/oder oberflächenaktiven Mitteln vermischt.

## Claims

**Claims for the following Contracting States : AT, BE, CH, DE, FR, GB, GR, IT, LI, NL**

**1.** (2-Cyano-2-oximinoacetyl)-amino acid derivatives of the general formula (I)

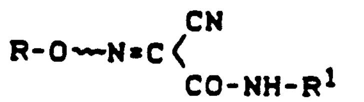

(I)

in which

$R^1$ represents the groupings -A-COOR$^2$ or -B-CONR$^3$R$^4$,

A represents an optionally substituted straight or branched alkylene chain,

B represents an optionally substituted straight or branched alkylene chain,

$R^2$ represents optionally substituted alkyl, with the following substituents: halogen, hydroxyl, alkoxy, acyloxy, optionally substituted aryl, optionally substituted cycloalkyl and optionally substituted heterocyclyl; or $R^2$ furthermore represents optionally substituted cycloalkyl or optionally substituted cycloalkenyl,

$R^3$ represents hydrogen, or represents optionally substituted alkyl, with the following substituents: halogen, cyano, -COOR$^I$, -CONR$^{II}$R$^{III}$, -NR$^{II}$R$^{III}$, -OR$^{IV}$, -S(O)$_n$R$^{IV}$,acyl and in each case optionally substituted aryl, heterocyclyl, cycloalkyl and cycloalkenyl; or $R^3$ furthermore represents in each case optionally substituted alkenyl, alkinyl, cycloalkyl, cycloalkenyl, aryl or heterocyclyl,

$R^4$ represents hydrogen, or represents optionally substituted alkyl, with the following substituents: halogen, cyano, -COOR$^I$, -CONR$^{II}$R$^{III}$, -NR$^{II}$R$^{III}$, -OR$^{IV}$, -S(O)$_n$R$^{IV}$, acyl and in each case optionally substituted aryl, heterocyclyl, cycloalkyl and cycloalkenyl; or $R^4$ furthermore represents in each case optionally substituted alkenyl, alkinyl, cycloalkyl, cycloalkenyl, aryl, heterocyclyl or polycyclic carbocyclic radicals or the grouping -OR$^{IV}$,

or

A and $R^2$, together with the atoms to which they are bonded, represent an optionally substituted ring,

or

B and $R^3$, together with the atoms to which they are bonded, represent an optionally substituted ring,

or

$R^3$ and $R^4$, together with the nitrogen atom to which they are bonded, represent an optionally substituted heterocyclic radical, which can contain further hetero atoms,

R represents substituted alkyl, with the following substituents: halogen, -COOR$^I$, -COOR$^{II}$R$^{III}$, -OR$^{IV}$, acyl and optionally substituted heterocyclyl, excluding azolyl; or R furthermore represents optionally substituted cycloalkenyl;

$R^I$ represents hydrogen or optionally substituted alkyl, with the following substituents: halogen and in each case optionally substituted aryl, cycloalkyl and cycloalkenyl,

$R^{II}$ and $R^{III}$ are identical or different and represent hydrogen or optionally substituted alkyl, with the following substituents: halogen, -COOR$^{IV}$, -CONR$^I$R$^{IV}$ and in each optionally substituted aryl, cycloalkyl and cycloalkenyl; or $R^{II}$ and $R^{III}$ furthermore represent alkenyl, alkinyl or in each case optionally substituted cycloalkyl, cycloalkenyl, aryl or heterocyclyl, or, together with the nitrogen atom to which they are bonded, represent an optionally substituted heterocyclic radical, which can contain further hetero atoms,

$R^{IV}$ represents hydrogen, alkyl, aralkyl or acyl and

n represents the number 0, 1 or 2,

and their geometric and optical isomers and isomer mixtures.

**2.** (2-Cyano-2-oximinoacetyl)-amino acid derivatives according to Claim 1, wherein, in the formula (I),

$R^1$ represents the groupings -A-COOR$^2$ or -B-CONR$^3$R$^4$,

A represents a straight or branched alkylene chain with 1 to 4 carbon atoms,

B represents a straight or branched alkylene chain with 1 to 4 carbon atoms,

$R^2$ represents optionally substituted straight-chain or branched alkyl with 1 to 6 carbon atoms, substituents which may be mentioned being: halogen, and phenyl and cycloalkyl with 3 to 6 carbon atoms which are optionally substituted by one to five identical or different substituents from the group comprising halogen, alkyl and alkoxy with in each case 1 to 4 carbon atoms; or $R^2$ furthermore preferably represents cycloalkyl which has 3 to 6 carbon atoms and is optionally substituted by one to five identical or different alkyl substituents with 1 to 4 carbon atoms, or represents cycloalkenyl which has 5 to 7 carbon atoms and is optionally substituted by one to five identical or different alkyl substituents with 1 to 4 carbon atoms;

$R^3$ represents hydrogen, or represents straight-chain or branched alkyl which has 1 to 4 carbon atoms and is optionally substituted by one or two substituents, substituents which may be mentioned being: halogen, cyano, $-COOR^{I}$, $COOR^{II}R^{III}$, $NR^{II}R^{III}$, $-OR^{IV}$, $-S(O)_nR^{IV}$, acyl with 2 to 9 carbon atoms, phenyl which is optionally substituted by one to five identical or different substituents from the group comprising halogen and alkyl with 1 to 4 carbon atoms, a 5- or 6-membered heterocyclic radical which has 1 to 3 hetero atoms, and is optionally substituted by one to five identical or different substituents from the group comprising halogen and alkyl and alkoxy with in each case 1 to 4 carbon atoms, and cycloalkyl with 3 to 6 carbon atoms and cycloalkenyl with 5 to 7 carbon atoms, in each case optionally substituted by one to five identical or different alkyl substituents with 1 to 4 carbon atoms; or $R^3$ furthermore represents straight-chain or branched alkenyl or alkinyl with in each case 2 to 6 carbon atoms; or represents cycloalkenyl which has 5 to 7 carbon atoms and is optionally substituted by one to five identical or different alkyl substituents with 1 to 4 carbon atoms, or represents cycloalkyl which has 3 to 6 carbon atoms and is optionally substituted by one to five identical or different substituents, substituents which may be mentioned being: halogen, alkyl and alkoxy with in each case 1 to 4 carbon atoms, cyano, amino, carbamoyl, alkylamino, dialkylamino, alkylcarbamoyl and di-alkylcarbamoyl with in each case 1 to 4 carbon atoms in each alkyl part, alkoxycarbonylamino with 1 to 4 carbon atoms in the alkoxy part, cycloalkyl and cycloalkylalkyl with 5 or 6 carbon atoms in the cycloalkyl part and 1 to 4 carbon atoms in the straight-chain or branched alkyl part, and phenyl and pyrrolidone which are optionally substituted by one to five identical or different substituents from the group comprising halogen and alkyl with 1 to 4 carbon atoms; or $R^3$ furthermore represents phenyl which is optionally substituted by one to five identical or different substituents from the group comprising halogen, alkyl and alkoxy with in each case 1 to 4 carbon atoms and halogenoalkyl and halogenoalkoxy with in each case 1 or 2 carbon atoms and 1 to 5 identical or different halogen atoms, or represents a 5- or 6-membered heterocyclic radical which has 1 to 3 identical or different hetero atoms, and is optionally substituted by one to five identical or different substituents; substituents which may be mentioned being: halogen, mercapto, phenyl and straight-chain or branched alkyl, alkoxy, alkylthio, alkylsulphinyl and alkylsulphonyl with 1 to 4 carbon atoms per alkyl part;

$R^4$ has the meanings of $R^3$, or represents polycyclic carbocyclic radicals or the grouping $-OR^{IV}$,

or

A and $R^2$, together with the atoms to which they are bonded, represent a ring of the formula

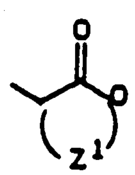

in which $Z^1$ represents a straight or branched alkylene chain with 1 to 4 carbon atoms,

or

B and $R^3$, together with the atoms to which they are bonded, represent a ring of the formula

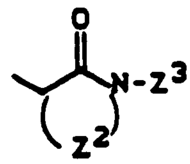

in which $Z^2$ represents a straight or branched alkylene chain with 1 to 4 carbon atoms and $Z^3$ represents hydrogen or alkyl with 1 to 4 carbon atoms,

or

$R^3$ and $R^4$, together with the nitrogen atom to which they are bonded, represent a mono-, bi- or tricyclic heterocyclic radical or spiroheterocyclic radical which has one to three other identical or different hetero atoms, and is optionally substituted by one to five identical or different substituents, substituents which may be mentioned being: straight-chain or branched alkyl or alkoxy with in each case 1 to 4 carbon atoms, cycloalkyl with 3 to 6 carbon atoms, the hydroxyl and the oxo group, straight-chain or branched alkenyl with 2 to 4 carbon atoms, hydroxycarbonyl, alkoxycarbonyl with 1 to 4 carbon atoms in the straight-chain or branched alkyl part, carbamoyl, alkyl- and dialkylcarbamoyl with in each case 1 to 4 carbon atoms in each alkyl part and phenyl and phenylalkyl with 1 to 4 carbon atoms in the straight-chain or branched alkyl part, in each case optionally substituted by one or two identical or different substituents from the group comprising halogen and straight-chain or branched alkyl and alkoxy with in each case 1 to 4 carbon atoms,

R represents straight-chain or branched alkyl which has 1 to 6 carbon atoms and is substituted by one or two substituents, substituents which may be mentioned being: halogen, $-COOR^I$, $-CONR^{II}R^{III}$, $-OR^{IV}$, acyl with 2 to 9 carbon atoms and 3- to 6-membered heterocyclyl which has 1 to 3 hetero atoms, excluding azolyl, and is optionally substituted by one to five identical or different substituents from the group comprising halogen and alkyl with 1 to 4 carbon atoms; or R furthermore preferably represents cycloalkenyl which has 5 to 7 carbon atoms and is optionally substituted by one to five identical or different substituents from the group comprising alkyl with 1 to 4 carbon atoms,

$R^I$ represents hydrogen or straight-chain or branched alkyl with 1 to 6 carbon atoms,

$R^{II}$ represents hydrogen, straight-chain or branched alkyl with 1 to 6 carbon atoms, phenylalkyl which has 1 to 4 carbon atoms in the straight-chain or branched alkyl part and is optionally substituted by one to five identical or different substituents, possible substituents on the phenyl which may be mentioned being: halogen, alkyl and alkoxy with in each case 1 to 4 carbon atoms and halogenoalkyl and halogenoalkoxy with in each case 1 or 2 carbon atoms and 2 to 5 identical or different halogen atoms; or $R^{II}$ furthermore represents alkoxycarbonylalkyl, carbamoylalkyl, alkylcarbamoylalkyl or dialkylcarbamoylalkyl with in each case 1 to 4 carbon atoms in each alkyl part, or represents cycloalkyl which has 3 to 6 carbon atoms and is optionally substituted by straight-chain or branched alkyl with 1 to 4 carbon atoms,

$R^{III}$ represents hydrogen, straight-chain or branched alkyl with 1 to 6 carbon atoms, or phenylalkyl which has 1 to 4 carbon atoms in the straight-chain or branched alkyl part and is optionally substituted by one to five identical or different substituents, possible substituents on the phenyl being the substituents on the phenyl mentioned for $R^{II}$; $R^{III}$ furthermore represents alkoxycarbonylalkyl, carbamoylalkyl, alkylcarbamoylalkyl or dialkylcarbamoylalkyl with in each case 1 to 4 carbon atoms in each alkyl part, or represents cycloalkyl which has 3 to 6 carbon atoms and is optionally substituted by straight-chain or branched alkyl with 1 to 4 carbon atoms;

$R^{IV}$ represents hydrogen, straight-chain or branched alkyl with 1 to 6 carbon atoms or phenylalkyl which has 1 to 4 carbon atoms in the straight-chain or branched alkyl part and is optionally substituted by one to five identical or different substituents, possible substituents on the phenyl being the substituents on the phenyl mentioned for $R^{II}$; or $R^{IV}$ furthermore represents acyl with 2 to 9 carbon atoms and

n represents the number 0, 1 or 2,

and their geometric and optical isomers and isomer mixtures.

**3.** (2-Cyano-2-oximinoacetyl)-amino acid derivatives according to Claim 1, wherein, in the general formula (I)

$R^1$ represents the groupings $-A-COOR^2$ or $-B-CONR^3R^4$,

A represents the groupings $-CH_2-$, $-CH(CH_3)-$ or $-CH_2CH_2-$,

B represents the groupings $-CH_2-$, $-CH(CH_3)-$ or $-CH_2CH_2-$,

$R^2$ represents straight-chain or branched alkyl with 1 to 4 carbon atoms,

$R^3$ represents hydrogen, or represents straight-chain or branched alkyl which has 1 to 4 carbon atoms and is optionally substituted by one or two identical or different substituents, substituents which may be mentioned being: halogen, cyano, $-COOR^I$, $-CONR^{II}R^{III}$, $NR^{II}R^{III}$,$-OR^{IV}$, $-S(O)_nR^{IV}$, acyl with 2 to 9 carbon atoms, phenyl which is optionally substituted by one to three identical or different substituents from the group comprising halogen and alkyl with 1 to 4 carbon atoms, a 5- or 6-membered heterocyclic radical which has 1 to 3 identical or different hetero atoms, and is optionally substituted by one to three identical or different substituents from the group comprising halogen and straight-chain or branched alkyl and alkoxy with in each case 1 to 4 carbon atoms, and cycloalkyl with 3 to 6 carbon atoms and cycloalkenyl with 5 to 7 carbon atoms, in each case optionally substituted by straight-chain or branched alkyl with 1 to 4 carbon atoms; or $R^3$ furthermore represents straight-chain or branched alkenyl or alkinyl with in each case 2 to 6 carbon atoms, or represents cycloalkenyl which has 5 to 7 carbon atoms and is optionally substituted by straight-chain or branched alkyl with 1 to 4 carbon atoms, or represents optionally substituted cycloalkyl with 3 to 6 carbon atoms, substituents which may be mentioned being: halogen, straight-chain or branched alkyl and alkoxy with in each case 1 to 4 carbon atoms, cyano, amino, carbamoyl, in each case straight-chain or branched alkylamino, dialkylamino, alkylcarbamoyl and dialkylcarbamoyl with in each case 1 to 4 carbon atoms in each alkyl part, alkoxycarbonylamino with 1 to 4 carbon atoms in the straight-chain or branched alkoxy part, cycloalkyl and cycloalkylalkyl with in each case 5 or 6 carbon atoms in the cycloalkyl part and 1 to 4 carbon atoms in the straight-chain or branched alkyl part, and phenyl and pyrrolidone, optionally substituted by one to three identical or different substituents from the group comprising halogen and alkyl with 1 to 4 carbon atoms; or $R^3$ furthermore represents phenyl which is optionally substituted by one to three identical or different substituents from the group comprising halogen, straight-chain or branched alkyl and alkoxy with in each case 1 to 4 carbon atoms and halogenoalkyl and halogenoalkoxy with in each case 1 or 2 carbon atoms and 1 to 5 identical or different halogen atoms, or represents a 5- or 6-membered heterocyclic radical which has 1 to 3 hetero atoms, and is optionally substituted by one to three identical or different substituents; substituents which may be mentioned for the heterocyclic radicals are: halogen, mercapto, phenyl and straight-chain or branched alkyl, alkoxy, alkylthio, alkylsulphinyl and alkylsulphonyl with 1 to 4 carbon atoms per alkyl;

$R^4$ has the meanings of $R^3$, or represents polycyclic carbocyclic radicals or the grouping $-OR^{IV}$,

or

A and $R^2$, together with the atoms to which they are bonded, represent 5- or 6-membered rings of the formulae

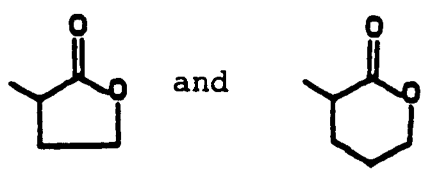

and

which are optionally substituted by alkyl with 1 to 4 carbon atoms,

or

B and $R^3$, together with the atoms to which they are bonded, represent 5- to 7-membered rings of the formulae

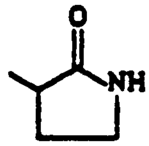, 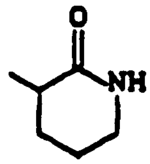 and 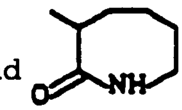

which are optionally substituted by alkyl with 1 to 4 carbon atoms,

or

$R^3$ and $R^4$, together with the nitrogen atom to which they are bonded, represent a mono-, bi- or tricyclic heterocyclic or spiroheterocyclic radical which is optionally substituted by one to five identical or different substituents, heterocyclic radicals which may be mentioned being: oxazolidine, pyrrolidine, imidazolidine, piperidine, piperazine, morpholine, thiomorpholine, 1,3-oxazane and 1,3-diazane; these heterocyclic radicals can in each case be optionally fused to 1 or 2 benzene or cyclohexane rings or optionally bridged with methylene or ethylene.

Substituents which may be mentioned for all the hetero systems are:

straight-chain or branched alkyl or alkoxy with in each case 1 to 4 carbon atoms, cycloalkyl with 3 to 6 carbon atoms, the hydroxyl and the oxo group, straight-chain or branched alkenyl with 2 to 4 carbon atoms, hydroxycarbonyl, alkoxycarbonyl with 1 to 4 carbon atoms in the straight-chain or branched alkyl part, carbamoyl, alkyl- and dialkylcarbamoyl with in each case 1 to 4 carbon atoms in each alkyl part and phenyl and phenylalkyl with 1 to 4 carbon atoms in the straight-chain or branched alkyl part, in each case optionally substituted by one or two identical or different substituents from the group comprising halogen and straight-chain or branched alkyl and alkoxy with in each case 1 to 4 carbon atoms,

R represents substituted straight-chain or branched alkyl with 1 to 4 carbon atoms, preferred substituents which may be mentioned being: halogen, $-COOR^I$, $-CONR^{II}R^{III}$, $-OR^{IV}$, acyl with 2 to 9 carbon atoms and heterocyclic radicals of the formulae

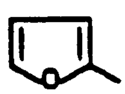, 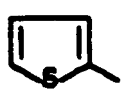, 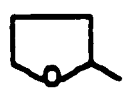, 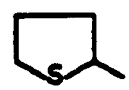,

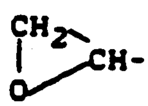, 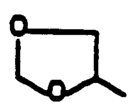 and 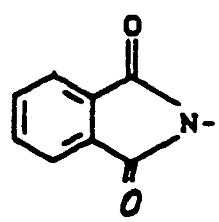,

which are optionally substituted by one to three identical or different alkyl substituents with 1 or 2 carbon atoms, or R furthermore represents cyclohexenyl or cyclopentenyl, optionally substituted by one to three methyl substituents,

$R^I$ represents straight-chain or branched alkyl with 1 to 4 carbon atoms,

$R^{II}$ represents hydrogen, straight-chain or branched alkyl with 1 to 4 carbon atoms or benzyl or phenethyl, in each case optionally substituted by one to three identical or different substituents, substituents on the phenyl which may be mentioned in each case being: fluorine, chlorine, methyl, methoxy and trifluoromethyl; or $R^{II}$ furthermore represents alkoxycarbonylalkyl with 1 to 4 carbon atoms in the alkoxy part and 1 or 2 carbon atoms in the alkyl part, carbamoylalkyl with 1 or 2 carbon atoms in the alkyl

part or alkylcarbamoylalkyl or dialkylcarbamoylalkyl with in each case 1 or 2 carbon atoms in each alkyl part, or represents cycloalkyl which has 3 to 6 carbon atoms and is optionally substituted by one to three identical or different substituents from the group comprising methyl and ethyl,

$R^{III}$ has the meanings of $R^{II}$,

$R^{IV}$ represents hydrogen, straight-chain or branched alkyl with 1 to 4 carbon atoms or benzyl or phenethyl, in each case optionally substituted by one to three identical or different substituents, substituents on the phenyl which may be mentioned in each case being: fluorine, chlorine, methyl, methoxy and trifluoromethyl; or $R^{IV}$ furthermore represents acyl with 2 to 9 carbon atoms, and

n represents the number 0, 1 or 2,

and their geometric and optical isomers and isomer mixtures.

**4.** (2-Cyano-2-oximinoacetyl)-amino acid derivatives according to Claim 1, wherein, in the formula (I),

$R^1$ represents the grouping $-B-CO-NR^3R^4$,

B represents the groupings $-CH_2-$, $-CH(CH_3)-$or $-CH_2CH_2-$,

$R^3$ represents hydrogen or straight-chain or branched alkyl with 1 to 4 carbon atoms,

$R^4$ represents hydrogen or straight-chain or branched alkyl with 1 to 4 carbon atoms,

R represents substituted alkyl with 1 or 2 carbon atoms, substituents which may be mentioned being: halogen, alkylcarbonyl with 1 to 4 carbon atoms in the straight-chain or branched alkyl part, the groupings $-COOR^I$, $-CONR^{II}R^{III}$ and $-OR^{IV}$ and the following heterocyclic radicals:

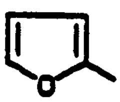 , 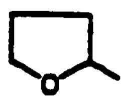 , 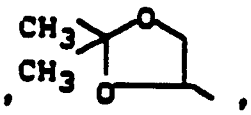 , 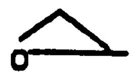 and

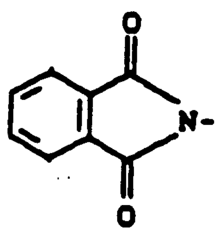 ;

or R furthermore represents 1-cyclohexenyl which is optionally substituted by one to three methyl substituents;

$R^I$ represents methyl, ethyl, n- or i-propyl or n-, i-, s- or t-butyl,

$R^{II}$ and $R^{III}$ are identical or different and represent hydrogen, methyl, ethyl, n- or i-propyl, or represent benzyl which is optionally substituted by one to three identical or different substituents, with chlorine and methyl as substituents, or represent alkoxycarbonylalkyl with 1 or 2 carbon atoms in each alkyl part, or represent carbamoylalkyl, alkylcarbamoylalkyl or dialkylcarbamoylalkyl with in each case 1 or 2 carbon atoms in each alkyl part, or represent cyclohexyl which is optionally substituted by one to three methyl substituents and

$R^{IV}$ represents hydrogen, alkyl with 1 or 2 carbon atoms or benzyl which is optionally substituted by one to three identical or different substituents, substituents on the phenyl which may be mentioned being fluorine, chlorine and methyl,

and their geometric and optical isomers and isomer mixtures.

**5.** (2-Cyano-2-oximinoacetyl)-amino acid derivatives according to Claim 1, wherein, in the formula (I),

$R^1$ represents the grouping $-B-CO-NR^3R^4$,

B represents the groupings $-CH_2-$, $-CH(CH_3)-$ or $-CH_2CH_2-$,

$R^3$ represents hydrogen, straight-chain or branched alkyl with 1 to 4 carbon atoms or alkyl which has 1 to 3 carbon atoms and is substituted by one or two substituents, substituents which

may be mentioned being: chlorine, hydroxyl, alkylcarbonyloxy with 1 to 4 carbon atoms in the straight-chain or branched alkyl part, alkylcarbonyl with 1 to 4 carbon atoms in the straight-chain or branched alkyl part, alkoxy and alkylthio with in each case 1 or 2 carbon atoms and phenyl, 2-furyl, 2-pyridyl, 1-morpholino, cyclopropyl and cyclohexyl which are optionally substituted by one to three identical or different substituents from the group comprising chlorine and methyl; or $R^3$ furthermore represents allyl or propargyl, or represents 1-cyclohexenyl, or represents cyclohexyl which is substituted by one to three identical or different substituents, substituents which may be mentioned being: chlorine, methyl, ethyl, methoxy, cyano, amino, alkyl- and dialkylamino with in each case 1 or 2 carbon atoms in each alkyl part, carbamoyl, alkyl- and dialkylcarbamoyl with in each case 1 or 2 carbon atoms in each alkyl part, cyclohexyl, cyclohexylalkyl with 1 or 2 carbon atoms in the alkyl part and 1-pyrrolidin-2-one; or

$R^3$ also represents phenyl which is optionally substituted by one to three identical or different substituents from the group comprising chlorine and methyl, or represents 2-pyridyl or 2-pyrimidinyl, in each case optionally substituted by one to three methyl substituents, or represents 2-thiazolyl which is optionally substituted by phenyl, or represents 2-benzothiazolyl which is optionally substituted by one to three identical or different substituents from the group comprising chlorine, methyl and methoxy, or represents 1,2,4-triazol-3-yl, or represents 1,2,4-thiadiazol-5-yl which is optionally substituted by phenyl, or represents 1,3,4-thiadiazol-5-yl which is optionally substituted by one to three identical or different substituents from the group comprising mercapto, straight-chain or branched alkyl with 1 to 4 carbon atoms and in each case straight-chain or branched alkylthio, alkylsulphinyl and alkylsulphonyl with in each case 1 to 4 carbon atoms, or represents the grouping

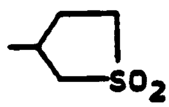 ,

$R^4$ represents alkyl which has 1 to 3 carbon atoms and is substituted by one or two substituents, substituents which may be mentioned being: chlorine, hydroxyl, alkylcarbonyloxy with 1 to 4 carbon atoms in the straight-chain or branched alkyl part, alkylcarbonyl with 1 to 4 carbon atoms in the straight-chain or branched alkyl part, alkoxy and alkylthio with in each case 1 or 2 carbon atoms, and phenyl, 2-furyl, 2-pyridyl, 1-morpholino, cyclopropyl and cyclohexyl which are optionally substituted by one to three identical or different substituents from the group comprising chlorine and methyl; or $R^4$ furthermore represents allyl or propargyl, or represents 1-cyclohexenyl, or represents cyclohexyl which is substituted by one to three identical or different substituents, substituents which may be mentioned being: chlorine, methyl, ethyl, methoxy, cyano, amino, alkyl- or dialkylamino with in each case 1 or 2 carbon atoms in each alkyl part, carbamoyl, alkyl- and dialkylcarbamoyl with in each case 1 or 2 carbon atoms in each alkyl part, cyclohexyl, cyclohexylalkyl with 1 or 2 carbon atoms in the alkyl part and 1-pyrrolidin-2-one;

or $R^4$ also represents phenyl which is optionally substituted by one to three identical or different substituents from the group comprising chlorine and methyl, or represents 2-pyridyl or 2-pyrimidinyl, in each case optionally substituted by one to three methyl substituents, or represents 2-thiazolyl which is optionally substituted by phenyl, or represents 2-benzothiazolyl which is optionally substituted by one to three identical or different substituents from the group comprising chlorine, methyl and methoxy, or represents 1,2,4-triazol-3-yl, or represents 1,2,4-thiadiazol-5-yl which is optionally substituted by phenyl, or represents 1,3,4-thiadiazol-5-yl which is optionally substituted by one to three identical or different substituents from the group comprising mercapto, straight-chain or branched alkyl with 1 to 4 carbon atoms and in each case straight-chain or branched alkylthio, alkylsulphinyl and alkylsulphonyl with in each case 1 to 4 carbon atoms, or represents the grouping

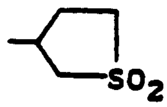 ,

or $R^4$ furthermore represents hydroxyl, alkoxy with 1 or 2 carbon atoms, benzyloxy which is optionally substituted by one to three identical or different substituents from the group comprising chlorine and methyl, or represents 1-adamantyl, 2-norbornyl, 1-or 2-decalyl or 1- or 2-tetralyl;

R represents substituted alkyl with 1 or 2 carbon atoms, substituents which may be mentioned being: halogen, alkylcarbonyl with 1 to 4 carbon atoms in the alkyl part, the groupings - $COOR^I$, $-CONR^{II}R^{III}$ and $OR^{IV}$ and the following heterocyclic radicals:

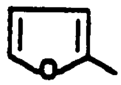 , 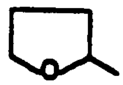 , 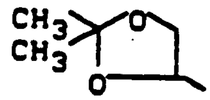 , 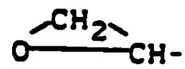

and 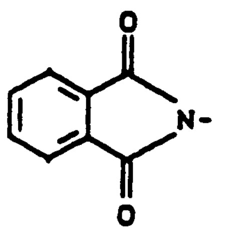 ;

or R furthermore represents 1-cyclohexenyl, optionally substituted by one to three methyl substituents,

$R^I$ represents methyl, ethyl, n- or i-propyl or n-, i-, s- or t-butyl,

$R^{II}$ and $R^{III}$ are identical or different and represent hydrogen, methyl, ethyl, n- or i-propyl, or represent benzyl which is optionally substituted by one to three identical or different substituents, with chlorine and methyl as substituents, or represent alkoxycarbonylalkyl with 1 or 2 carbon atoms in each alkyl part, or represent carbamoylalkyl, alkylcarbamoylalkyl or dialkylcarbamoylalkyl with in each case 1 or 2 carbon atoms in each alkyl part, or represent cyclohexyl which is optionally substituted by one to three methyl substituents and

$R^{IV}$ represents hydrogen, alkyl with 1 or 2 carbon atoms or benzyl which is optionally substituted by one to three identical or different substituents, substituents on the phenyl which may be mentioned being fluorine, chlorine and methyl;

and their geometric and optical isomers and isomer mixtures.

**6.** (2-Cyano-2-oximinoacetyl)-amino acid derivatives according to Claim 1, wherein, in the formula (I),

$R^1$ represents the grouping $-B-CO-NR^3R^3$,

B represents the groupings $-CH_2-$, $-CH(CH_3)-$ or $-CH_2CH_2-$,

$R^3$ and $R^4$, together with the nitrogen atom to which they are bonded, represent the following mono-, bi- or tricyclic heterocyclic or spiroheterocyclic radicals:

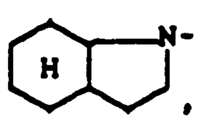 , 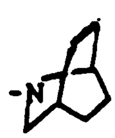 , 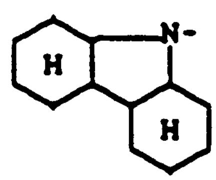 ,

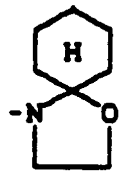 , 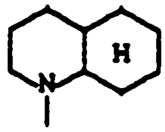 ,

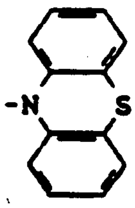 , 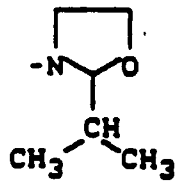 , 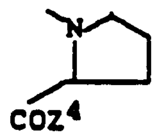

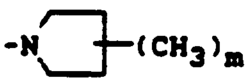 , 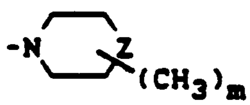

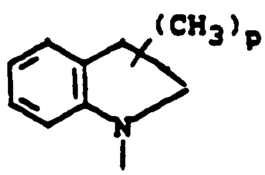 , 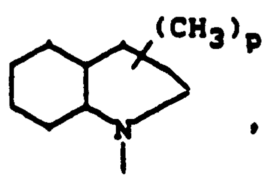 ,

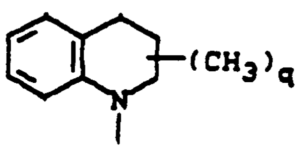 , 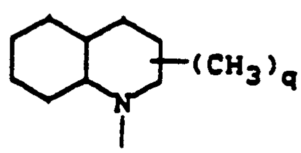 ,

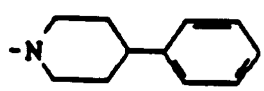 , 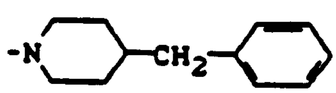 ,

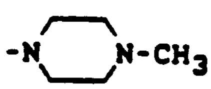 , 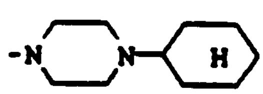 , 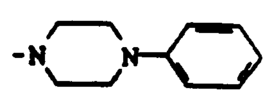 ,

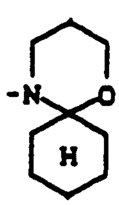 , 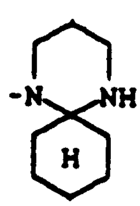 , 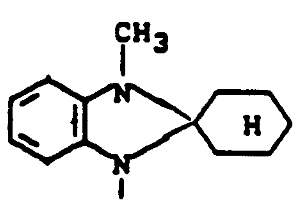 ,

wherein

Z represents oxygen or the $CH_2$ group,

$Z^4$ represents hydroxyl, methoxy, ethoxy, amino, methylamino, ethylamino, dimethylamino or ethylmethylamino,

m represents the number 0, 1, 2, 3 or 4,

| | |
|---|---|
| p | represents the number 0, 1 or 2 and |
| q | represents the number 0, 1, 2 or 3, and |
| R | represents substituted alkyl with 1 or 2 carbon atoms, substituents which may be mentioned being: halogen, alkylcarbonyl with 1 to 4 carbon atoms in the straight-chain or branched alkyl part, -COOR$^{I}$, -CONR$^{II}$R$^{III}$, OR$^{IV}$ and the following heterocyclic radicals: |

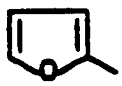, 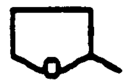, 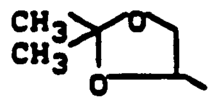, 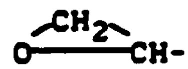

and 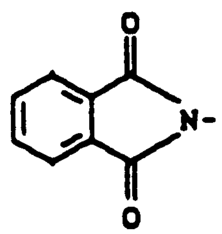 ;

or R furthermore represents 1-cyclohexenyl, optionally substituted by one to three methyl substituents,

| | |
|---|---|
| R$^{I}$ | represents methyl, ethyl, n- or i-propyl or n-, i-, s- or t-butyl, |
| R$^{II}$ and R$^{III}$ | are identical or different and represent hydrogen, methyl, ethyl, n- or i-propyl, or represent benzyl which is optionally substituted by one to three identical or different substituents, with chlorine and methyl as substituents, or represent alkoxycarbonylalkyl with 1 or 2 carbon atoms in each alkyl part, or represent carbamoylalkyl, alkylcarbamoylalkyl or dialkylcarbamoylalkyl with in each case 1 or 2 carbon atoms in each alkyl part, or represent cyclohexyl which is optionally substituted by one to three methyl substituents and |
| R$^{IV}$ | represents hydrogen, alkyl with 1 or 2 carbon atoms or benzyl which is optionally substituted by one to three identical or different substituents, substituents on the phenyl which may be mentioned being fluorine, chlorine and methyl; |

and their geometric and optical isomers and isomer mixtures.

**7.** (2-Cyano-2-oximinoacetyl)-amino acid derivatives according to Claim 1, wherein, in formula (I),

| | |
|---|---|
| $R^1$ | represents the grouping -A-COOR$^2$, |
| A | represents the groupings $-CH_2-$, $-CH(CH_3)-$ or $-CR_2CH_2-$, |
| $R^2$ | represents methyl or ethyl and |
| R | represents alkyl which has 1 or 2 carbon atoms and is substituted by one or two substituents, substituents which may be mentioned being: halogen, alkylcarbonyl with 1 to 4 carbon atoms in the straight-chain or branched alkyl part, -COOR$^{I}$, -CONR$^{II}$R$^{III}$, -OR$^{IV}$ and the following heterocyclic radicals: |

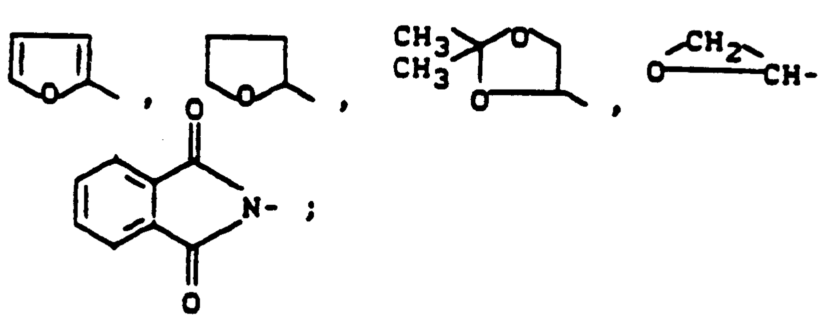

, , , and ;

or

| | |
|---|---|
| R | furthermore represents 1-cyclohexenyl, optionally substituted by one to three methyl |

substitutents,

$R^{I}$ represents methyl, ethyl, n- or i-propyl or n-, i-, s- or t-butyl,

$R^{II}$ and $R^{III}$ are identical or different and represent hydrogen, methyl, ethyl, n- or i-propyl, or represent benzyl which is optionally substituted by one to three identical or different substituents, with chlorine and methyl as substituents, or represent alkoxycarbonylalkyl with 1 or 2 carbon atoms in each alkyl part, or represent carbamoylalkyl or dialkylcarbamoylalkyl with in each case 1 or 2 carbon atoms in each alkyl part, or represent cyclohexyl which is optionally substituted by one to three methyl substituents and

$R^{IV}$ represents hydrogen, alkyl with 1 or 2 carbon atoms or benzyl which is optionally substituted by one to three identical or different substituents, substituents on the phenyl which may be mentioned being fluorine, chlorine and methyl,

and their geometric and optical isomers and isomer mixtures.

**8.** Process for the preparation of (2-cyano-2-oximinoacetyl)-amino acid derivatives of the general formula (I)

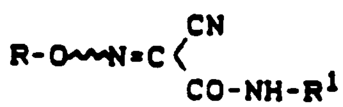

(I)

in which

$R^1$ represents the groupings $-A-COOR^2$ or $-B-CONR^3R^4$,

A represents an optionally substituted straight or branched alkylene chain,

B represents an optionally substituted straight or branched alkylene chain,

$R^2$ represents optionally substituted alkyl, with the following substituents: halogen, hydroxyl, alkoxy, acyloxy, optionally substituted aryl, optionally substituted cycloalkyl and optionally substituted heterocyclyl; or $R^2$ furthermore represents optionally substituted cycloalkyl or optionally substituted cycloalkenyl,

$R^3$ represents hydrogen, or represents optionally substituted alkyl, with the following substituents: halogen, cyano, $-COOR^{I}$, $-CONR^{II}R^{III}$, $NR^{II}R^{III}$, $-OR^{IV}$, $-S(O)_nR^{IV}$,acyl and in each case optionally substituted aryl, heterocyclyl, cycloalkyl and cycloalkenyl; or $R^3$ furthermore represents in each case optionally substituted alkenyl, alkinyl, cycloalkyl, cycloalkenyl, aryl or heterocyclyl,

$R^4$ represents hydrogen, or represents optionally substituted alkyl, with the following substituents: halogen, cyano, $-COOR^{I}$, $-CONR^{II}R^{III}$, $NR^{II}R^{III}$, $-OR^{IV}$, $-S(O)_nR^{IV}$,acyl and in each case optionally substituted aryl, heterocyclyl, cycloalkyl and cycloalkenyl; or $R^4$ furthermore represents in each case optionally substituted alkenyl, alkinyl, cycloalkyl, cycloalkenyl, aryl, heterocyclyl or polycyclic carbocyclic radicals or the grouping $-OR^{IV}$,

or

A and $R^2$, together with the atoms to which they are bonded, represent an optionally substituted ring,

or

B and $R^3$, together with the atoms to which they are bonded, represent an optionally substituted ring,

or

$R^3$ and $R^4$, together with the nitrogen atom to which they are bonded, represent an optionally substituted heterocyclic radical, which can contain further hetero atoms,

R represents substituted alkyl, with the following substituents: halogen, $-COOR^{I}$, $-CONR^{II}R^{III}$, $-OR^{IV}$, acyl and optionally substituted heterocyclyl, excluding azolyl; or R furthermore represents optionally substituted cycloalkenyl;

$R^{I}$ represents hydrogen or optionally substituted alkyl, with the following substituents: halogen and in each case optionally substituted aryl, cycloalkyl and cycloalkenyl,

$R^{II}$ and $R^{III}$ are identical or different and represent hydrogen or optionally substituted alkyl, with the following substituents: halogen, $-COOR^{IV}$, $-CONR^{I}R^{IV}$ and in each case optionally substituted aryl, cycloalkyl and cycloalkenyl; or $R^{II}$ and $R^{III}$ furthermore represent alkenyl, alkinyl or in each case optionally substituted cycloalkyl, cycloalkenyl, aryl or heterocyclyl, or, together with the nitrogen atom to which they are bonded, represent an optionally substituted heterocyclic radical, which can contain further hetero atoms,

$R^{IV}$ represents hydrogen, alkyl, aralkyl or acyl and

n represents the number 0, 1 or 2,

and their geometric and optical isomers and isomer mixtures, characterized in that

a) 2-cyano-2-oximino-acetic acid esters of the general formula (II)

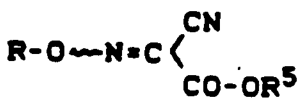

(II)

in which

R has the abovementioned meaning and

$R^5$ represents alkyl,

are reacted with amines of the formula (III)

$R^1-NH_2$ (III)

in which

$R^1$ has the abovementioned meaning, if appropriate in the presence of a diluent and if appropriate in the presence of a base;

or

b) carboxy-activated derivatives of carboxylic acids of the general formula (IV)

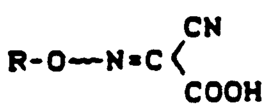

(IV)

in which

R has the abovementioned meaning, are reacted with amines of the formula (III)

$R^1-NH_2$ (III)

in which

$R^1$ has the abovementioned meaning, if appropriate in the presence of a catalyst and if appropriate in the presence of an acid-binding agent, and if appropriate in the presence of a diluent;

or

c) 2-cyano-2-oximino-acetamides of the general formula (V)

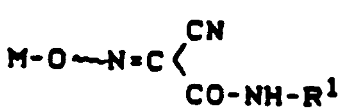

(V)

in which

M represents hydrogen, an alkali metal ion, a protonated tertiary base or a quaternary ammonium ion and

$R^1$ has the abovementioned meaning, are reacted with compounds of the formula (VI)

R-X (VI)

in which

X represents halogen or a sulphonyloxy radical and

R has the abovementioned meaning,

if appropriate in the presence of a base and if appropriate in the presence of a diluent;

or

d) substituted acetyl-amino acid esters of the general formula (VII)

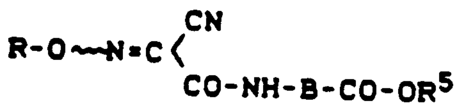

(VII)

in which

B, R and $R^5$ have the abovementioned meaning, are reacted with amines of the formula (VIII)

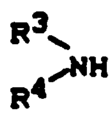

(VIII)

in which

$R^3$ and $R^4$ have the abovementioned meaning,

if appropriate in the presence of a diluent and if appropriate in the presence of a base;

or

e) carboxy-activated acetyl-amino acids of the general formula (IX)

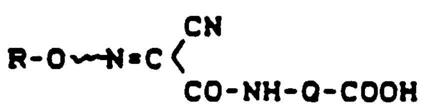

(IX)

in which

R has the abovementioned meaning and

Q represents A or B, these having the above-mentioned meaning,

are reacted with compounds of the formula (X)

Y-H (X)

in which

Y represents the groupings $R^2O$- or $R^3R^4N$-, wherein $R^2$, $R^3$ and $R^4$ have the abovementioned meaning,

if appropriate in the presence of a catalyst and if appropriate in the presence of an acid-binding agent, and if appropriate in the presence of a diluent;

or

f) ammonium salts of 2-cyano-2-oximino-acetic acids of the general formula (XI)

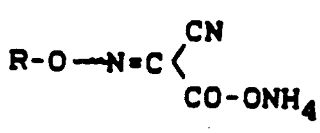

(XI)

in which

R has the abovementioned meaning,
are reacted with aldehydes of the formula (XII)

$R^6$-CH=O (XII)

in which
the grouping $R^6$-CH= has the abovementioned meanings of A or B,
and with isonitriles of the formula (XIII)

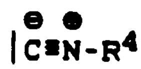

(XIII)

in which
$R^4$ has the abovementioned meaning,
if appropriate in the presence of a diluent;
or
g) 2-cyano-2-oximino-acetic acids of the general formula (IV)

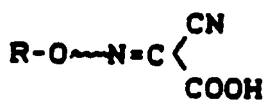

(IV)

in which
R has the abovementioned meaning,
are reacted with isocyanates of the formula (XIV)

$R^1$-NCO (XIV)

in which
$R^1$ has the abovementioned meaning,
if appropriate in the presence of a catalyst and if appropriate in the presence of a diluent;
or
h) 2-cyano-2-oximino-acetamides of the general formula (XV)

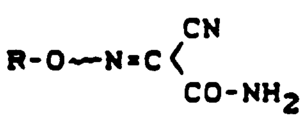

(XV)

in which
R has the abovementioned meaning,
are reacted with a base, if appropriate in the presence of a diluent, and the salts formed are reacted directly, or if appropriate after intermediate isolation, with compounds of the formula (XVI)

$R^1$-X (XVI)

in which
$R^1$ and X have the abovementioned meaning,
if appropriate in the presence of a diluent.

**9.** Agents for combating pests, characterized in that they contain at least one (2-cyano-2-oximinoacetyl)-amino acid derivative of the formula (I) according to Claims 1 and 8.

**10.** Use of (2-cyano-2-oximinoacetyl)-amino acid derivatives of the formula (I) according to Claims 1 and 8 for combating pests.

**11.** Method of combating pests, characterized in that (2-cyano-2-oximinoacetyl)-amino acid derivatives of the formula (I) according to Claims 1 and 8 are allowed to act on pests and/or their environment.

**12.** Process for the preparation of agents for combating pests, characterized in that (2-cyano-2-oximinoacetyl)-amino acid derivatives of the formula (I) according to Claims 1 and 8 are mixed with extenders and/or surface-active agents.

**Claims for the following Contracting State : ES**

**1.** Process for the preparation of (2-cyano-2-oximinoacetyl)-amino acid derivatives of the general formula (I)

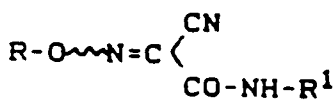

(I)

in which

| | |
|---|---|
| $R^1$ | represents the groupings $-A-COOR^2$ or $-B-CONR^3R^4$, |
| A | represents an optionally substituted straight or branched alkylene chain, |
| B | represents an optionally substituted straight or branched alkylene chain, |
| $R^2$ | represents optionally substituted alkyl, with the following substituents: halogen, hydroxyl, alkoxy, acyloxy, optionally substituted aryl, optionally substituted cycloalkyl and optionally substituted heterocyclyl; or $R^2$ furthermore represents optionally substituted cycloalkyl or optionally substituted cycloalkenyl, |
| $R^3$ | represents hydrogen, or represents optionally substituted alkyl, with the following substituents: halogen, cyano, $-COOR^{I}$, $-CONR^{II}R^{III}$, $-NR^{II}R^{III}$, $-OR^{IV}$, $-S(O)_nR^{IV}$,acyl and in each case optionally substituted aryl, heterocyclyl, cycloalkyl and cycloalkenyl; or $R^3$ furthermore represents in each case optionally substituted alkenyl, alkinyl, cycloalkyl, cycloalkenyl, aryl or heterocyclyl, |
| $R^4$ | represents hydrogen, or represents optionally substituted alkyl, with the following substituents: halogen, cyano, $-COOR^{I}$, $-CONR^{II}R^{III}$, $-NR^{II}R^{III}$, $-OR^{IV}$, $-S(O)_nR^{IV}$, acyl and in each case optionally substituted aryl, heterocyclyl, cycloalkyl and cycloalkenyl; or $R^4$ furthermore represents in each case optionally substituted alkenyl, alkinyl, cycloalkyl, cycloalkenyl, aryl, heterocyclyl or polycyclic carbocyclic radicals or the grouping $-OR^{IV}$, |
| | or |
| A and $R^2$, | together with the atoms to which they are bonded, represent an optionally substituted ring, |
| | or |
| B and $R^3$, | together with the atoms to which they are bonded, represent an optionally substituted ring, |
| | or |
| $R^3$ and $R^4$, | together with the nitrogen atom to which they are bonded, represent an optionally substituted heterocyclic radical, which can contain further hetero atoms, |
| R | represents substituted alkyl, with the following substituents: halogen, $-COOR^{I}$, $-CONR^{II}R^{III}$, $-OR^{IV}$, acyl and optionally substituted heterocyclyl, excluding azolyl; or R furthermore represents optionally substituted cycloalkenyl; |
| $R^{I}$ | represents hydrogen or optionally substituted alkyl, with the following substituents: halogen and in each case optionally substituted aryl, cycloalkyl and cycloalkenyl, |
| $R^{II}$ and $R^{III}$ | are identical or different and represent hydrogen or optionally substituted alkyl, with the following substituents: halogen, $-COOR^{IV}$, $-CONR^{IV}$, and in each case optionally substituted aryl, cycloalkyl and cycloalkenyl; or $R^{II}$ and $R^{III}$ furthermore represent alkenyl, alkinyl or in each case optionally substituted cycloalkyl, cycloalkenyl, aryl or |

heterocyclyl, or, together with the nitrogen atom to which they are bonded, represent an optionally substituted heterocyclic radical, which can contain further hetero atoms,

$R^{IV}$ represents hydrogen, alkyl, aralkyl or acyl and

n represents the number 0, 1 or 2,

and their geometric and optical isomers and isomer mixtures, characterized in that

a) 2-cyano-2-oximino-acetic acid esters of the general formula (II)

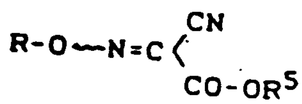 (II)

in which

R has the abovementioned meaning and

$R^5$ represents alkyl,

are reacted with amines of the formula (III)

$R^1$-$NH_2$ (III)

in which

$R^1$ has the abovementioned meaning, if appropriate in the presence of a diluent and if appropriate in the presence of a base;

or

b) carboxy-activated derivatives of carboxylic acids of the general formula (IV)

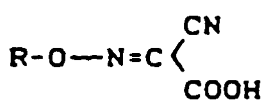 (IV)

in which

R has the abovementioned meaning,

are reacted with amines of the formula (III)

$R^1$-$NH_2$ (III)

in which

$R^1$ has the abovementioned meaning, if appropriate in the presence of a catalyst and if appropriate in the presence of an acid-binding agent, and if appropriate in the presence of a diluent;

or

c) 2-cyano-2-oximino-acetamides of the general formula (V)

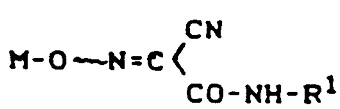 (V)

in which

M represents hydrogen, an alkali metal ion, a protonated tertiary base or a quaternary ammonium ion and

$R^1$ has the abovementioned meaning,

are reacted with compounds of the formula (VI)

R-X (VI)

in which

X represents halogen or a sulphonyloxy radical and

R has the abovementioned meaning,

if appropriate in the presence of a base and if appropriate in the presence of a diluent;

or

d) substituted acetyl-amino acid esters of the general formula (VII)

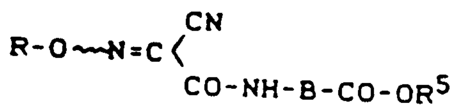

(VII)

in which

B, R and $R^5$ have the abovementioned meaning, are reacted with amines of the formula (VIII)

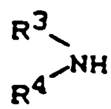

(VIII)

in which

$R^3$ and $R^4$ have the abovementioned meaning,

if appropriate in the presence of a diluent and if appropriate in the presence of a base;

or

e) carboxy-activated acetyl-amino acids of the general formula (IX)

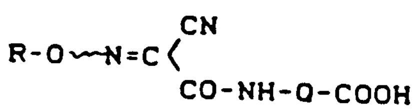

(IX)

in which

R has the abovementioned meaning and

Q represents A or B, these having the above-mentioned meaning,

are reacted with compounds of the formula (X)

Y-H (X)

in which

Y represents the groupings $R^2O$ or $R^3R^4N$-, wherein $R^2$, $R^3$ and $R^4$ have the abovementioned meaning, if appropriate in the presence of a catalyst and if appropriate in the presence of an acid-binding agent, and in appropriate in the presence of a diluent;

or

f) ammonium salts of 2-cyano-2-oximino-acetic acids of the general formula (XI)

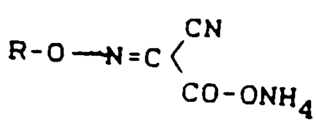

(XI)

in which

R has the abovementioned meaning,

are reacted with aldehydes of the formula (XIII)

$R^6$-CH = O (XII)

in which
the grouping $R^6$-CH = has the abovementioned meanings of A or B,
and with isonitriles of the formula (XIII)

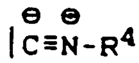

(XIII)

in which
$R^4$ has the abovementioned meaning,
if appropriate in the presence of a diluent;
or
g) 2-cyano-2-oximino-acetic acids of the general formula ((IV)

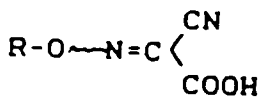

(IV)

in which
R has the abovementioned meaning,
are reacted with isocyanates of the formula (XIV)

$R^1$-NCO (XIV)

in which
$R^1$ has the abovementioned meaning,
if appropriate in the presence of a catalyst and if appropriate in the presence of a diluent;
or
h) 2-cyano-2-oximino-acetamides of the general formula (XV)

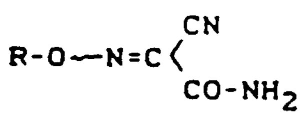

(XV)

in which
R has the abovementioned meaning, are reacted with a base, if appropriate in the presence of a diluent, and the salts formed are reacted directly, or if appropriate after intermediate isolation, with compounds of the formula (XVI)

$R^1$-X (XVI)

in which
$R^1$ and X have the abovementioned meaning,
if appropriate in the presence of a diluent.

**2.** Process according to Claim 1, wherein, in the formula (I),
$R^1$ represents the groupings -A-$COOR^2$ or -B-$CONR^3R^4$,
A represents a straight or branched alkylene chain with 1 to 4 carbon atoms,
B represents a straight or branched alkylene chain with 1 to 4 carbon atoms,
$R^2$ represents optionally substituted straight-chain or branched alkyl with 1 to 6 carbon atoms, substituents which may be mentioned being: halogen, and phenyl and

cycloalkyl with 3 to 6 carbon atoms which are optionally substituted by one to five identical or different substituents from the group comprising halogen, alkyl and alkoxy with in each case 1 to 4 carbon atoms; or $R^2$ furthermore preferably represents cycloalkyl which has 3 to 6 carbon atoms and is optionally substituted by one to five identical or different alkyl substituents with 1 to 4 carbon atoms, or represents cycloalkenyl which has 5 to 7 carbon atoms and is optionally substituted by one to five identical or different alkyl substituents with 1 to 4 carbon atoms;

$R^3$ represents hydrogen, or represents straight-chain or branched alkyl which has 1 to 4 carbon atoms and is optionally substituted by one or two substituents, substituents which may be mentioned being; halogen, cyano, $-COOR^{I}$, $-COOR^{II}R^{III}$, $NR^{II}R^{III}$, $-OR^{IV}$, $-S(O)_nR^{IV}$, acyl with 2 to 9 carbon atoms, phenyl which is optionally substituted by one to five identical or different substituents from the group comprising halogen and alkyl with 1 to 4 carbon atoms, a 5- or 6-membered heterocyclic radical which has 1 to 3 hetero atoms, and is optionally substituted by one to five identical or different substituents from the group comprising halogen and alkyl and alkoxy with in each case 1 to 4 carbon atoms, and cycloalkyl with 3 to 6 carbon atoms and cycloalkenyl with 5 to 7 carbon atoms, in each case optionally substituted by one to five identical or different alkyl substituents with 1 to 4 carbon atoms; or $R^3$ furthermore represents straight-chain or branched alkenyl or alkinyl with in each case 2 to 6 carbon atoms; or represents cyclo-alkenyl which has 5 to 7 carbon atoms and is optionally substituted by one to five identical or different alkyl substituents with 1 to 4 carbon atoms, or represents cycloalkyl which has 3 to 6 carbon atoms and is optionally substituted by one to five identical or different substituents, substituents which may be mentioned being: halogen, alkyl and alkoxy with in each case 1 to 4 carbon atoms, cyano, amino, carbamoyl, alkylamino, dialkylamino, alkylcarbamoyl and di-alkylcarbamoyl with in each case 1 to 4 carbon atoms in each alkyl part, alkoxycarbonylamino with 1 to 4 carbon atoms in the alkoxy part, cycloalkyl and cycloalkylalkyl with 5 or 6 carbon atoms in the cycloalkyl part and 1 to 4 carbon atoms in the straight-chain or branched alkyl part, and phenyl and pyrrolidone which are optionally substituted by one to five identical or different substituents from the group comprising halogen and alkyl with 1 to 4 carbon atoms; or $R^3$ furthermore represents phenyl which is optionally substituted by one to five identical or different substituents from the group comprising halogen, alkyl and alkoxy with in each case 1 to 4 carbon atoms and halogenoalkyl and halogenoalkoxy with in each case 1 or 2 carbon atoms and 1 to 5 identical or different halogen atoms, or represents a 5- or 6-membered heterocyclic radical which has 1 to 3 identical or different hetero atoms, and is optionally substituted by one to five identical or different substituents; substituents which may be mentioned being: halogen, mercapto, phenyl and straight-chain or branched alkyl, alkoxy, alkylthio, alkylsulphinyl and alkylsulphonyl with 1 to 4 carbon atoms per alkyl part;

$R^4$ has the meanings of $R^3$, or represents polycyclic carbocyclic radicals or the grouping $-OR^{IV}$,

or

A and $R^2$, together with the atoms to which they are bonded, represent a ring of the formula

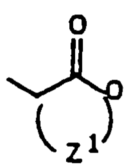

in which $Z^1$ represents a straight or branched alkylene chain with 1 to 4 carbon atoms,

or

B and $R^3$, together with the atoms to which they are bonded, represent a ring of the formula

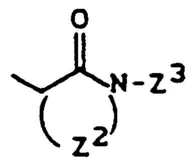

in which $Z^2$ represents a straight or branched alkylene chain with 1 to 4 carbon atoms and $Z^3$ represents hydrogen or alkyl with 1 to 4 carbon atoms,

or

$R^3$ and $R^4$, together with the nitrogen atom to which they are bonded, represent a mono-, bi- or tricyclic heterocyclic radical or spiroheterocyclic radical which has one to three other identical or different hetero atoms, and is optionally substituted by one to five identical or different substituents, substituents which may be mentioned being: straight-chain or branched alkyl or alkoxy with in each case 1 to 4 carbon atoms, cycloalkyl with 3 to 6 carbon atoms, the hydroxyl and the oxo group, straight-chain or branched alkenyl with 2 to 4 carbon atoms, hydroxycarbonyl, alkoxycarbonyl with 1 to 4 carbon atoms in the straight-chain or branched alkyl part, carbamoyl, alkyl- and dialkylcarbamoyl with in each case 1 to 4 carbon atoms in each alkyl part and phenyl and phenylalkyl with 1 to 4 carbon atoms in the straight-chain or branched alkyl part, in each case optionally substituted by one or two identical or different substituents from the group comprising halogen and straight-chain or branched alkyl and alkoxy with in each case 1 to 4 carbon atoms,

R represents straight-chain or branched alkyl which has 1 to 6 carbon atoms and is substituted by one or two substituents, substituents which may be mentioned being: halogen, $-COOR^I$, $-CONR^{II}R^{III}$, $-OR^{IV}$, acyl with 2 to 9 carbon atoms and 3- to 6-membered heterocyclyl which has 1 to 3 hetero atoms, excluding azolyl, and is optionally substituted by one to five identical or different substituents from the group comprising halogen and alkyl with 1 to 4 carbon atoms; or R furthermore preferably represents cycloalkenyl which has 5 to 7 carbon atoms and is optionally substituted by one to five identical or different substituents from the group comprising alkyl with 1 to 4 carbon atoms,

$R^I$ represents hydrogen or straight-chain or branched alkyl with 1 to 6 carbon atoms,

$R^{II}$ represents hydrogen, straight-chain or branched alkyl with 1 to 6 carbon atoms, phenylalkyl which has 1 to 4 carbon atoms in the straight-chain or branched alkyl part and is optionally substituted by one to five identical or different substituents, possible substituents on the phenyl which may be mentioned being: halogen, alkyl and alkoxy with in each case 1 to 4 carbon atoms and halogenoalkyl and halogenoalkoxy with in each case 1 or 2 carbon atoms and 2 to 5 identical or different halogen atoms; or $R^{II}$ furthermore represents alkoxycarbonylalkyl, carbamoylalkyl, alkylcarbamoylalkyl or dialkylcarbamoylalkyl with in each case 1 to 4 carbon atoms in each alkyl part, or represents cycloalkyl which has 3 to 6 carbon atoms and is optionally substituted by straight-chain or branched alkyl with 1 to 4 carbon atoms,

$R^{III}$ represents hydrogen, straight-chain or branched alkyl with 1 to 6 carbon atoms, or phenylalkyl which has 1 to 4 carbon atoms in the straight-chain or branched alkyl part and is optionally substituted by one to five identical or different substituents, possible substituents on the phenyl being the substituents on the phenyl mentioned for $R^{II}$; $R^{III}$ furthermore represents alkoxycarbonylalkyl, carbamoylalkyl, alkylcarbamoylalkyl or dialkylcarbamoylalkyl with in each case 1 to 4 carbon atoms in each alkyl part, or represents cycloalkyl which has 3 to 6 carbon atoms and is optionally substituted by straight-chain or branched alkyl with 1 to 4 carbon atoms;

$R^{IV}$ represents hydrogen, straight-chain or branched alkyl with 1 to 6 carbon atoms or phenylalkyl which has 1 to 4 carbon atoms in the straight-chain or branched alkyl part and is optionally substituted by one to five identical or different substituents, possible substituents on the phenyl being the substituents on the phenyl mentioned for $R^{II}$; or $R^{IV}$ furthermore represents acyl with 2 to 9 carbon atoms and

n represents the number 0, 1 or 2,

and their geometric and optical isomers and isomer mixtures.

**3.** Process according to Claim 1, wherein, in the general formula (I)

$R^1$ represents the groupings -A-COOR$^2$ or -B-CONR$^3$R$^4$,

A represents the groupings $-CH_2-$, $-CH(CH_3)-$ or $-CH_2CH_2-$,

B represents the groupings $-CH_2-$, $-CH(CH_3)-$ or $-CH_2CH_2-$,

$R^2$ represents straight-chain or branched alkyl with 1 to 4 carbon atoms,

$R^3$ represents hydrogen, or represents straight-chain or branched alkyl which has 1 to 4 carbon atoms and is optionally substituted by one or two identical or different substituents, substituents which may be mentioned being: halogen, cyano, -COOR$^I$, -CONR$^{II}$R$^{III}$, NR$^{II}$R$^{III}$,-OR$^{IV}$, -S(O)$_n$R$^{IV}$, acyl with 2 to 9 carbon atoms, phenyl which is optionally substituted by one to three identical or different substituents from the group comprising halogen and alkyl with 1 to 4 carbon atoms, a 5- or 6-membered heterocyclic radical which has 1 to 3 identical or different hetero atoms, and is optionally substituted by one to three identical or different substituents from the group comprising halogen and straight-chain or branched alkyl and alkoxy with in each case 1 to 4 carbon atoms, and cycloalkyl with 3 to 6 carbon atoms and cycloalkenyl with 5 to 7 carbon atoms, in each case optionally substituted by straight-chain or branched alkyi with 1 to 4 carbon atoms; or $R^3$ furthermore represents straight-chain or branched alkenyl or alkinyl with in each case 2 to 6 carbon atoms, or represents cycloalkenyl which has 5 to 7 carbon atoms and is optionally substituted by straight-chain or branched alkyl with 1 to 4 carbon atoms, or represents optionally substituted cycloalkyl with 3 to 6 carbon atoms, substituents which may be mentioned being: halogen, straight-chain or branched alkyl and alkoxy with in each case 1 to 4 carbon atoms, cyano, amino, carbamoyl, in each case straight-chain or branched alkylamino, dialkylamino, alkylcarbamoyl and dialkylcarbamoyl with in each case 1 to 4 carbon atoms in each alkyl part, alkoxycarbonylamino with 1 to 4 carbon atoms in the straight-chain or branched alkoxy part, cycloalkyl and cycloalkylalkyl with in each case 5 or 6 carbon atoms in the cycloalkyl part and 1 to 4 carbon atoms in the straight-chain or branched alkyl part, and phenyl and pyrrolidone, optionally substituted by one to three identical or different substituents from the group comprising halogen and alkyl with 1 to 4 carbon atoms; or $R^3$ furthermore represents phenyl which is optionally substituted by one to three identical or different substituents from the group comprising halogen, straight-chain or branched alkyl and alkoxy with in each case 1 to 4 carbon atoms and halogenoalkyl and halogenoalkoxy with in each case 1 or 2 carbon atoms and 1 to 5 identical or different halogen atoms, or represents a 5- or 6-membered heterocyclic radical which has 1 to 3 hetero atoms, and is optionally substituted by one to three identical or different substituents; substituents which may be mentioned for the heterocyclic radicals are: halogen, mercapto, phenyl and straight-chain or branched alkyl, alkoxy, alkylthio, alkylsulphinyl and alkylsulphonyl with 1 to 4 carbon atoms per alkyl;

$R^4$ has the meanings of $R^3$, or represents polycyclic carbocyclic radicals or the grouping -OR$^{IV}$,

or

A and $R^2$, together with the atoms to which they are bonded, represent 5- or 6-membered rings of the formulae

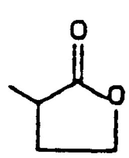 and 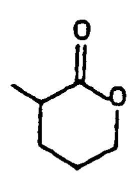

which are optionally substituted by alkyl with 1 to 4 carbon atoms,

or

B and $R^3$, together with the atoms to which they are bonded, represent 5- to 7-membered rings of the formulae

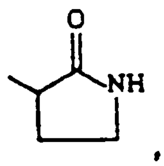, 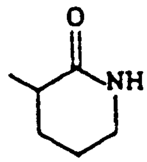 and 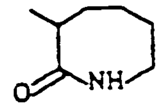

which are optionally substituted by alkyl with 1 to 4 carbon atoms,
or

$R^3$ and $R^4$, together with the nitrogen atom to which they are bonded, represent a mono-, bi- or tricyclic heterocyclic or spiroheterocyclic radical which is optionally substituted by one to five identical or different substituents, heterocyclic radicals which may be mentioned being, oxazolidine, pyrrolidine, imidazolidine, piperidine, piperazine, morpholine, thiomorpholine, 1,3-oxazane and 1,3-diazane; these heterocyclic radicals can in each case be optionally fused to 1 or 2 benzene or cyclohexane rings or optionally bridged with methylene or ethylene.

Substituents which may be mentioned for all the hetero systems are:
straight-chain or branched alkyl or alkoxy with in each case 1 to 4 carbon atoms, cycloalkyl with 3 to 6 carbon atoms, the hydroxyl and the oxo group, straight-chain or branched alkenyl with 2 to 4 carbon atoms, hydroxycarbonyl, alkoxycarbonyl with 1 to 4 carbon atoms in the straight-chain or branched alkyl part, carbamoyl, alkyl- and dialkylcarbamoyl with in each case 1 to 4 carbon atoms in each alkyl part and phenyl and phenylalkyl with 1 to 4 carbon atoms in the straight-chain or branched alkyl part, in each case optionally substituted by one or two identical or different substituents from the group comprising halogen and straight-chain or branched alkyl and alkoxy with in each case 1 to 4 carbon atoms,

R represents substituted straight-chain or branched alkyl with 1 to 4 carbon atoms, preferred substituents which may be mentioned being: halogen, $-COOR^I$, $-CONR^{II}R^{III}$, $-OR^{IV}$, acyl with 2 to 9 carbon atoms and heterocyclic radicals of the formulae

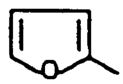, 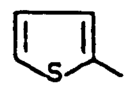, 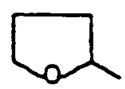, 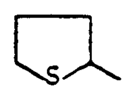,

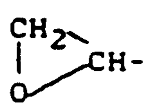, 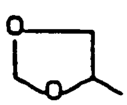 and 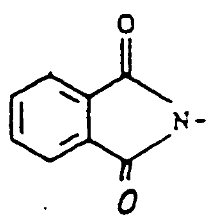,

which are optionally substituted by one to three identical or different alkyl substituents with 1 or 2 carbon atoms, or R furthermore represents cyclohexenyl or cyclopentenyl, optionally substituted by one to three methyl substituents,

$R^I$ represents straight-chain or branched alkyl with 1 to 4 carbon atoms,

$R^{II}$ represents hydrogen, straight-chain or branched alkyl with 1 to 4 carbon atoms or benzyl or phenethyl, in each case optionally substituted by one to three identical or different substituents, substituents on the phenyl which may be mentioned in each case being: fluorine, chlorine, methyl, methoxy and trifluoromethyl; or $R^{II}$ furthermore represents alkoxycarbonylalkyl with 1 to 4 carbon atoms in the alkoxy part and 1 or 2 carbon atoms in the alkyl part, carbamoylalkyl with 1 or 2 carbon atoms in the alkyl part or alkylcarbamoylalkyl or dialkylcarbamoylalkyl with in each case 1 or 2 carbon atoms in each alkyl part, or represents cycloalkyl which has 3 to 6 carbon atoms and is optionally substituted by one to three identical or different substituents from the group comprising methyl and ethyl,

$R^{III}$ has the meanings of $R^{II}$,

$R^{IV}$ represents hydrogen, straight-chain or branched alkyl with 1 to 4 carbon atoms or benzyl or phenethyl, in each case optionally substituted by one to three identical or different substituents, substituents on the phenyl which may be mentioned in each case being: fluorine, chlorine, methyl, methoxy and trifluoromethyl; or $R^{IV}$ furthermore represents acyl with 2 to 9 carbon atoms, and

n represents the number 0, 1 or 2,

and their geometric and optical isomers and isomer mixtures.

**4.** Process according to Claim 1, wherein in the formula (I),

$R^1$ represents the grouping $-B-CO-NR^3R^4$,

B represents the groupings $-CH_2-$, $-CH(CH_3)-$ or $-CH_2CH_2-$,

$R^3$ represents hydrogen or straight-chain or branched alkyl with 1 to 4 carbon atoms,

$R^4$ represents hydrogen or straight-chain or branched alkyl with 1 to 4 carbon atoms,

R represents substituted alkyl with 1 or 2 carbon atoms, substituents which may be mentioned being: halogen, alkylcarbonyl with 1 to 4 carbon atoms in the straight-chain or branched alkyl part, the groupings $-COOR^I$, $-CONR^{II}R^{III}$ and $-OR^{IV}$ and the following heterocyclic radicals:

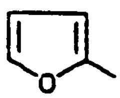 , 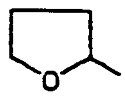 , 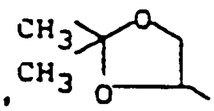 , 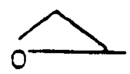 and

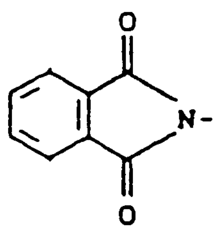 ;

or R furthermore represents 1-cyclohexenyl which is optionally substituted by one to thee methyl substituents;

$R^1$ represents methyl, ethyl, n- or i-propyl or n-, i-, s- or t-butyl,

$R^{II}$ and $R^{III}$ are identical or different and represent hydrogen, methyl, ethyl, n- or i-propyl, or represent benzyl which is optionally substituted by one to three identical or different substituents, with chlorine and methyl as substituents, or represent alkoxycarbonylalkyl with 1 or 2 carbon atoms in each alkyl part, or represent carbamoylalkyl, alkylcarbamoylalkyl or dialkylcarbamoylalkyl with in each case 1 or 2 carbon atoms in each alkyl part, or represent cyclohexyl which is optionally substituted by one to three methyl substituents and

$R^{IV}$ represents hydrogen, alkyl with 1 or 2 carbon atoms or benzyl which is optionally substituted by one to three identical or different substituents, substituents on the phenyl which may be mentioned being fluorine, chlorine and methyl,

and their geometric and optical isomers and isomer mixtures.

**5.** Process according to Claim 1, wherein, in the formula (I),

$R^1$ represents the grouping $-B-CO-NR^3R^4$,

B represents the groupings $-CH_2-$, $-CH(CH_3)-$ or $-CH_2CH_2-$,

$R^3$ represents hydrogen, straight-chain or branched all with 1 to 4 carbon atoms or alkyl which has 1 to 3 carbon atoms and is substituted by one or two substituents, substituents which may be mentioned being: chlorine, hydroxyl, alkylcarbonyloxy with 1 to 4 carbon atoms in the straight-chain or branched alkyl part, alkylcarbonyl with 1 to 4 carbon atoms in the straight-chain or branched alkyl part, alkoxy and alkylthio with in each case 1 or 2 carbon atoms and phenyl, 2-furyl, 2-pyridyl, 1-morpholino, cyclopropyl and cyclohexyl which are optionally substituted by one to

three identical or different substituents from the group comprising chlorine and methyl; or $R^3$ furthermore represents allyl or propargyl, or represents 1-cyclohexenyl, or represents cyclohexyl which is substituted by one to three identical or different substituents, substituents which may be mentioned being: chlorine, methyl, ethyl, methoxy, cyano, amino, alkyl- and dialkylamino with in each case 1 or 2 carbon atoms in each alkyl part, carbamoyl, alkyl- and dialkylcarbamoyl with in each case 1 or 2 carbon atoms in each alkyl part, cyclohexyl, cyclohexylalkyl with 1 or 2 carbon atoms in the alkyl part and 1-pyrrolidin-2-one; or

$R^3$ also represents phenyl which is optionally substituted by one to three identical or different substituents from the group comprising chlorine and methyl, or represents 2-pyridyl or 2-pyrimidinyl, in each case optionally substituted by one to three methyl substituents, or represents 2-thiazolyl which is optionally substituted by phenyl, or represents 2-benzo-thiazolyl which is optionally substituted by one to three identical or different substituents from the group comprising chlorine, methyl and methoxy, or represents 1,2,4-triazol-3-yl, or represents 1,2,4-thiadiazol-5-yl which is optionally substituted by phenyl, or represents 1,3,4-thiadiazol-5-yl which is optionally substituted by one to three identical or different substituents from the group comprising mercapto, straight-chain or branched alkyl with 1 to 4 carbon atoms and in each case straight-chain or branched alkylthio, alkylsulphinyl and alkylsulphonyl with in each case 1 to 4 carbon atoms, or represents the grouping

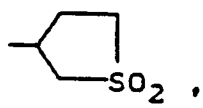

$R^4$ represents alkyl which has 1 to 3 carbon atoms and is substituted by one or two substituents, substituents which may be mentioned being: chlorine, hydroxyl, alkylcarbonyloxy with 1 to 4 carbon atoms in the straight-chain or branched alkyl part, alkylcarbonyl with 1 to 4 carbon atoms in the straight-chain or branched alkyl part, alkoxy and alkylthio with in each case 1 or 2 carbon atoms, and phenyl, 2-furyl, 2-pyridyl, 1-morpholino, cyclopropyl and cyclohexyl which are optionally substituted by one to three identical or different substituents from the group comprising chlorine and methyl; or $R^4$ furthermore represents allyl or propargyl, or represents 1-cyclohexenyl, or represents cyclohexyl which is substituted by one to three identical or different substituents, substituents which may be mentioned being: chlorine, methyl, ethyl, methoxy, cyano, amino, alkyl- or dialkylamino with in each case 1 or 2 carbon atoms in each alkyl part, carbamoyl, alkyl- and dialkylcarbamoyl with in each case 1 or 2 carbon atoms in each alkyl part, cyclohexyl, cyclohexylalkyl with 1 or 2 carbon atoms in the alkyl part and 1-pyrrolidin-2-one;

or $R^4$ also represents phenyl which is optionally substituted by one to three identical or different substituents from the group comprising chlorine and methyl, or represents 2-pyridyl or 2-pyrimidinyl, in each case optionally substituted by one to three methyl substituents, or represents 2-thiazolyl which is optionally substituted by phenyl, or represents 2-benzo-thiazolyl which is optionally substituted by one to three identical or different substituents from the group comprising chlorine, methyl and methoxy, or represents 1,2,4-triazol-3-yl, or represents 1,2,4-thiadiazol-5-yl which is optionally substituted by phenyl, or represents 1,3,4-thiadiazol-5-yl which is optionally substituted by one to three identical or different substituents from the group comprising mercapto, straight-chain or branched alkyl with 1 to 4 carbon atoms and in each case straight-chain or branched alkylthio, alkylsulphinyl and alkylsulphonyl with in each case 1 to 4 carbon atoms, or represents the grouping

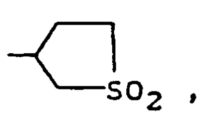

or $R^4$ furthermore represents hydroxyl, alkoxy with 1 or 2 carbon atoms, benzyloxy which is optionally substituted by one to three identical or different substituents from the group comprising chlorine and methyl, or represents 1-adamantyl, 2-norbornyl, 1- or 2-decalyl or 1- or 2-tetralyl;

R represents substituted alkyl with 1 or 2 carbon atoms, substituents which may be mentioned being: halogen, alkylcarbonyl with 1 to 4 carbon atoms in the alkyl part, the groupings $-COOR^I$, $-CONR^{II}R^{III}$ and $-OR^{IV}$ and the following heterocyclic radicals:

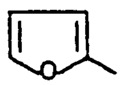 , 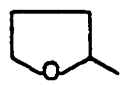 , 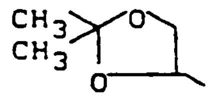 , 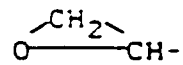

and 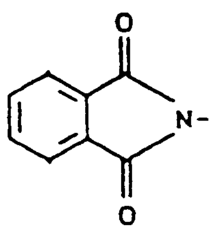 ;

or R furthermore represents 1-cyclohexenyl, optionally substituted by one to three methyl substituents,

$R^I$ represents methyl, ethyl, n- or i-propyl or n-, i-, s- or t-butyl,

$R^{II}$ and $R^{III}$ are identical or different and represent hydrogen, methyl, ethyl, n- or i-propyl, or represent benzyl which is optionally substituted by one to three identical or different substituents, with chlorine and methyl as substituents, or represent alkoxycarbonylalkyl with 1 or 2 carbon atoms in each alkyl part, carbamoylalkyl, alkylcarbamoylalkyl or dialkylcarbamoylalkyl with in each case 1 or 2 carbon atoms in each alkyl part, or represent cyclohexyl which is optionally substituted by one to three methyl substituents and

$R^{IV}$ represents hydrogen, alkyl with 1 or 2 carbon atoms or benzyl which is optionally substituted by one to three identical or different substituents, substituents on the phenyl which may be mentioned being fluorine, chlorine and methyl;

and their geometric and optical isomers and isomer mixtures.

**6.** Process according to Claim 1, wherein, in the formula (I),

$R^1$ represents the grouping $-B-CO-NR^3R^3$,

B represents the groupings $-CH_2-$, $-CH(CH_3)-$ or $-CH_2CH_2-$,

$R^3$ and $R^4$, together with the nitrogen atom to which they are bonded, represent the following mono-, bi- or tricyclic heterocyclic or spiroheterocyclic radicals:

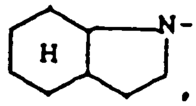 , 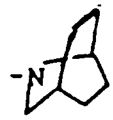 , 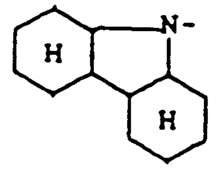 ,

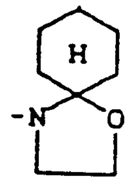 , 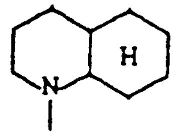 ,

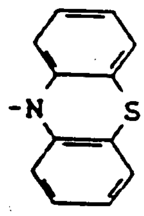 , 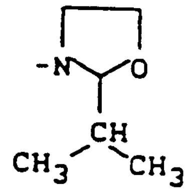 , 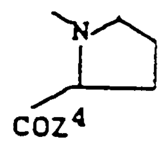

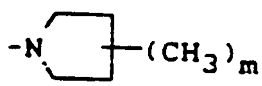 , 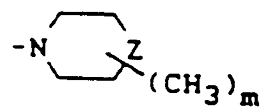

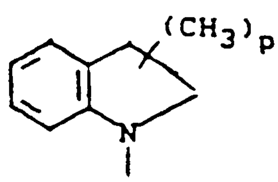 , 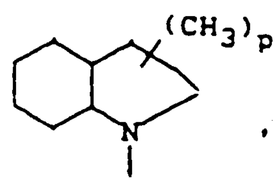 ,

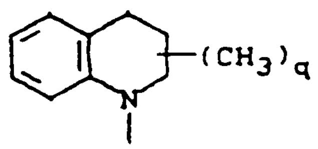 , 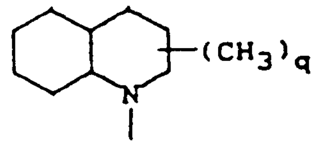 ,

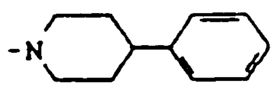 , 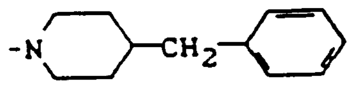 ,

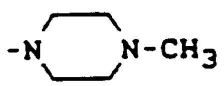 , 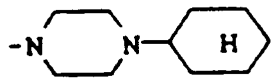 , 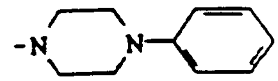 ,

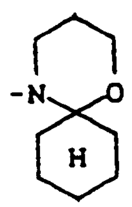 , 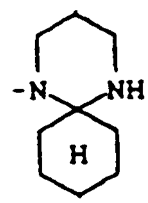 , 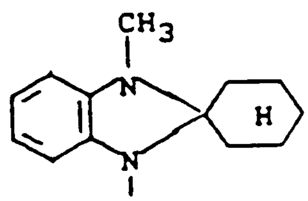 ,

wherein

Z represents oxygen or the $CH_2$ group,

$Z_4$ represents hydroxyl, methoxy, ethoxy, amino, methylamino, ethylamino, dimethylamino or ethylmethylamino,

m represents the number 0, 1, 2, 3 or 4,

p represents the number 0, 1 or 2 and

q represents the number 0, 1, 2 or 3, and

R represents substituted alkyl with 1 or 2 carbon atoms, substituents which may be mentioned being: halogen, alkylcarbonyl with 1 to 4 carbon atoms in the straight-chain or branched alkyl part, $-COOR^I$, $-CONR^{II}R^{III}$, $-OR^{IV}$ and the following heterocyclic radicals:

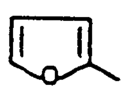 , 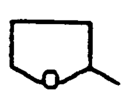 , 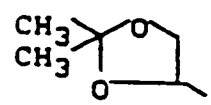 , 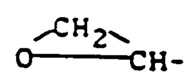

and 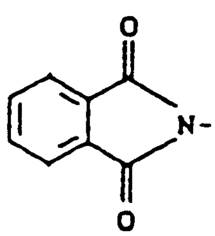 ;

or R furthermore represents 1-cyclohexenyl, optionally substituted by one to three methyl substituents,

$R^I$ represents methyl, ethyl, n- or i-propyl or n, i-, s- or t-butyl,

$R^{II}$ and $R^{III}$ are identical or different and represent hydrogen, methyl, ethyl, n- or i-propyl, or represent benzyl which is optionally substituted by one to three identical or different substituents, with chlorine and methyl as substituents, or represent alkoxycarbonylalkyl with 1 or 2 carbon atoms in each alkyl part, or represent carbamoylalkyl,

alkylcarbamoylalkyl or dialkylcarbamoylalkyl with in each case 1 or 2 carbon atoms in each alkyl part, or represent cyclohexyl which is optionally substituted by one to three methyl substituents and

$R^{IV}$ represents hydrogen, alkyl with 1 or 2 carbon atoms or benzyl which is optionally substituted by one to three identical or different substituents, substituents on the phenyl which may be mentioned being fluorine, chlorine and methyl;

and their geometric and optical isomers and isomer mixtures.

**7.** Process according to Claim 1, wherein, in formula (I),

$R^1$ represents the grouping $-A-COOR^2$,

A represents the groupings $-CH_2-$, $-CH(CH_3)-$ or $-CH_2CH_2-$,

$R^2$ represents methyl or ethyl and

R represents alkyl which has 1 or 2 carbon atoms and is substituted by one or two substituents, substituents which may be mentioned being: halogen, alkylcarbonyl with 1 to 4 carbon atoms in the straight-chain or branched alkyl part, $-COOR^I$, $-CONR^{II}R^{III}$, $-OR^{IV}$ and the following heterocyclic radicals:

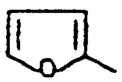 , 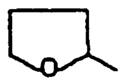 , 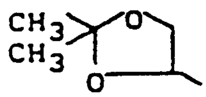 , 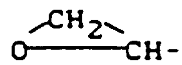

and 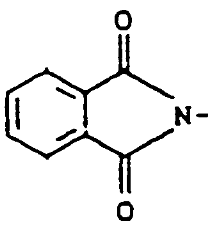 ;

or R furthermore represents 1-cyclohexenyl, optionally substituted by one to three methyl substitutents,

$R^I$ represents methyl, ethyl, n- or i-propyl or n-, i-, s- or t-butyl,

$R^{II}$ and $R^{III}$ are identical or different and represent hydrogen, methyl, ethyl, n- or i-propyl, or represent benzyl which is optionally substituted by one to three identical or different substituents, with chlorine and methyl as substituents, or represent alkoxycarbonylalkyl with 1 or 2 carbon atoms in each alkyl part, or represent carbamoylalkyl, alkylcarbamoylalkyl or dialkylcarbamoylalkyl with in each case 1 or 2 carbon atoms in each alkyl part, or represent cyclohexyl which is optionally substituted by one to three methyl substituents and

$R^{IV}$ represents hydrogen, alkyl with 1 or 2 carbon atoms or benzyl which is optionally substituted by one to three identical or different substituents, substituents on the phenyl which may be mentioned being fluorine, chlorine and methyl,

and their geometric and optical isomers and isomer mixtures.

**8.** Agents for combating pests, characterized in that they contain at least one (2-cyano-2-oximinoacetyl)-amino acid derivative of the formula (I) according to Claims 1 to 7.

**9.** Use of (2-cyano-2-oximinoacetyl)-amino acid derivatives of the formula (I) according to Claims 1 to 7 for combating pests.

**10.** Method of combating pests, characterized in that (2-cyano-2-oximinoacetyl)-amino acid derivatives of the formula (I) according to Claims 1 to 7 are allowed to act on pests and/or their environment.

**11.** Process for the preparation of agents for combating pests, characterized in that (2-cyano-2-oximinoacetyl)-amino acid derivatives of the formula (I) according to Claims 1 to 7 are mixed with

extenders and/or surface-active agents.

## Revendications

### Revendications pour les Etats contractants suivants : AT, BE, CH, DE, FR, GB, GR, IT, LI, NL

**1.** Dérivés de (2-cyano-2-oximinoacétyl)-aminoacides répondant à la formule générale (I)

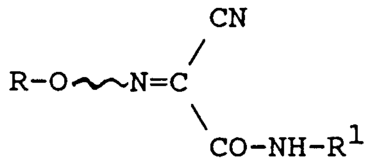

(I)

dans laquelle

$R^1$ représente les groupements $-A-COOR^2$ ou $-B-CONR^3R^4$

A représente une chaîne alkylène droite ou ramifiée éventuellement substituée,

B représente une chaîne alkylène droite ou ramifiée éventuellement substituée,

$R^2$ représente un groupe alkyle éventuellement substitué avec les substituants ci-après : un atome d'halogène, un groupe hydroxyle, un groupe alcoxy, un groupe acyloxy, un groupe aryle éventuellement substitué, un groupe cycloalkyle éventuellement substitué et un groupe hétérocyclyle éventuellement substitué; $R^2$ représente, en outre, un groupe cycloalkyle éventuellement substitué ou un groupe cycloalcényle éventuellement substitué,

$R^3$ représente un atome d'hydrogène ou un groupe alkyle éventuellement substitué avec les substituants ci-après : un atome d'halogène, un groupe cyano, $-COOR^I$, $-CONR^{II}R^{III}$, $-NR^{II}R^{III}$, $-OR^{IV}$, $-S(O)_nR^{IV}$, un groupe acyle; un groupe aryle, un groupe hétérocyclyle, un groupe cycloalkyle et un groupe cycloalcényle, chacun étant éventuellement substitué; $R^3$ représente, en outre, un groupe alcényle, un groupe alcynyle, un groupe cycloalkyle, un groupe cycloalcényle, un groupe aryle ou un groupe hétérocyclyle, chacun étant éventuellement substitué,

$R^4$ représente un atome d'hydrogène ou un groupe alkyle éventuellement substitué avec les substituants ci-après : un atome d'halogène, un groupe cyano, $-COOR^I$, $-CONR^{II}R^{III}$, $-NR^{II}R^{III}$,$-OR^{IV}$, $-S(O)_nR^{IV}$, un groupe acyle; un groupe aryle, un groupe hétérocyclyle, un groupe cycloalkyle et un groupe cycloalcényle, chacun étant éventuellement substitué; $R^4$ représente, en outre, un groupe alcényle, un groupe alcynyle, un groupe cycloalkyle, un groupe cycloalcényle, un groupe aryle, un groupe hétérocyclyle, chacun étant éventuellement substitué, ou bien des composés carbocycliques polycycliques, ou encore le groupement $-OR^{IV}$,

ou

A et $R^2$ représentent, conjointement avec les atomes auxquels ils sont liés, un noyau éventuellement substitué,

ou

B et $R^3$ représentent, conjointement avec les atomes auxquels ils sont liés, un noyau éventuellement substitué,

ou

$R^3$ et $R^4$ représentent, conjointement avec l'atome d'azote auquel ils sont liés, un composé hétérocyclique éventuellement substitué, qui peut contenir d'autres hétéroatomes,

R représente un groupe alkyle substitué avec les substituants ci-après : un atome d'halogène, $-COOR^I$,$-CONR^{II}R^{III}$, $-OR^{IV}$, un groupe acyle ou un groupe hétérocyclyle éventuellement substitué, à l'exception d'un groupe azolyle; R représente, en outre, un groupe cycloalcényle éventuellement substitué;

$R^I$ représente un atome d'hydrogène ou un groupe alkyle éventuellement substitué avec les substituants ci-après: un atome d'halogène, un groupe aryle, un groupe cycloalkyle et un groupe cycloalcényle, chacun de ces groupes étant éventuellement substitué,

$R^{II}$ et $R^{III}$ sont identiques ou différents, et représentent un atome d'hydrogène ou un groupe alkyle éventuellement substitué avec les substituants ci-après : un atome d'halogène,

-COOR$^{IV}$, CONR$^{I}$R$^{IV}$, un groupe aryle, un groupe cycloalkyle et un groupe cycloalcényle, chacun de ces groupes étant éventuellement substitué; R$^{II}$ et R$^{III}$ représentent, en outre, un groupe alcényle, un groupe alcynyle; ou encore un groupe cycloalkyle, un groupe cycloalcényle, un groupe aryle ou un groupe hétérocyclyle, chacun de ces groupes étant éventuellement substitué; ou encore représentent, conjointement avec l'atome d'azote auquel ils sont liés, un composé hétérocyclique éventuellement substitué, qui peut contenir d'autres hétéroatomes,

R$^{IV}$ représente un atome d'hydrogène, un groupe alkyle, un groupe aralkyle ou un groupe acyle et

n représente les nombres 0, 1 ou 2,

leurs isomères géométriques et optiques, ainsi que leurs mélanges d'isomères.

**2.** Dérivés de (2-cyano-2-oximinoacétyl)-aminoacides selon la revendication 1, dans lesquels, dans la formule (I) :

R$^{1}$ représente les groupements -A-COOR$^{2}$ ou -B-CONR$^{3}$R$^{4}$,

A représente une chaîne alkylène droite ou ramifiée contenant de 1 à 4 atomes de carbone,

B représente une chaîne alkylène droite ou ramifiée contenant de 1 à 4 atomes de carbone,

R$^{2}$ représente un groupe alkyle à chaîne droite ou ramifiée contenant de 1 à 6 atomes de carbone, éventuellement substitué, dans lequel, comme substituants, il convient de mentionner : un atome d'halogène, un groupe phényle éventuellement substitué de 1 à 5 fois de manière identique ou différente par un atome d'halogène, par un groupe alkyle et un groupe alcoxy contenant chacun de 1 à 4 atomes de carbone, ainsi qu'un groupe cycloalkyle contenant de 3 à 6 atomes de carbone; en outre, R$^{2}$ représente un groupe cycloalkyle contenant de 3 à 6 atomes de carbone, éventuellement substitué de 1 à 5 fois de manière identique ou différente par un groupe alkyle contenant de 1 à 4 atomes de carbone, ou encore un groupe cycloalcényle contenant de 5 à 7 atomes de carbone, éventuellement substitué de 1 à 5 fois de manière identique ou différente par un groupe alkyle contenant de 1 à 4 atomes de carbone;

R$^{3}$ représente un atome d'hydrogène ou un groupe alkyle à chaîne droite ou ramifiée contenant de 1 à 4 atomes de carbone, éventuellement substitué 1 ou 2 fois, dans lequel, comme substituants, il convient de mentionner:

un atome d'halogène, un groupe cyano, -COOR$^{I}$, -CONR$^{II}$R$^{III}$, -NR$^{II}$R$^{III}$, -OR$^{IV}$, -S(O)-$_{n}$R$^{IV}$, un groupe acyle contenant de 2 à 9 atomes de carbone, un groupe phényle éventuellement substitué de 1 à 5 fois de manière identique ou différente par un atome d'halogène et par un groupe alkyle contenant de 1 à 4 atomes de carbone, un composé hétérocyclique penta- ou hexagonal contenant de 1 à 3 hétéroatomes, éventuellement substitué de 1 à 5 fois de manière identique ou différente par un atome d'halogène, de même que par un groupe alkyle et un groupe alcoxy contenant chacun de 1 à 4 atomes de carbone; un groupe cycloalkyle contenant de 3 à 6 atomes de carbone ou un groupe cycloalcényle contenant de 5 à 7 atomes de carbone, chacun de ces groupes étant éventuellement substitué de 1 à 5 fois de manière identique ou différente par un groupe alkyle contenant de 1 à 4 atomes de carbone; R$^{3}$ représente, en outre,

un groupe alcényle ou un groupe alcynyle à chaîne droite ou ramifiée contenant chacun de 2 à 6 atomes de carbone; un groupe cycloalcényle contenant de 5 à 7 atomes de carbone, éventuellement substitué de 1 à 5 fois de manière identique ou différente par un groupe alkyle contenant de 1 à 4 atomes de carbone; ou représente un groupe cycloalkyle contenant de 3 à 6 atomes de carbone, éventuellement substitué de 1 à 5 fois de manière identique ou différente, dans lequel, comme substituants, il convient de mentionner :

un atome d'halogène, un groupe alkyle et un groupe alcoxy contenant chacun de 1 à 4 atomes de carbone, un groupe cyano, une groupe amino, un groupe carbamoyle, un groupe alkylamino, un groupe dialkylamino, un groupe alkylcarbamoyle et un groupe dialkylcarbamoyle contenant chacun de 1 à 4 atomes de carbone dans chaque fraction alkyle, un groupe alcoxycarbonylamino contenant de 1 à 4 atomes de carbone dans la fraction alcoxy, un groupe cycloalkyle et un groupe cycloalkylalkyle contenant 5 ou 6 atomes de carbone dans la fraction cycloalkyle et de 1 à 4 atomes de carbone dans la

fraction alkyle à chaîne droite ou ramifiée, un groupe phényle éventuellement substitué de 1 à 5 fois de manière identique ou différente par un atome d'halogène et par un groupe alkyle contenant de 1 à 4 atomes de carbone et un groupe pyrrolidone; $R^3$ représente, en outre,

un groupe phényle éventuellement substitué de 1 à 5 fois de manière identique ou différente par un atome d'halogène, par un groupe alkyle et un groupe alcoxy contenant chacun de 1 à 4 atomes de carbone, par un groupe halogénalkyle ou par un groupe halogénalcoxy contenant chacun 1 ou 2 atomes de carbone et de 1 à 5 atomes d'halogène identiques ou différents; ou représente un composé hétérocyclique penta- ou hexagonal contenant de 1 à 3 hétéroatomes identiques ou différents, éventuellement substitué de 1 à 5 fois de manière identique ou différente; comme substituants, on mentionnera : un atome d'halogène, un groupe mercapto, un groupe phényle, un groupe alkyle à chaîne droite ou ramifiée, un groupe alcoxy, un groupe alkylthio, un groupe alkylsulfinyle et un groupe alkylsulfonyle contenant de 1 à 4 atomes de carbone dans chaque fraction alkyle;

$R^4$ a la signification donnée pour $R^3$, représente des composés carbocycliques polycycliques ou encore le groupement $-OR^{IV}$,

ou

A et $R^2$ représentent ensemble avec les atomes auxquels ils sont liés, un noyau de formule

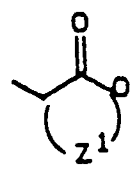

dans laquelle $Z^1$ représente une chaîne alkylène droite ou ramifiée contenant de 1 à 4 atomes de carbone,

ou

B et $R^3$ représentent, conjointement avec les atomes auxquels ils sont liés, un noyau de formule

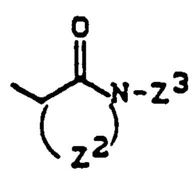

dans laquelle $Z^2$ représente une chaîne alkylène droite ou ramifiée contenant de 1 à 4 atomes de carbone et $Z^3$ représente un atome d'hydrogène ou un groupe alkyle contenant de 1 à 4 atomes de carbone,

ou

$R^3$ et $R^4$ représentent, conjointement avec l'atome d'azote auquel ils sont liés, un composé hétérocyclique mono-, bi- ou tricyclique ou un composé spirohétérocyclique contenant de 1 à 3 hétéroatomes supplémentaires identiques ou différents, éventuellement substitué de 1 à 5 fois de manière identique ou différente, et dans lequel, comme substituants, on mentionnera : un groupe alkyle ou un groupe alcoxy à chaîne droite ou ramifiée contenant chacun de 1 à 4 atomes de carbone, un groupe cycloalkyle contenant de 3 à 6 atomes de carbone, le groupe hydroxy ou le groupe oxo, un groupe alcényle à chaîne droite ou ramifiée contenant de 2 à 4 atomes de carbone, un groupe hydroxycarbonyle, un groupe alcoxycarbonyle contenant de 1 à 4 atomes de carbone dans la fraction alkyle à chaîne droite ou ramifiée; un groupe carbamoyle, un groupe alkyl- ou dialkylcarbamoyle contenant chacun de 1 à 4 atomes de carbone dans chaque fraction alkyle, ainsi qu'un groupe phényle ou bien un groupe phénylalkyle contenant de 1 à 4 atomes de carbone dans la fraction alkyle à chaîne droite ou ramifiée, chacun étant éventuellement substitué 1 ou 2 fois de manière identique ou différente par un

atome d'halogène ou par un groupe alkyle et un groupe alcoxy à chaîne droite ou ramifiée contenant chacun de 1 à 4 atomes de carbone,

R représente un groupe alkyle à chaîne droite ou ramifiée contenant de 1 à 6 atomes de carbone, substitué une ou deux fois, dans lequel, comme substituants, il convient de mentionner : un atome d'halogène, $-COOR^{I}$, $-CONR^{II}R^{III}$, $-OR^{IV}$, un groupe acyle contenant de 2 à 9 atomes de carbone, un groupe hétérocyclyle à trois termes ou hexagonal contenant de 1 à 3 hétéroatomes, éventuellement substitué de 1 à 5 fois de manière identique ou différente par un atome d'halogène et par un groupe alkyle contenant de 1 à 4 atomes de carbone, à l'exception du groupe azolyle; en outre, R représente de préférence un groupe cycloalcényle contenant de 5 à 7 atomes de carbone, éventuellement substitué de 1 à 5 fois de manière identique ou différente par un groupe alkyle contenant de 1 à 4 atomes de carbone,

$R^{I}$ représente un atome d'hydrogène ou un groupe alkyle à chaîne droite ou ramifiée contenant de 1 à 6 atomes de carbone,

$R^{II}$ représente un atome d'hydrogène, un groupe alkyle à chaîne droite ou ramifiée contenant de 1 à 6 atomes de carbone, un groupe phénylalkyle contenant de 1 à 4 atomes de carbone dans la fraction alkyle à chaîne droite ou ramifiée, éventuellement substitué de 1 à 5 fois de manière identique ou différente, dans lequel, comme substituants du groupe phényle, on mentionnera : un atome d'halogène, un groupe alkyle et un groupe alcoxy contenant chacun de 1 à 4 atomes de carbone, un groupe halogénalkyle et un groupe halogénalcoxy contenant chacun 1 ou 2 atomes de carbone et de 2 à 5 atomes d'halogène identiques ou différents; $R^{II}$ représente, en outre, un groupe alcoxycarbonylalkyle, un groupe carbamoylalkyle, un groupe alkylcarbamoylalkyle ou un groupe dialkylcarbamoylalkyle contenant chacun de 1 à 4 atomes de carbone dans chaque fraction alkyle ou encore un groupe cycloalkyle contenant de 3 à 6 atomes de carbone, éventuellement substitué par un groupe alkyle à chaîne droite ou ramifiée contenant de 1 à 4 atomes de carbone,

$R^{III}$ représente un atome d'hydrogène, un groupe alkyle à chaîne droite ou ramifiée contenant de 1 à 6 atomes de carbone, un groupe phénylalkyle contenant de 1 à 4 atomes de carbone dans la fraction alkyle à chaîne droite ou ramifiée, éventuellement substitué de 1 à 5 fois de manière identique ou différente, dans lequel, comme substituants du groupe phényle, entrent

en ligne de compte, les substituants du groupe phényle mentionnés pour $R^{II}$; $R^{III}$ représente, en outre, un groupe alcoxycarbonylalkyle, un groupe carbamoylalkyle, un groupe alkylcarbamoylalkyle ou un groupe dialkylcarbamoylalkyle contenant chacun de 1 à 4 atomes de carbone dans chaque fraction alkyle ou encore un groupe cycloalkyle contenant de 3 à 6 atomes de carbone, éventuellement substitué par un groupe alkyle à chaîne droite ou ramifiée contenant de 1 à 4 atomes de carbone;

$R^{IV}$ représente un atome d'hydrogène, un groupe alkyle à chaîne droite ou ramifiée contenant de 1 à 6 atomes de carbone, un groupe phénylalkyle contenant de 1 à 4 atomes de carbone dans la fraction alkyle à chaîne droite ou ramifiée, éventuellement substitué de 1 à 5 fois de manière identique ou différente, dans lequel, comme substituants du groupe phényle, entrent

en ligne de compte, les substituants du groupe phényle mentionnés pour $R^{II}$; $R^{IV}$ représente, en outre, un groupe acyle contenant de 2 à 9 atomes de carbone, et

n représente les nombres 0, 1 ou 2,

leurs isomères géométriques et optiques, ainsi que leurs mélanges d'isomères.

**3.** Dérivés de (2-cyano-2-oximinoacétyl)-aminoacides selon la revendication 1, dans lesquels, dans la formule générale (I):

$R^1$ représente les groupements $-A-COOR^2$ ou $-B-CONR^3R^4$,

A représente les groupements $-CH_2-$, $-CH(CH_3)-$ ou $-CH_2CH_2-$,

B représente les groupements $-CH_2-$, $-CH(CH_3)-$ ou $-CH_2CH_2-$,

$R^2$ représente un groupe alkyle à chaîne droite ou ramifiée contenant de 1 à 4 atomes de carbone,

$R^3$ représente un atome d'hydrogène ou un groupe alkyle contenant de 1 à 4 atomes de carbone, à chaîne droite ou ramifiée, éventuellement substitué 1 ou 2 fois de manière identique ou différente, dans lequel, comme substituants, on mentionnera : un atome

d'halogène, un groupe cyano, $-COOR^I$, $-CONR^{II}R^{III}$, $-NR^{II}R^{III}$,$-OR^{IV}$, $-S(O)_nR^{IV}$, un groupe acyle contenant de 2 à 9 atomes de carbone, un groupe phényle éventuellement substitué de 1 à 3 fois de manière identique ou différente par un atome d'halogène et par un groupe alkyle contenant de 1 à 4 atomes de carbone, un composé hétérocyclique penta- ou hexagonal contenant de 1 à 3 hétéroatomes identiques ou différents, éventuellement substitué de 1 à 3 fois de manière identique ou différente par un atome d'halogène, par un groupe alkyle et un groupe alcoxy à chaîne droite ou ramifiée contenant chacun de 1 à 4 atomes de carbone, ainsi qu'un groupe cycloalkyle contenant de 3 à 6 atomes de carbone et un groupe cycloalcényle contenant de 5 à 7 atomes de carbone, chacun étant éventuellement substitué par un groupe alkyle à chaîne droite ou ramifiée contenant de 1 à 4 atomes de carbone; $R^3$ représente, en outre, un groupe alcényle ou un groupe alcynyle à chaîne droite ou ramifiée contenant chacun de 2 à 6 atomes de carbone; un groupe cycloalcényle contenant de 5 à 7 atomes de carbone, éventuellement substitué par un groupe alkyle à chaîne droite ou ramifiée contenant de 1 à 4 atomes de carbone; un groupe cycloalkyle contenant de 3 à 6 atomes de carbone, éventuellement substitué, dans lequel, comme substituants, on mentionnera : un atome d'halogène, un groupe alkyle et un groupe alcoxy à chaîne droite ou ramifiée contenant chacun de 1 à 4 atomes de carbone, un groupe cyano, une groupe amino, un groupe carbamoyle, un groupe alkylamino, un groupe dialkylamino, un groupe alkylcarbamoyle et un groupe dialkylcarbamoyle, chacun de ces groupes étant à chaîne droite ou ramifiée et contenant chacun de 1 à 4 atomes de carbone dans chaque fraction alkyle, un groupe alcoxycarbonylamino contenant de 1 à 4 atomes de carbone dans la fraction alcoxy à chaîne droite ou ramifiée, un groupe cycloalkyle et un groupe cycloalkylalkyle contenant chacun 5 ou 6 atomes de carbone dans la fraction cycloalkyle et de 1 à 4 atomes de carbone dans la fraction alkyle à chaîne droite ou ramifiée, un groupe phényle éventuellement substitué de 1 à 3 fois de manière identique ou différente par un atome d'halogène ou par un groupe alkyle contenant de 1 à 4 atomes de carbone et un groupe pyrrolidone; $R^3$ représente, en outre, un groupe phényle éventuellement substitué de 1 à 3 fois de manière identique ou différente par un atome d'halogène, par un groupe alkyle et un groupe alcoxy à chaîne droite ou ramifiée contenant chacun de 1 à 4 atomes de carbone, par un groupe halogénalkyle et un groupe halogénalcoxy contenant chacun 1 ou 2 atomes de carbone et de 1 à 5 atomes d'halogène identiques ou différents; ou encore représente un composé hétérocyclique penta- ou hexagonal contenant de 1 à 3 hétéroatomes identiques ou différents, éventuellement substitué de 1 à 3 fois de manière identique ou différente; comme substituants pour les composés hétérocycliques, on mentionnera : un atome d'halogène, un groupe mercapto, un groupe phényle, un groupe alkyle, un groupe alcoxy, un groupe alkylthio, un groupe alkylsulfinyle et un groupe alkylsulfonyle, chacun de ces groupes étant à chaîne droite ou ramifiée et contenant chacun de 1 à 4 atomes de carbone dans la fraction alkyle;

$R^4$ a les significations données pour $R^3$, représente des composés carbocycliques polycycliques ou le groupement $-OR^{IV}$,

ou

A et $R^2$ conjointement avec les atomes auxquels ils sont liés, représentent des cycles penta- ou hexagonaux éventuellement substitués par un groupe alkyle contenant de 1 à 4 atomes de carbone, répondant aux formules

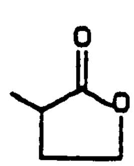

et

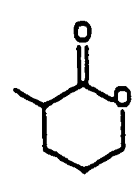

ou

B et $R^3$ représentent ensemble avec les atomes auxquels ils sont liés, des cycles penta- à

heptagonaux éventuellement substitués par un groupe alkyle contenant de 1 à 4 atomes de carbone, répondant aux formules

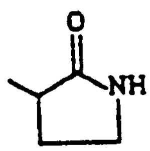 , 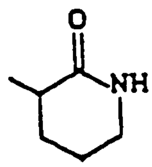 et 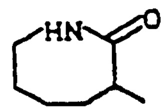

ou

$R^3$ et $R^4$ représentent, conjointement avec l'atome d'azote auquel ils sont liés, un composé hétérocyclique mono-, bi- ou tricyclique ou un composé spirohétérocyclique éventuellement substitué de 1 à 5 fois de manière identique ou différente, dans lequel, comme composés hétérocycliques, on mentionnera : l'oxazolidine, la pyrrolidine, l'imidazolidine, la pipéridine, la pipérazine, la morpholine, la thiomorpholine, le 1,3-oxazane ou le 1,3-diazane; ces composés hétérocycliques peuvent être éventuellement condensés à 1 ou 2 cycles benzéniques ou cyclohexane, ou éventuellement être reliés par un pont méthylène ou éthylène.

Comme substituants pour tous les hétérosystèmes, on mentionnera :

un groupe alkyle ou un groupe alcoxy à chaîne droite ou ramifiée contenant chacun de 1 à 4 atomes de carbone, un groupe cycloalkyle contenant de 3 à 6 atomes de carbone, le groupe hydroxy ou le groupe oxo, un groupe alcényle à chaîne droite ou ramifiée contenant de 2 à 4 atomes de carbone, un groupe hydroxycarbonyle, un groupe alcoxycarbonyle contenant de 1 à 4 atomes de carbone dans la fraction alkyle à chaîne droite ou ramifiée, un groupe carbamoyle, un groupe alkyl- ou dialkylcarbamoyle contenant chacun de 1 à 4 atomes de carbone dans chaque fraction alkyle, ainsi qu'un groupe phényle ou un groupe phénylalkyle contenant de 1 à 4 atomes de carbone dans la fraction alkyle à chaîne droite ou ramifiée, chacun étant éventuellement substitué 1 ou 2 fois de manière identique ou différente par un atome d'halogène ou par un groupe alkyle et un groupe alcoxy à chaîne droite ou ramifiée contenant chacun de 1 à 4 atomes de carbone,

R représente un groupe alkyle substitué contenant de 1 à 4 atomes de carbone à chaîne droite ou ramifiée, dans lequel, comme substituants, on mentionnera :

un atome d'halogène, $-COOR^{I}$, $-CONR^{II}R^{III}$, $-OR^{IV}$, un groupe acyle contenant de 2 à 9 atomes de carbone, des composés hétérocycliques éventuellement substitués de 1 à 3 fois de manière identique ou différente par un groupe alkyle contenant 1 ou 2 atomes de carbone, répondant aux formules

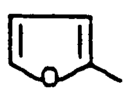 , 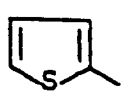 , 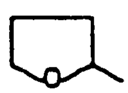 , 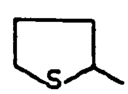 ,

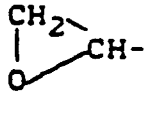 , 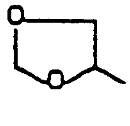 et 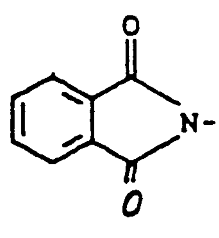 ,

R représente, en outre, un groupe cyclohexényle ou un groupe cyclopentényle éventuellement substitué de 1 à 3 fois par un groupe méthyle,

$R^{I}$ représente un groupe alkyle à chaîne droite ou ramifiée contenant de 1 à 4 atomes de carbone,

$R^{II}$ représente un atome d'hydrogène, un groupe alkyle à chaîne droite ou ramifiée contenant de 1 à 4 atomes de carbone, un groupe benzyle et un groupe phénéthyle, chacun étant éventuellement substitué de 1 à 3 fois de manière identique ou différente, dans lesquels, comme substituants du groupe phényle, on mentionnera chaque fois : un atome de fluor, un atome de chlore, un groupe méthyle, un groupe méthoxy et un groupe trifluorométhyle; $R^{II}$ représente, en outre, un groupe alcoxycarbonylalkyle contenant de 1 à 4 atomes de carbone dans la fraction alcoxy et 1 ou 2 atomes de carbone dans la fraction alkyle, un groupe carbamoylalkyle contenant 1 ou 2 atomes de carbone dans la fraction alkyle, un groupe alkylcarbamoylalkyle et un groupe dialkylcarbamoylalkyle contenant chacun 1 ou 2 atomes de carbone dans chaque fraction alkyle ou encore un groupe cycloalkyle contenant de 3 à 6 atomes de carbone, éventuellement substitué de 1 à 3 fois de manière identique ou différente par un groupe méthyle et par un groupe éthyle,

$R^{III}$ a les significations données pour $R^{II}$,

$R^{IV}$ représente un atome d'hydrogène, un groupe alkyle à chaîne droite ou ramifiée contenant de 1 à 4 atomes de carbone, un groupe benzyle ou un groupe phénéthyle, chacun étant éventuellement substitué de 1 à 3 fois de manière identique ou différente, dans lequel, comme substituants du groupe phényle, on mentionnera chaque fois : un atome de fluor, un atome de chlore, un groupe méthyle, un groupe méthoxy et un groupe trifluorométhyle; $R^{IV}$ représente, en outre, un groupe acyle contenant de 2 à 9 atomes de carbone, et

n représente les nombres 0, 1 ou 2,

leurs isomères géométriques et optiques, ainsi que leurs mélanges d'isomères.

**4.** Dérivés de (2-cyano-2-oximinoacétyl)-aminoacides selon la revendication 1, dans lesquels, dans la formule (I) :

$R^1$ représente le groupement $-B-CO-NR^3R^4$,

B représente les groupements $-CH_2-$, $-CH(CH_3)-$ ou $-CH_2CH_2-$

$R^3$ représente un atome d'hydrogène ou un groupe alkyle à chaîne droite ou ramifiée contenant de 1 à 4 atomes de carbone,

$R^4$ représente un atome d'hydrogène ou un groupe alkyle à chaîne droite ou ramifiée contenant de 1 à 4 atomes de carbone,

R représente un groupe alkyle substitué contenant 1 ou 2 atomes de carbone, dans lequel, comme substituants, on mentionnera : un atome d'halogène, un groupe alkylcarbonyle contenant de 1 à 4 atomes de carbone dans la fraction alkyle à chaîne droite ou ramifiée, les groupements $-COOR^I$, $-CONR^{II}R^{III}$, $-OR^{IV}$, ainsi que les composés hétérocycliques ci-après :

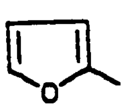 , 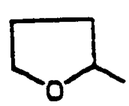 , 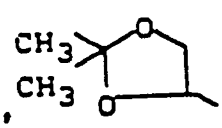 , 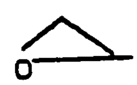 et

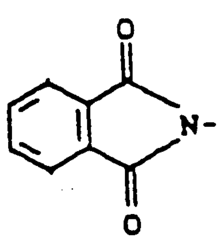 ;

R représente, en outre, un groupe 1-cyclohexényle éventuellement substitué de 1 à 3 fois par un groupe méthyle;

$R^I$ représente un groupe méthyle, un groupe éthyle, un groupe n- ou i-propyle ou un groupe n-, i-, s- ou t-butyle,

$R^{II}$ et $R^{III}$ sont identiques ou différents et représentent un atome d'hydrogène, un groupe méthyle, un groupe éthyle, un groupe n- ou i-propyle, un groupe benzyle éventuellement

substitué de 1 à 3 fois de manière identique ou différente par un atome de chlore et par un groupe méthyle à titre de substituants, un groupe alcoxycarbonylalkyle contenant 1 ou 2 atomes de carbone dans chaque fraction alkyle, un groupe carbamoylalkyle, un groupe alkylcarbamoylalkyle ou un groupe dialkylcarbamoylalkyle contenant chacun 1 ou 2 atomes de carbone dans chaque fraction alkyle ou encore un groupe cyclohexyle éventuellement substitué de 1 à 3 fois par un groupe méthyle, et

$R^{IV}$ représente un atome d'hydrogène, un groupe alkyle contenant 1 ou 2 atomes de carbone ou un groupe benzyle éventuellement substitué de 1 à 3 fois de manière identique ou différente, dans lequel, comme substituants du groupe phényle, on mentionnera : un atome de fluor, un atome de chlore et un groupe méthyle, leurs isomères géométriques et optiques, ainsi que leurs mélanges d'isomères.

**5.** Dérivés de (2-cyano-2-oximinoacétyl)-aminoacides selon la revendication 1, dans lesquels, dans la formule (I) :

$R^1$ représente le groupement $-B-CO-NR^3R^4$,

B représente les groupements $-CH_2-$, $-CH(CH_3)-$ ou $-CH_2CH_2-$

$R^3$ représente un atome d'hydrogène, un groupe alkyle à chaîne droite ou ramifiée contenant de 1 à 4 atomes de carbone ou un groupe alkyle contenant de 1 à 3 atomes de carbone, substitué 1 ou 2 fois, dans lequel, comme substituants, on mentionnera : un atome de chlore, un groupe hydroxyle, un groupe alkylcarbonyloxy contenant de 1 à 4 atomes de carbone dans la fraction alkyle à chaîne droite ou ramifiée, un groupe alkylcarbonyle contenant de 1 à 4 atomes de carbone dans la fraction alkyle à chaîne droite ou ramifiée, un groupe alcoxy et un groupe alkylthio contenant chacun 1 ou 2 atomes de carbone, un groupe phényle éventuellement substitué de 1 à 3 fois de manière identique ou différente par un atome de chlore et par un groupe méthyle, un groupe 2-furyle, un groupe 2-pyridyle, un groupe 1-morpholino, un groupe cyclopropyle et un groupe cyclohexyle; $R^3$ représente, en outre, un groupe allyle ou un groupe propargyle, un groupe 1-cyclohexényle ou un groupe cyclohexyle substitué de 1 à 3 fois de manière identique ou différente, dans lequel, comme substituants, on mentionnera : un atome de chlore, un groupe méthyle, un groupe éthyle, un groupe méthoxy, un groupe cyano, un groupe amino, un groupe alkyl- ou dialkylamino contenant chacun 1 ou 2 atomes de carbone dans chaque fraction alkyle, un groupe carbamoyle, un groupe alkyl- ou dialkylcarbamoyle contenant chacun 1 ou 2 atomes de carbone dans chaque fraction alkyle, un groupe cyclohexyle, un groupe cyclohexylalkyle contenant 1 ou 2 atomes de carbone dans la fraction alkyle ou encore une 1-pyrrolidin-2-one;

$R^3$ représente, en outre, un groupe phényle éventuellement substitué de 1 à 3 fois de manière identique ou différente par un atome de chlore et par un groupe méthyle, un groupe 2-pyridyle ou un groupe 2-pyrimidinyle, chacun étant éventuellement substitué de 1 à 3 fois par un groupe méthyle, un groupe 2-thiazolyle éventuellement substitué par un groupe phényle, un groupe 2-benzothiazolyle éventuellement substitué de 1 à 3 fois de manière identique ou différente par un atome de chlore, par un groupe méthyle et par un groupe méthoxy, un groupe 1,2,4-triazol-3-yle, un groupe 1,2,4-thiadiazol-5-yle éventuellement substitué par un groupe phényle, un groupe 1,3,4-thiadiazol-5-yle éventuellement substitué de 1 à 3 fois de manière identique ou différente par un groupe mercapto, par un groupe alkyle à chaîne droite ou ramifiée contenant de 1 à 4 atomes de carbone, par un groupe alkylthio, par un groupe alkylsulfinyle et un groupe alkylsulfonyle, chacun étant à chaîne droite ou ramifiée et contenant chacun de 1 à 4 atomes de carbone, ou encore représente le groupement

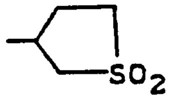 ,

$R^4$ représente un groupe alkyle contenant de 1 à 3 atomes de carbone, substitué 1 ou 2 fois, dans lequel, comme substituants, on mentionnera : un atome de chlore, un groupe hydroxyle, un groupe alkylcarbonyloxy contenant de 1 à 4 atomes de carbone dans la fraction alkyle à chaîne droite ou ramifiée, un groupe alkylcarbonyle contenant de 1 à 4 atomes de carbone dans la fraction alkyle à chaîne droite ou ramifiée, un groupe alcoxy et un groupe alkylthio

contenant chacun 1 ou 2 atomes de carbone, un groupe phényle éventuellement substitué de 1 à 3 fois de manière identique ou différente par un atome de chlore et par un groupe méthyle, un groupe 2-furyle, un groupe 2-pyridyle, un groupe 1-morpholino, un groupe cyclopropyle et un groupe cyclohexyle; $R^4$ représente, en outre, un groupe allyle ou un groupe propargyle, un groupe 1-cyclohexényle ou un groupe cyclohexyle substitué de 1 à 3 fois de manière identique ou différente, dans lequel, comme substituants, on mentionnera : un atome de chlore, un groupe méthyle, un groupe éthyle, un groupe méthoxy, un groupe cyano, un groupe amino, un groupe alkyl- ou dialkylamino contenant chacun 1 ou 2 atomes de carbone dans chaque fraction alkyle, un groupe carbamoyle, un groupe alkyl- ou dialkylcarbamoyle contenant chacun 1 ou 2 atomes de carbone dans chaque fraction alkyle, un groupe cyclohexyle, un groupe cyclohexylalkyle contenant 1 ou 2 atomes de carbone dans la fraction alkyle, ainsi que la 1-pyrrolidin-2-one;

$R^4$ représente, en outre, un groupe phényle éventuellement substitué de 1 à 3 fois de manière identique ou différente par un atome de chlore et par un groupe méthyle, un groupe 2-pyridyle ou un groupe 2-pyrimidinyle, chacun étant éventuellement substitué de 1 à 3 fois par un groupe méthyle, un groupe 2-thiazolyle éventuellement substitué par un groupe phényle, un groupe 2-benzothiazolyle éventuellement substitué de 1 à 3 fois de manière identique ou différente par un atome de chlore, par un groupe méthyle et par un groupe méthoxy, un groupe 1,2,4-triazol-3-yle, un groupe 1,2,4-thiadiazol-5-yle éventuellement substitué par un groupe phényle, un groupe 1,3,4-thiadiazol-5-yle éventuellement substitué de 1 à 3 fois de manière identique ou différente par un groupe mercapto, par un groupe alkyle à chaîne droite ou ramifiée contenant de 1 à 4 atomes de carbone, par un groupe alkylthio, par un groupe alkylsulfinyle et un groupe alkylsulfonyle, chacun étant à chaîne droite ou ramifiée et contenant chacun de 1 à 4 atomes de carbone, ou encore représente le groupement

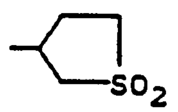 ,

$R^4$ représente, en outre, un groupe hydroxyle, un groupe alcoxy contenant 1 ou 2 atomes de carbone, un groupe benzyloxy éventuellement substitué de 1 à 3 fois de manière identique ou différente par un atome de chlore et par un groupe méthyle, ainsi qu'un groupe 1-adamantyle, un groupe 2-norbornyle, un groupe 1- ou 2-décalyle ou encore un groupe 1- ou 2-tétralyle;

R représente un groupe alkyle substitué contenant 1 ou 2 atomes de carbone, dans lequel, comme substituants, on mentionnera : un atome d'halogène, un groupe alkylcarbonyle contenant de 1 à 4 atomes de carbone dans la fraction alkyle, les groupements $-COOR^{I}$, $-CONR^{II}R^{III}$, $-OR^{IV}$, ainsi que les composés hétérocycliques ci-après :

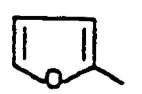 , 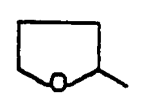 , 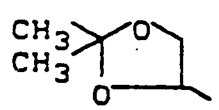 , 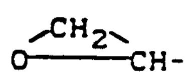 et

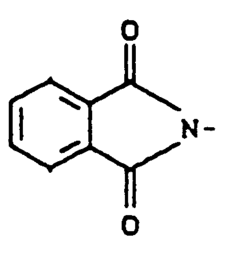 ;

R représente, en outre, un groupe 1-cyclohexényle éventuellement substitué de 1 à 3 fois

par un groupe méthyle;

$R^I$ représente un groupe méthyle, un groupe éthyle, un groupe n- ou i-propyle ou un groupe n-, i-, s- ou t-butyle,

$R^{II}$ et $R^{III}$ sont identiques ou différents et représentent un atome d'hydrogène, un groupe méthyle, un groupe éthyle, un groupe n- ou i-propyle, un groupe benzyle éventuellement substitué de 1 à 3 fois de manière identique ou différente par un atome de chlore ou par un groupe méthyle à titre de substituants, un groupe alcoxycarbonylalkyle contenant 1 ou 2 atomes de carbone dans chaque fraction alkyle, un groupe carbamoylalkyle, un groupe alkylcarbamoylalkyle ou un groupe dialkylcarbamoylalkyle contenant chacun 1 ou 2 atomes de carbone dans chaque fraction alkyle ou encore un groupe cyclohexyle éventuellement substitué de 1 à 3 fois par un groupe méthyle, et

$R^{IV}$ représente un atome d'hydrogène, un groupe alkyle contenant 1 ou 2 atomes de carbone ou encore un groupe benzyle éventuellement substitué de 1 à 3 fois de manière identique ou différente, dans lequel, comme substituants du groupe phényle, on mentionnera : un atome de fluor, un atome de chlore et un groupe méthyle,

leurs isomères géométriques et optiques, ainsi que leurs mélanges d'isomères.

**6.** Dérivés de (2-cyano-2-oximinoacétyl)-aminoacides selon la revendication 1, dans lesquels, dans la formule (I) :

$R^1$ représente le groupement -B-CO-$NR^3R^3$,

B représente les groupements $-CH_2-$, $-CH(CH_3)-$ ou $-CH_2CH_2-$

$R^3$ et $R^4$ représentent, conjointement avec l'atome d'azote auquel ils sont liés, les composés hétérocycliques mono-, bi- ou tricycliques ou les composés spirohétérocycliques ci-après :

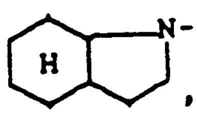 , 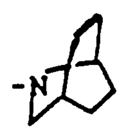 , 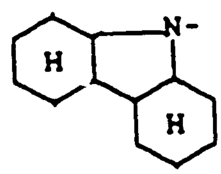 ,

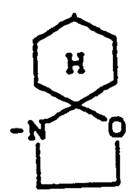 , 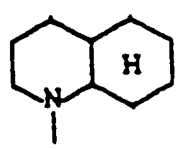 ,

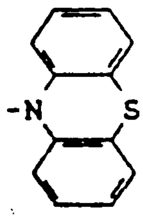 , 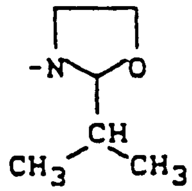 , 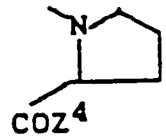

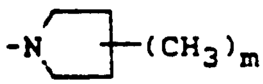 , 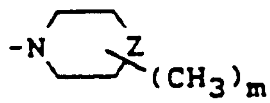

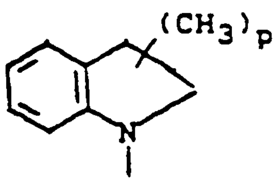 , 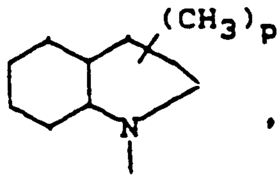 ,

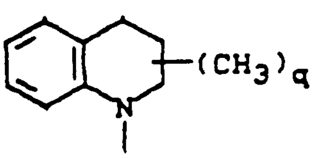 , 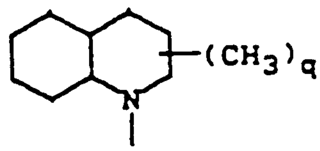 ,

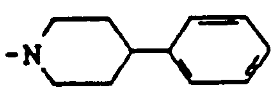 , 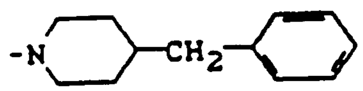 ,

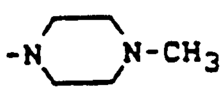 , 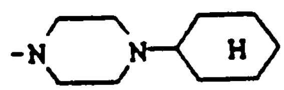 , 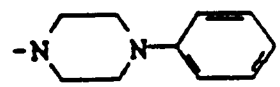 ,

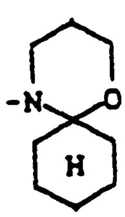 , 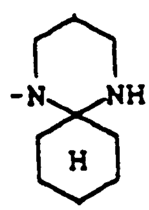 , 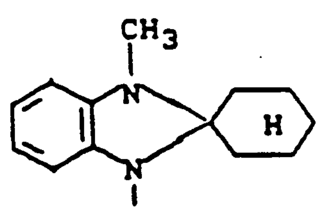 ,

dans lesquels

- Z représente un atome d'oxygène ou le groupe $CH_2$,
- $Z^4$ représente un groupe hydroxyle, un groupe méthoxy, un groupe éthoxy, un groupe amino, un groupe méthylamino, un groupe éthylamino, un groupe diméthylamino ou un groupe éthylméthylamino,
- m représente les nombres 0, 1, 2, 3 ou 4,
- p représente les nombres 0, 1 ou 2 et
- q représente les nombres 0, 1, 2 ou 3 et
- R représente un groupe alkyle substitué contenant 1 ou 2 atomes de carbone, dans lequel, comme substituants, on mentionnera : un atome d'halogène, un groupe alkylcarbonyle contenant de 1 à 4 atomes de carbone dans la fraction alkyle à chaîne droite ou

ramifiée, les groupements -COOR$^{I}$, -CONR$^{II}$R$^{III}$, -OR$^{IV}$, ainsi que les composés hétérocycliques ci-après :

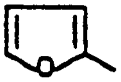 , 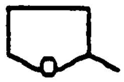 , 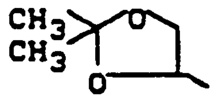 , 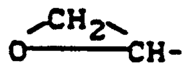 et

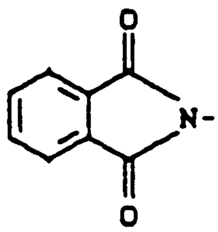 ;

R représente, en outre, un groupe 1-cyclohexényle éventuellement substitué de 1 à 3 fois par un groupe méthyle;

R$^{I}$ représente un groupe méthyle, un groupe éthyle, un groupe n- ou i-propyle ou un groupe n-, i-, s- ou t-butyle,

R$^{II}$ et R$^{III}$ sont identiques ou différents et représentent un atome d'hydrogène, un groupe méthyle, un groupe éthyle, un groupe n- ou i-propyle, un groupe benzyle éventuellement substitué de 1 à 3 fois de manière identique ou différente par un atome de chlore et par un groupe méthyle à titre de substituants, un groupe alcoxycarbonylalkyle contenant 1 ou 2 atomes de carbone dans chaque fraction alkyle, un groupe carbamoylalkyle, un groupe alkylcarbamoylalkyle ou un groupe dialkylcarbamoylalkyle contenant chacun 1 ou 2 atomes de carbone dans chaque fraction alkyle ou encore un groupe cyclohexyle éventuellement substitué de 1 à 3 fois par un groupe méthyle, et

R$^{IV}$ représente un atome d'hydrogène, un groupe alkyle contenant 1 ou 2 atomes de carbone ou un groupe benzyle éventuellement substitué de 1 à 3 fois de manière identique ou différente, dans lequel, comme substituants du groupe phényle, on mentionnera : un atome de fluor, un atome de chlore et un groupe méthyle,

leurs isomères géométriques et optiques, ainsi que leurs mélanges d'isomères.

**7.** Dérivés de (2-cyano-2-oximinoacétyl)-aminoacides selon la revendication 1, dans lesquels, dans la formule (I) :

$R^1$ représente le groupement -A-COOR$^2$,

A représente les groupements $-CH_2-$, $-CH(CH_3)-$ ou $-CH_2CH_2-$

$R^2$ représente un groupe méthyle ou un groupe éthyle,

R représente un groupe alkyle contenant 1 ou 2 atomes de carbone, substitué 1 ou 2 fois, dans lequel, comme substituants, on mentionnera : un atome d'halogène, un groupe alkylcarbonyle contenant de 1 à 4 atomes de carbone dans la fraction alkyle à chaîne droite ou ramifiée, -COOR$^{I}$, -CONR$^{II}$R$^{III}$, -OR$^{IV}$, ainsi que les composés hétérocycliques ci-après :

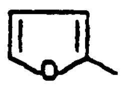 , 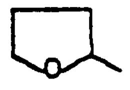 , 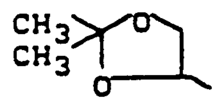 , 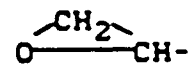

et

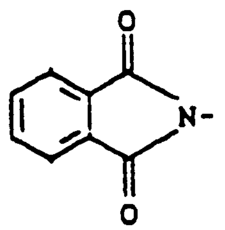 ;

R représente, en outre, un groupe 1-cyclohexényle éventuellement substitué de 1 à 3 fois par un groupe méthyle;

$R^I$ représente un groupe méthyle, un groupe éthyle, un groupe n- ou i-propyle ou un groupe n-, i-, s- ou t-butyle,

$R^{II}$ et $R^{III}$ sont identiques ou différents et représentent un atome d'hydrogène, un groupe méthyle, un groupe éthyle, un groupe n- ou i-propyle, un groupe benzyle éventuellement substitué de 1 à 3 fois de manière identique ou différente par un atome de chlore et par un groupe méthyle à titre de substituants, un groupe alcoxycarbonylalkyle contenant 1 ou 2 atomes de carbone dans chaque fraction alkyle, un groupe carbamoylalkyle, un groupe alkylcarbamoylalkyle ou un groupe dialkylcarbamoylalkyle contenant chacun 1 ou 2 atomes de carbone dans chaque fraction alkyle ou encore un groupe cyclohexyle éventuellement substitué de 1 à 3 fois par un groupe méthyle, et

$R^{IV}$ représente un atome d'hydrogène, un groupe alkyle contenant 1 ou 2 atomes de carbone ou un groupe benzyle éventuellement substitué de 1 à 3 fois de manière identique ou différente, dans lequel, comme substituants du groupe phényle, on mentionnera : un atome de fluor, un atome de chlore et un groupe méthyle,

leurs isomères géométriques et optiques, ainsi que leurs mélanges d'isomères.

**8.** Procédé pour la préparation de dérivés de (2-cyano-2-oximinoacétyl)-aminoacides répondant à la formule générale (I) :

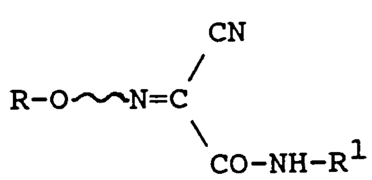 (I)

dans laquelle

$R^1$ représente les groupements $-A-COOR^2$ ou $-B-CONR^3R^4$

A représente une chaîne alkylène droite ou ramifiée éventuellement substituée,

B représente une chaîne alkylène droite ou ramifiée éventuellement substituée,

$R^2$ représente un groupe alkyle éventuellement substitué avec les substituants ci-après : un atome d'halogène, un groupe hydroxyle, un groupe alcoxy, un groupe acyloxy, un groupe aryle éventuellement substitué, un groupe cycloalkyle éventuellement substitué et un groupe hétérocyclyle éventuellement substitué; $R^2$ représente, en outre, un groupe cycloalkyle éventuellement substitué ou un groupe cycloalcényle éventuellement substitué,

$R^3$ représente un atome d'hydrogène ou un groupe alkyle éventuellement substitué avec les substituants ci-après : un atome d'halogène, un groupe cyano, $-COOR^I$, $-CONR^{II}R^{III}$, $-NR^{II}R^{III}$, $-OR^{IV}$, $-S(O)_nR^{IV}$, un groupe acyle; un groupe aryle, un groupe hétérocyclyle, un groupe cycloalkyle et un groupe cycloalcényle, chacun étant éventuellement substitué;

$R^3$ représente, en outre, un groupe alcényle, un groupe alcynyle, un groupe cycloalkyle, un groupe cycloalcényle, un groupe aryle ou un groupe hétérocyclyle, chacun étant éventuellement substitué,

$R^4$ représente un atome d'hydrogène ou un groupe alkyle éventuellement substitué avec les substituants ci-après : un atome d'halogène, un groupe cyano, $-COOR^I$, $-CONR^{II}R^{III}$, $-NR^{II}R^{III}$,$-OR^{IV}$, $-S(O)_nR^{IV}$, un groupe acyle; un groupe aryle, un groupe hétérocyclyle, un groupe cycloalkyle et un groupe cycloalcényle, chacun étant éventuellement substitué; $R^4$ représente, en outre, un groupe alcényle, un groupe alcynyle, un groupe cycloalkyle, un groupe cycloalcényle, un groupe aryle, un groupe hétérocyclyle, chacun étant éventuellement substitué, ou bien des composés carbocycliques polycycliques, ou encore le groupement $-OR^{IV}$,

ou

A et $R^2$ représentent, conjointement avec les atomes auxquels ils sont liés, un noyau éventuellement substitué,

ou

B et $R^3$ représentent, conjointement avec les atomes auxquels ils sont liés, un noyau éventuellement substitué,

ou

$R^3$ et $R^4$ représentent, conjointement avec l'atome d'azote auquel ils sont liés, un composé hétérocyclique éventuellement substitué, qui peut contenir d'autres hétéroatomes,

R représente un groupe alkyle substitué avec les substituants ci-après : un atome d'halogène, $-COOR^I$, $-CONR^{II}R^{III}$, $-OR^{IV}$, un groupe acyle ou un groupe hétérocyclyle éventuellement substitué, à l'exception d'un groupe azolyle; R représente, en outre, un groupe cycloalcényle éventuellement substitué;

$R^I$ représente un atome d'hydrogène ou un groupe alkyle éventuellement substitué avec les substituants ci-après: un atome d'halogène, un groupe aryle, un groupe cycloalkyle et un groupe cycloalcényle, chacun de ces groupes étant éventuellement substitué,

$R^{II}$ et $R^{III}$ sont identiques ou différents, et représentent un atome d'hydrogène ou un groupe alkyle éventuellement substitué avec les substituants ci-après : un atome d'halogène, $-COOR^{IV,}$ $-CONR^IR^{IV,}$ un groupe aryle, un groupe cycloalkyle et un groupe cycloalcényle, chacun de ces groupes étant éventuellement substitué; $R^{II}$ et $R^{III}$ représentent, en outre, un groupe alcényle, un groupe alcynyle; ou encore un groupe cycloalkyle, un groupe cycloalcényle, un groupe aryle ou un groupe hétérocyclyle, chacun de ces groupes étant éventuellement substitué; ou encore représentent, conjointement avec l'atome d'azote auquel ils sont liés, un composé hétérocyclique éventuellement substitué, qui peut contenir d'autres hétéroatomes,

$R^{IV}$ représente un atome d'hydrogène, un groupe alkyle, un groupe aralkyle ou un groupe acyle et

n représente les nombres 0, 1 ou 2.

leurs isomères géométriques et optiques, ainsi que leurs mélanges d'isomères, caractérisé en ce qu'on fait réagir

a) des esters d'acides 2-cyano-2-oximinoacétiques de formule générale (II)

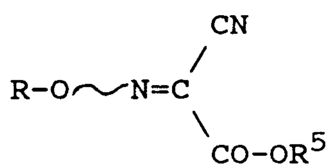

(II)

dans laquelle

R a la signification mentionnée ci-dessus et

$R^5$ représente un groupe alkyle,

avec des amines de formule (III)

$R^1$-$NH_2$ (III)

dans laquelle

$R^1$ a la signification mentionnée ci-dessus,

éventuellement en présence d'un diluant et éventuellement en présence d'une base;

ou

b) des dérivés d'acides carboxyliques activés par un groupe carboxyle, répondant à la formule générale (IV)

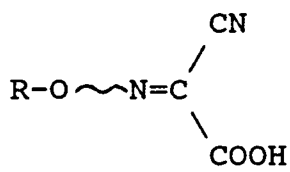

(IV)

dans laquelle

R a la signification mentionnée ci-dessus,

avec des amines de formule (III)

$R^1$-$NH_2$ (III)

dans laquelle

$R^1$ a la signification mentionnée ci-dessus,

éventuellement en présence d'un catalyseur et éventuellement en présence d'un agent neutralisateur d'acides, ainsi qu'éventuellement en présence d'un diluant;

ou

c) des 2-cyano-2-oximinoacétamides de formule générale (V)

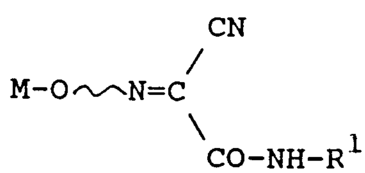

(V)

dans laquelle

M représente un atome d'hydrogène, un ion de métal alcalin, une base tertlaire protonée ou un ion d'ammonium quaternaire et

$R^1$ a la signification mentiannée ci-dessus,

avec des composés de formule (VI)

R-X (VI)

dans laquelle

X représente un atome d'halogène ou un radical sulfonyloxy et

R a la signification mentionnée ci-dessus,

éventuellement en présence d'une base et éventuellement en présence d'un diluant;

ou

d) des esters d'acétylaminoacides substitués, répondant à la formule générale (VII)

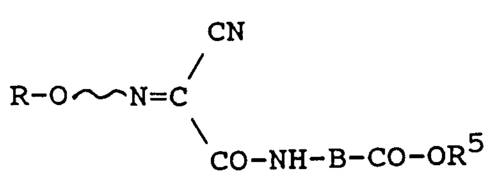

(VII)

dans laquelle
B, R et $R^5$ ont la signification mentionnée ci-dessus,
avec des amines de formule (VIII)

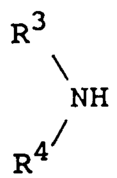

(VIII)

dans laquelle
$R^3$ et $R^4$ ont la signification mentionnée ci-dessus,
éventuellement en présence d'un diluant et éventuellement en présence d'une base;
ou
e) des acétylaminoacides activés par un groupe carboxyle, répondant à la formule générale (IX)

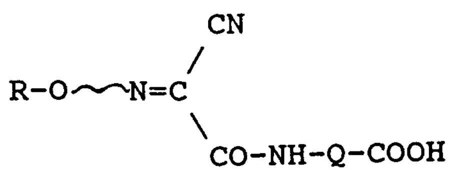

(IX)

dans laquelle
R a la signification mentionnée ci-dessus et
Q représente A ou B, ces derniers ayant la signification indiquée ci-dessus,
avec des composés de formule (X)

Y-H (X)

dans laquelle
Y représente les groupements $R^2O$- ou $R^3R^4N$-, $R^2$, $R^3$ et $R^4$ ayant la signification indiquée ci-dessus,
éventuellement en présence d'un catalyseur et éventuellement en présence d'un agent neutralisateur d'acides, ainsi qu'éventuellement en présence d'un diluant;
ou
f) des sels d'ammonium d'acides 2-cyano-2-oximinoacétiques, répondant à la formule générale (XI)

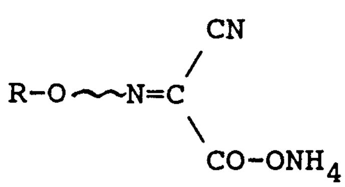

(XI)

dans laquelle
R a la signification indiquée ci-dessus,
avec des aldéhydes de formule (XII)

$R^6$-CH=O (XII)

dans laquelle
le groupement $R^6$-CH= a la signification donnée ci-dessus pour A ou B,
et avec des isonitriles de formule (XIII)

$$|\overset{\ominus}{C}\equiv \overset{\oplus}{N}-R^4 \qquad (XIII)$$

dans laquelle

$R^4$ a la signification mentionnée ci-dessus,

éventuellement en présence d'un diluant;

ou

g) des acides 2-cyano-2-oximinoacétiques répondant à la formule générale (IV)

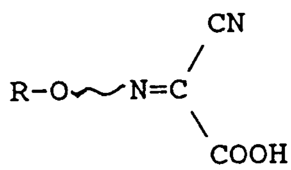

(IV)

dans laquelle

R a la signification mentionnée ci-dessus,

avec des isocyanates de formule (XIV)

$R^1$-NCO (XIV)

dans laquelle

$R^1$ a la signification mentionnée ci-dessus,

éventuellement en présence d'un catalyseur et éventuellement en présence d'un diluant;

ou

h) des 2-cyano-2-oximinoacétamides répondant à la formule générale (XV)

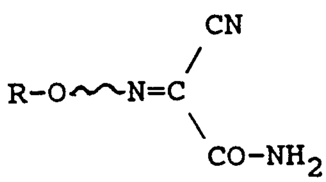

(XV)

dans laquelle

R a la signification mentionnée ci-dessus,

avec une base, éventuellement en présence d'un diluant, et on fait réagir les sels que l'on obtient, directement ou éventuellement après isolation intermédiaire, avec des composés de formule (XVI)

$R^1$-X (XVI)

dans laquelle

$R^1$ et X ont la signification mentionnée ci-dessus,

éventuellement en présence d'un diluant.

**9.** Agents de lutte contre les parasites, caractérisés par une teneur en au moins un dérivé de (2-cyano-2-oximinoacétyl)-aminoacides de formule (I) selon les revendications 1 et 8.

**10.** Utilisation de dérivés de (2-cyano-2-oximinoacétyl)-aminoacides de formule (I) selon les revendications 1 et 8, pour lutter contre les parasites.

**11.** Procédé pour lutter contre les parasites, caractérisé en ce qu'on laisse agir des dérivés de (2-cyano-2-oximinoacétyl)-aminoacides de formule (I) selon les revendications 1 et 8, sur des parasites et/ou sur leur biotope.

**12.** Procédé pour la préparation d'agents de lutte contre les parasites, caractérisé en ce qu'on mélange des dérivés de (2-cyano-2-oximinoacétyl)-aminoacides de formule (I) selon les revendications 1 et 8, avec des diluants et/ou des agents tensioactifs.

**Revendications pour l'Etat contractant suivant : ES**

**1.** Procédé pour la préparation de dérivés de (2-cyano-2-oximinoacétyl)-aminoacides répondant à la formule (I) :

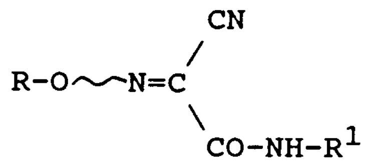

(I)

dans laquelle

| | |
|---|---|
| $R^1$ | représente les groupements $-A-COOR^2$ ou $-B-CONR^3R^4$ |
| A | représente une chaîne alkylène droite ou ramifiée éventuellement substituée, |
| B | représente une chaîne alkylène droite ou ramifiée éventuellement substituée, |
| $R^2$ | représente un groupe alkyle éventuellement substitué avec les substituants ci-après : un atome d'halogène, un groupe hydroxyle, un groupe alcoxy, un groupe acyloxy, un groupe aryle éventuellement substitué, un groupe cycloalkyle éventuellement substitué et un groupe hétérocyclyle éventuellement substitué; $R^2$ représente, en outre, un groupe cycloalkyle éventuellement substitué ou un groupe cycloalcényle éventuellement substitué, |
| $R^3$ | représente un atome d'hydrogène ou un groupe alkyle éventuellement substitué avec les substituants ci-après : un atome d'halogène, un groupe cyano, $-COOR^I$, $-CONR^{II}R^{III}$, $-NR^{II}R^{III}$,$-OR^{IV}$, $-S(O)_nR^{IV}$, un groupe acyle; un groupe aryle, un groupe hétérocyclyle, un groupe cycloalkyle et un groupe cycloalcényle, chacun étant éventuellement substitué; $R^3$ représente, en outre, un groupe alcényle, un groupe alcynyle, un groupe cycloalkyle, un groupe cycloalcényle, un groupe aryle ou un groupe hétérocyclyle, chacun étant éventuellement substitué, |
| $R^4$ | représente un atome d'hydrogène ou un groupe alkyle éventuellement substitué avec les substituants ci-après : un atome d'halogène, un groupe cyano, $-COOR^I$, $-CONR^{II}R^{III}$, $-NR^{II}R^{III}$,$-OR^{IV}$, $-S(O)_nR^{IV}$,un groupe acyle; un groupe aryle, un groupe hétérocyclyle, un groupe cycloalkyle et un groupe cycloalcényle, chacun étant éventuellement substitué; $R^4$ représente, en outre, un groupe alcényle, un groupe alcynyle, un groupe cycloalkyle, un groupe cycloalcényle, un groupe aryle, un groupe hétérocyclyle, chacun étant éventuellement substitué, ou bien des composés carbocycliques polycycliques, ou encore le groupement $-OR^{IV}$, |
| | ou |
| A et $R^2$ | représentent, conjointement avec les atomes auxquels ils sont liés, un noyau éventuellement substitué, |
| | ou |
| B et $R^3$ | représentent, conjointement avec les atomes auxquels ils sont liés, un noyau éventuellement substitué, |
| | ou |
| $R^3$ et $R^4$ | représentent, conjointement avec l'atome d'azote auquel ils sont liés, un composé hétérocyclique éventuellement substitué, qui peut contenir d'autres hétéroatomes, |
| R | représente un groupe alkyle substitué avec les substituants ci-après : un atome d'halogène, $-COOR^I$,$-CONR^{II}R^{III}$, $-OR^{IV}$, un groupe acyle ou un groupe hétérocyclyle éventuellement substitué, à l'exception d'un groupe azolyle; R représente, en outre, un groupe cycloalcényle éventuellement substitué; |
| $R^I$ | représente un atome d'hydrogène ou un groupe alkyle éventuellement substitué avec les substituants ci-après: un atome d'halogène, un groupe aryle, un groupe cycloalkyle et un groupe cycloalcényle, chacun de ces groupes étant éventuellement substitué, |

$R^{II}$ et $R^{III}$ sont identiques ou différents, et représentent un atome d'hydrogène ou un groupe alkyle éventuellement substitué avec les substituants ci-après : un atome d'halogène, $-COOR^{IV}$, $-CONR^{I}R^{IV}$, un groupe aryle, un groupe cycloalkyle et un groupe cycloalcényle, chacun de ces groupes étant éventuellement substitué; $R^{II}$ et $R^{III}$ représentent, en outre, un groupe alcényle, un groupe alcynyle; ou encore un groupe cycloalkyle, un groupe cycloalcényle, un groupe aryle ou un groupe hétérocyclyle, chacun de ces groupes étant éventuellement substitué; ou encore représentent, conjointement avec l'atome d'azote auquel ils sont liés, un composé hétérocyclique éventuellement substitué, qui peut contenir d'autres hétéroatomes,

$R^{IV}$ représente un atome d'hydrogène, un groupe alkyle, un groupe aralkyle ou un groupe acyle et

n représente les nombres 0, 1 ou 2.

leurs isomères géométriques et optiques, ainsi que leurs mélanges d'isomères, caractérisé en ce qu'on fait réagir

a) des esters d'acides 2-cyano-2-oximinoacétiques de formule générale (II)

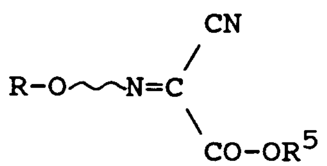

(II)

dans laquelle

R a la signification mentionnée ci-dessus et

$R^5$ représente un groupe alkyle,

avec des amines de formule (III)

$R^1-NH_2$ (III)

dans laquelle

$R^1$ a la signification mentionnée ci-dessus,

éventuellement en présence d'un diluant et éventuellement en présence d'une base;

ou

b) des dérivés d'acides carboxyliques activés par un groupe carboxyle, répondant à la formule générale (IV)

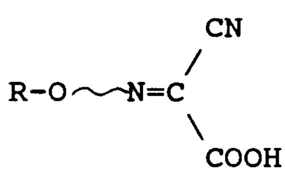

(IV)

dans laquelle

R a la signification mentionnée ci-dessus,

avec des amines de formule (III)

$R^1-NH_2$ (III)

dans laquelle

$R^1$ a la signification mentionnée ci-dessus,

éventuellement en présence d'un catalyseur et éventuellement en présence d'un agent neutralisateur d'acides, ainsi qu'éventuellement en présence d'un diluant;

ou

c) des 2-cyano-2-oximinoacétamides de formule générale (V)

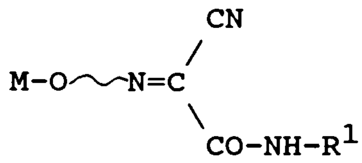

(V)

dans laquelle

M représente un atome d'hydrogène, un ion de métal alcalin, une base tertiaire protonée ou un ion d'ammonium quaternaire et

$R^1$ a la signification mentionnée ci-dessus,

avec des composés de formule (VI)

R-X (VI)

dans laquelle

X représente un atome d'halogène ou un radical sulfonyloxy et

R a la signification mentionnée ci-dessus,

éventuellement en présence d'une base et éventuellement en présence d'un diluant;

ou

d) des esters d'acétylaminoacides substitués, répondant à la formule générale (VII)

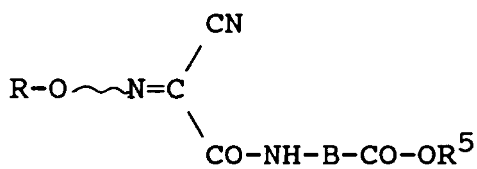

(VII)

dans laquelle

B, R et $R^5$ ont la signification mentionnée ci-dessus,

avec des amines de formule (VIII)

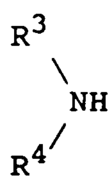

(VIII)

dans laquelle

$R^3$ et $R^4$ ont la signification mentionnée ci-dessus,

éventuellement en présence d'un diluant et éventuellement en présence d'une base;

ou

e) des acétylaminoacides activés par un groupe carboxyle, répondant à la formule générale (IX)

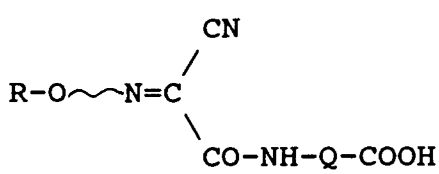

(IX)

dans laquelle

R a la signification mentionnée ci-dessus et

Q représente A ou B, ces derniers ayant la signification indiquée ci-dessus,

avec des composés de formule (X)

Y-H (X)

dans laquelle

Y représente les groupements $R^2O$- ou $R^3R^4N$-, $R^2$, $R^3$ et $R^4$ ayant la signification indiquée ci-dessus,

éventuellement en présence d'un catalyseur et éventuellement en présence d'un agent neutralisateur d'acides, ainsi qu'éventuellement en présence d'un diluant;

ou

f) des sels d'ammonium d'acides 2-cyano-2-oximinoacétiques, répondant à la formule générale (XI)

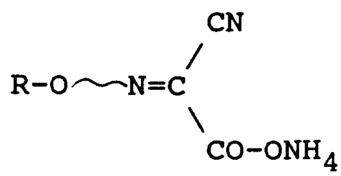

(XI)

dans laquelle

R a la signification indiquée ci-dessus,

avec des aldéhydes de formule (XII)

$R^6$-CH=O (XII)

dans laquelle

le groupement $R^6$-CH= a la signification donnée ci-dessus pour A ou B,

et avec des isonitriles de formule (XIII)

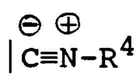

(XIII)

dans laquelle

$R^4$ a la signification mentionnée ci-dessus,

éventuellement en présence d'un diluant;

ou

g) des acides 2-cyano-2-oximinoacétiques répondant à la formule générale (IV)

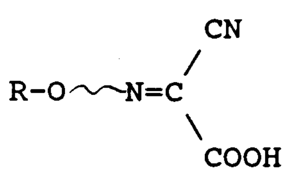

(IV)

dans laquelle

R a la signification mentionnée ci-dessus,

avec des isocyanates de formule (XIV)

$R^1$-NCO (XIV)

dans laquelle

$R^1$ a la signification mentionnée ci-dessus,

éventuellement en présence d'un catalyseur et éventuellement en présence d'un diluant;

ou

h) des 2-cyano-2-oximinoacétamides répondant à la formule générale (XV)

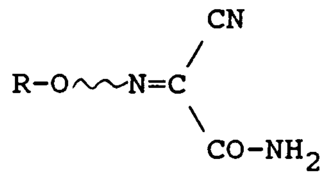

(XV)

dans laquelle

R a la signification mentionnée ci-dessus,

avec une base, éventuellement en présence d'un diluant, et on fait réagir les sels que l'on obtient, directement ou éventuellement après isolation intermédiaire, avec des composés de formule (XVI)

$R^1$-X (XVI)

dans laquelle

$R^1$ et X ont la signification mentionnée ci-dessus,

éventuellement en présence d'un diluant.

**2.** Procédé selon la revendication 1, dans laquelle, dans la formule (I)

$R^1$ représente les groupements -A-$COOR^2$ ou -B-$CONR^3R^4$,

A représente une chaîne alkylène droite ou ramifiée contenant de 1 à 4 atomes de carbone,

B représente une chaîne alkylène droite ou ramifiée contenant de 1 à 4 atomes de carbone,

$R^2$ représente un groupe alkyle à chaîne droite ou ramifiée contenant de 1 à 6 atomes de carbone, éventuellement substitué, dans lequel, comme substituants, il convient de mentionner: un atome d'halogène, un groupe phényle éventuellement substitué de 1 à 5 fois de manière identique ou différente par un atome d'halogène, par un groupe alkyle et un groupe alcoxy contenant chacun de 1 à 4 atomes de carbone, ainsi qu'un groupe cycloalkyle contenant de 3 à 6 atomes de carbone; en outre, $R^2$ représente un groupe cycloalkyle contenant de 3 à 6 atomes de carbone, éventuellement substitué de 1 à 5 fois de manière identique ou différente par un groupe alkyle contenant de 1 à 4 atomes de carbone, ou encore un groupe cycloalcényle contenant de 5 à 7 atomes de carbone, éventuellement substitué de 1 à 5 fois de manière identique ou différente par un groupe alkyle contenant de 1 à 4 atomes de carbone;

$R^3$ représente un atome d'hydrogène ou un groupe alkyle à chaîne droite ou ramifiée contenant de 1 à 4 atomes de carbone, éventuellement substitué 1 ou 2 fois, dans lequel, comme substituants, il convient de mentionner:

un atome d'halogène, un groupe cyano, $-COOR^{I}$, $-CONR^{II}R^{III}$, $-NR^{II}R^{III}$, $-OR^{IV}$, $-S(O)_nR^{IV}$, un groupe acyle contenant de 2 à 9 atomes de carbone, un groupe phényle éventuellement substitué de 1 à 5 fois de manière identique ou différente par un atome d'halogène et par un groupe alkyle contenant de 1 à 4 atomes de carbone, un composé hétérocyclique penta- ou hexagonal contenant de 1 à 3 hétéroatomes, éventuellement substitué de 1 à 5 fois de manière identique ou différente par un atome d'halogène, de même que par un groupe alkyle et un groupe alcoxy contenant chacun de 1 à 4 atomes de carbone; un groupe cycloalkyle contenant de 3 à 6 atomes de carbone ou un groupe cycloalcényle contenant de 5 à 7 atomes de carbone, chacun de ces groupes étant éventuellement substitué de 1 à 5 fois de manière identique ou différente par un groupe alkyle contenant de 1 à 4 atomes de carbone; $R^3$ représente, en outre,

un groupe alcényle ou un groupe alcynyle à chaîne droite ou ramifiée contenant chacun de 2 à 6 atomes de carbone; un groupe cycloalcényle contenant de 5 à 7

atomes de carbone, éventuellement substitué de 1 à 5 fois de manière identique ou différente par un groupe alkyle contenant de 1 à 4 atomes de carbone; ou représente un groupe cycloalkyle contenant de 3 à 6 atomes de carbone, éventuellement substitué de 1 à 5 fois de manière identique ou différente, dans lequel, comme substituants, il convient de mentionner

un atome d'halogène, un groupe alkyle et un groupe alcoxy contenant chacun de 1 à 4 atomes de carbone, un groupe cyano, une groupe amino, un groupe carbamoyle, un groupe alkylamino, un groupe dialkylamino, un groupe alkylcarbamoyle et un groupe dialkylcarbamoyle contenant chacun de 1 à 4 atomes de carbone dans chaque fraction alkyle, un groupe alcoxycarbonylamino contenant de 1 à 4 atomes de carbone dans la fraction alcoxy, un groupe cycloalkyle et un groupe cycloalkylalkyle contenant 5 ou 6 atomes de carbone dans la fraction cycloalkyle et de 1 à 4 atomes de carbone dans la fraction alkyle à chaîne droite ou ramifiée, un groupe phényle éventuellement substitué de 1 à 5 fois de manière identique ou différente par un atome d'halogène et par un groupe alkyle contenant de 1 à 4 atomes de carbone et un groupe pyrrolidone; $R^3$ représente, en outre,

un groupe phényle éventuellement substitué de 1 à 5 fois de manière identique ou différente par un atome d'halogène, par un groupe alkyle et un groupe alcoxy contenant chacun de 1 à 4 atomes de carbone, par un groupe halogénalkyle ou par un groupe halogénalcoxy contenant chacun 1 ou 2 atomes de carbone et de 1 à 5 atomes d'halogène identiques ou différents; ou représente un composé hétérocyclique penta- ou hexagonal contenant de 1 à 3 hétéroatomes identiques ou différents, éventuellement substitué de 1 à 5 fois de manière identique ou différente; comme substituants, on mentionnera : un atome d'halogène, un groupe mercapto, un groupe phényle, un groupe alkyle à chaîne droite ou ramifiée, un groupe alcoxy, un groupe alkylthio, un groupe alkylsulfinyle et un groupe alkylsulfonyle contenant de 1 à 4 atomes de carbone dans chaque fraction alkyle;

$R^4$ a la signification donnée pour $R^3$, représente des composés carbocycliques polycycliques ou encore le groupement $-OR^{IV}$,

ou

A et $R^2$ représentent ensemble avec les atomes auxquels ils sont liés, un noyau de formule

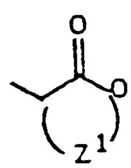

dans laquelle $Z^1$ représente une chaîne alkylène droite ou ramifiée contenant de 1 à 4 atomes de carbone,

ou

B et $R^3$ représentent, conjointement avec les atomes auxquels ils sont liés, un noyau de formule

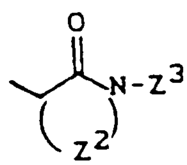

dans laquelle $Z^2$ représente une chaîne alkylène droite ou ramifiée contenant de 1 à 4 atomes de carbone et $Z^3$ représente un atome d'hydrogène ou un groupe alkyle contenant de 1 à 4 atomes de carbone,

$R^3$ et $R^4$ représentent, conjointement avec l'atome d'azote auquel ils sont liés, un composé hétérocyclique mono-, bi- ou tricyclique ou un composé spirohétérocyclique contenant

de 1 à 3 hétéroatomes supplémentaires identiques ou différents, éventuellement substitué de 1 à 5 fois de manière identique ou différente, et dans lequel, comme substituants, on mentionnera : un groupe alkyle ou un groupe alcoxy à chaîne droite ou ramifiée contenant chacun de 1 à 4 atomes de carbone, un groupe cycloalkyle contenant de 3 à 6 atomes de carbone, le groupe hydroxy ou le groupe oxo, un groupe alcényle à chaîne droite ou ramifiée contenant de 2 à 4 atomes de carbone, un groupe hydroxycarbonyle, un groupe alcoxycarbonyle contenant de 1 à 4 atomes de carbone dans la fraction alkyle à chaîne droite ou ramifiée; un groupe carbamoyle, un groupe alkyl- ou dialkylcarbamoyle contenant chacun de 1 à 4 atomes de carbone dans chaque fraction alkyle, ainsi qu'un groupe phényle ou bien un groupe phénylalkyle contenant de 1 à 4 atomes de carbone dans la fraction alkyle à chaîne droite ou ramifiée, chacun étant éventuellement substitué 1 ou 2 fois de manière identique ou différente par un atome d'halogène ou par un groupe alkyle et un groupe alcoxy à chaîne droite ou ramifiée contenant chacun de 1 à 4 atomes de carbone,

R représente un groupe alkyle à chaîne droite ou ramifiée contenant de 1 à 6 atomes de carbone, substitué une ou deux fois, dans lequel, comme substituants, il convient de mentionner : un atome d'halogène, $-COOR^{I}$, $-CONR^{II}R^{III}$, $-OR^{IV}$, un groupe acyle contenant de 2 à 9 atomes de carbone, un groupe hétérocyclyle à trois termes ou hexagonal contenant de 1 à 3 hétéroatomes, éventuellement substitué de 1 à 5 fois de manière identique ou différente par un atome d'halogène et par un groupe alkyle contenant de 1 à 4 atomes de carbone, à l'exception du groupe azolyle; en outre, R représente de préférence un groupe cycloalcényle contenant de 5 à 7 atomes de carbone, éventuellement substitué de 1 à 5 fois de manière identique ou différente par un groupe alkyle contenant de 1 à 4 atomes de carbone,

$R^{I}$ représente un atome d'hydrogène ou un groupe alkyle à chaîne droite ou ramifiée contenant de 1 à 6 atomes de carbone,

$R^{II}$ représente un atome d'hydrogène, un groupe alkyle à chaîne droite ou ramifiée contenant de 1 à 6 atomes de carbone, un groupe phénylalkyle contenant de 1 à 4 atomes de carbone dans la fraction alkyle à chaîne droite ou ramifiée, éventuellement substitué de 1 à 5 fois de manière identique ou différente, dans lequel, comme substituants du groupe phényle, on mentionnera : un atome d'halogène, un groupe alkyle et un groupe alcoxy contenant chacun de 1 à 4 atomes de carbone, un groupe halogénalkyle et un groupe halogénalcoxy contenant chacun 1 ou 2 atomes de carbone et de 2 à 5 atomes d'halogène identiques ou différents; $R^{II}$ représente, en outre, un groupe alcoxycarbonylalkyle, un groupe carbamoylalkyle, un groupe alkylcarbamoylalkyle ou un groupe dialkylcarbamoylalkyle contenant chacun de 1 à 4 atomes de carbone dans chaque fraction alkyle ou encore un groupe cycloalkyle contenant de 3 à 6 atomes de carbone, éventuellement substitué par un groupe alkyle à chaîne droite ou ramifiée contenant de 1 à 4 atomes de carbone,

$R^{III}$ représente un atome d'hydrogène, un groupe alkyle à chaîne droite ou ramifiée contenant de 1 à 6 atomes de carbone, un groupe phénylalkyle contenant de 1 à 4 atomes de carbone dans la fraction alkyle à chaîne droite ou ramifiée, éventuellement substitué de 1 à 5 fois de manière identique ou différente, dans lequel, comme substituants du groupe phényle, entrent

en ligne de compte, les substituants du groupe phényle mentionnés pour $R^{II}$; $R^{III}$ représente, en outre, un groupe alcoxycarbonylalkyle, un groupe carbamoylalkyle, un groupe alkylcarbamoylalkyle ou un groupe dialkylcarbamoylalkyle contenant chacun de 1 à 4 atomes de carbone dans chaque fraction alkyle ou encore un groupe cycloalkyle contenant de 3 à 6 atomes de carbone, éventuellement substitué par un groupe alkyle à chaîne droite ou ramifiée contenant de 1 à 4 atomes de carbone;

$R^{IV}$ représente un atome d'hydrogène, un groupe alkyle à chaîne droite ou ramifiée contenant de 1 à 6 atomes de carbone, un groupe phénylalkyle contenant de 1 à 4 atomes de carbone dans la fraction alkyle à chaîne droite ou ramifiée, éventuellement substitué de 1 à 5 fois de manière identique ou différente, dans lequel, comme substituants du groupe phényle, entrent

en ligne de compte, les substituants du groupe phényle mentionnés pour $R^{II}$; $R^{IV}$ représente, en outre, un groupe acyle contenant de 2 à 9 atomes de carbone, et

n représente les nombres 0, 1 ou 2,

leurs isomères géométriques et optiques, ainsi que leurs mélanges d'isomères.

**3.** Procédé selon la revendication 1, dans laquelle, dans la formule (I)

$R^1$ représente les groupements -A-COOR$^2$ ou -B-CONR$^3$R$^4$,

A représente les groupements $-CH_2-$, $-CH(CH_3)-$ ou $-CH_2CH_2-$,

B représente les groupements $-CH_2-$, $-CH(CH_3)-$ ou $-CH_2CH_2-$,

$R^2$ représente un groupe alkyle à chaîne droite ou ramifiée contenant de 1 à 4 atomes de carbone,

$R^3$ représente un atome d'hydrogène ou un groupe alkyle contenant de 1 à 4 atomes de carbone, à chaîne droite ou ramifiée, éventuellement substitué 1 ou 2 fois de manière identique ou différente, dans lequel, comme substituants, on mentionnera: un atome d'halogène, un groupe cyano, -COOR$^I$, -CONR$^{II}$R$^{III}$, -NR$^{II}$R$^{III}$,-OR$^{IV}$, -S(O)$_n$R$^{IV}$, un groupe acyle contenant de 2 à 9 atomes de carbone, un groupe phényle éventuellement substitué de 1 à 3 fois de manière identique ou différente par un atome d'halogène et par un groupe alkyle contenant de 1 à 4 atomes de carbone, un composé hétérocyclique penta- ou hexagonal contenant de 1 à 3 hétéroatomes identiques ou différents, éventuellement substitué de 1 à 3 fois de manière identique ou différente par un atome d'halogène, par un groupe alkyle et un groupe alcoxy à chaîne droite ou ramifiée contenant chacun de 1 à 4 atomes de carbone, ainsi qu'un groupe cycloalkyle contenant de 3 à 6 atomes de carbone et un groupe cycloalcényle contenant de 5 à 7 atomes de carbone, chacun étant éventuellement substitué par un groupe alkyle à chaîne droite ou ramifiée contenant de 1 à 4 atomes de carbone; $R^3$ représente, en outre, un groupe alcényle ou un groupe alcynyle à chaîne droite ou ramifiée contenant chacun de 2 à 6 atomes de carbone; un groupe cycloalcényle contenant de 5 à 7 atomes de carbone, éventuellement substitué par un groupe alkyle à chaîne droite ou ramifiée contenant de 1 à 4 atomes de carbone; un groupe cycloalkyle contenant de 3 à 6 atomes de carbone, éventuellement substitué, dans lequel, comme substituants, on mentionnera : un atome d'halogène, un groupe alkyle et un groupe alcoxy à chaîne droite ou ramifiée contenant chacun de 1 à 4 atomes de carbone, un groupe cyano, une groupe amino, un groupe carbamoyle, un groupe alkylamino, un groupe dialkylamino, un groupe alkylcarbamoyle et un groupe dialkylcarbamoyle, chacun de ces groupes étant à chaîne droite ou ramifiée et contenant chacun de 1 à 4 atomes de carbone dans chaque fraction alkyle, un groupe alcoxycarbonylamino contenant de 1 à 4 atomes de carbone dans la fraction alcoxy à chaîne droite ou ramifiée, un groupe cycloalkyle et un groupe cycloalkylalkyle contenant chacun 5 ou 6 atomes de carbone dans la fraction cycloalkyle et de 1 à 4 atomes de carbone dans la fraction alkyle à chaîne droite ou ramifiée, un groupe phényle éventuellement substitué de 1 à 3 fois de manière identique ou différente par un atome d'halogène ou par un groupe alkyle contenant de 1 à 4 atomes de carbone et un groupe pyrrolidone; $R^3$ représente, en outre, un groupe phényle éventuellement substitué de 1 à 3 fois de manière identique ou différente par un atome d'halogène, par un groupe alkyle et un groupe alcoxy à chaîne droite ou ramifiée contenant chacun de 1 à 4 atomes de carbone, par un groupe halogénalkyle et un groupe halogénalcoxy contenant chacun 1 ou 2 atomes de carbone et de 1 à 5 atomes d'halogène identiques ou différents; ou encore représente un compose hétérocyclique penta- ou hexagonal contenant de 1 à 3 hétéroatomes identiques ou différents, éventuellement substitué de 1 à 3 fois de manière identique ou différente; comme substituants pour les composés hétérocycliques, on mentionnera : un atome d'halogène, un groupe mercapto, un groupe phényle, un groupe alkyle, un groupe alcoxy, un groupe alkylthio, un groupe alkylsulfinyle et un groupe alkylsulfonyle, chacun de ces groupes étant à chaîne droite ou ramifiée et contenant chacun de 1 à 4 atomes de carbone dans la fraction alkyle;

$R^4$ a les significations données pour $R^3$, représente des composés carbocycliques polycycliques ou le groupement -OR$^{IV}$,

ou

A et $R^2$ conjointement avec les atomes auxquels ils sont liés, représentent des cycles penta- ou hexagonaux éventuellement substitués par un groupe alkyle contenant de 1 à 4 atomes de carbone, répondant aux formules

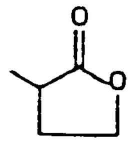

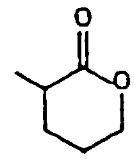

ou

ou

B et $R^3$ représentent ensemble avec les atomes auxquels ils sont liés, des cycles penta- à heptagonaux éventuellement substitués par un groupe alkyle contenant de 1 à 4 atomes de carbone, répondant aux formules

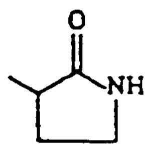

,

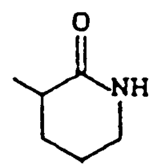

et

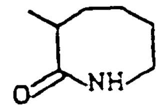

ou

$R^3$ et $R^4$ représentent, conjointement avec l'atome d'azote auquel ils sont liés, un composé hétérocyclique mono-, bi- ou tricyclique ou un composé spirohétérocyclique éventuellement substitué de 1 à 5 fois de manière identique ou différente, dans lequel, comme composés hétérocycliques, on mentionnera : l'oxazolidine, la pyrrolidine, l'imidazolidine, la pipéridine, la pipérazine, la morpholine, la thiomorpholine, le 1,3-oxazane ou le 1,3-diazane; ces composés hétérocycliques peuvent être éventuellement condensés à 1 ou 2 cycles benzéniques ou cyclohexane, ou éventuellement être reliés par un pont méthylène ou éthylène.

Comme substituants pour tous les hétérosystèmes, on mentionnera :

un groupe alkyle ou un groupe alcoxy à chaîne droite ou ramifiée contenant chacun de 1 à 4 atomes de carbone, un groupe cycloalkyle contenant de 3 à 6 atomes de carbone, le groupe hydroxy ou le groupe oxo, un groupe alcényle à chaîne droite ou ramifiée contenant de 2 à 4 atomes de carbone, un groupe hydroxycarbonyle, un groupe alcoxycarbonyle contenant de 1 à 4 atomes de carbone dans la fraction alkyle à chaîne droite ou ramifiée, un groupe carbamoyle, un groupe alkyl- ou dialkylcarbamoyle contenant chacun de 1 à 4 atomes de carbone dans chaque fraction alkyle, ainsi qu'un groupe phényle ou un groupe phénylalkyle contenant de 1 à 4 atomes de carbone dans la fraction alkyle à chaîne droite ou ramifiée, chacun étant éventuellement substitué 1 ou 2 fois de manière identique ou différente par un atome d'halogène ou par un groupe alkyle et un groupe alcoxy à chaîne droite ou ramifiée contenant chacun de 1 à 4 atomes de carbone,

R représente un groupe alkyle substitué contenant de 1 à 4 atomes de carbone à chaîne droite ou ramifiée, dans lequel, comme substituants, on mentionnera :

un atome d'halogène, $-COOR^{I}$, $-CONR^{II}R^{III}$, $-OR^{IV}$, un groupe acyle contenant de 2 à 9 atomes de carbone, des composés hétérocycliques éventuellement substitués de 1 à 3 fois de manière identique ou différente par un groupe alkyle contenant 1 ou 2 atomes de carbone, répondant aux formules

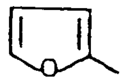, 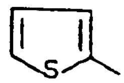, 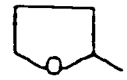, 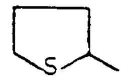,

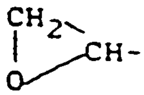, 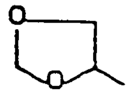 et 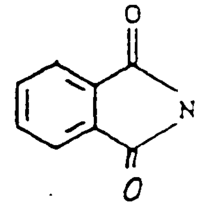,

- R représente, en outre, un groupe cyclohexényle ou un groupe cyclopentényle éventuellement substitué de 1 à 3 fois par un groupe méthyle,
- $R^{I}$ représente un groupe alkyle à chaîne droite ou ramifiée contenant de 1 à 4 atomes de carbone,
- $R^{II}$ représente un atome d'hydrogène, un groupe alkyle à chaîne droite ou ramifiée contenant de 1 à 4 atomes de carbone, un groupe benzyle et un groupe phénéthyle, chacun étant éventuellement substitué de 1 à 3 fois de manière identique ou différente, dans lesquels, comme substituants du groupe phényle, on mentionnera chaque fois : un atome de fluor, un atome de chlore, un groupe méthyle, un groupe méthoxy et un groupe trifluorométhyle; $R^{II}$ représente, en outre, un groupe alcoxycarbonylalkyle contenant de 1 à 4 atomes de carbone dans la fraction alcoxy et 1 ou 2 atomes de carbone dans la fraction alkyle, un groupe carbamoylalkyle contenant 1 ou 2 atomes de carbone dans la fraction alkyle, un groupe alkylcarbamoylalkyle et un groupe dialkylcarbamoylalkyle contenant chacun 1 ou 2 atomes de carbone dans chaque fraction alkyle ou encore un groupe cycloalkyle contenant de 3 à 6 atomes de carbone, éventuellement substitué de 1 à 3 fois de manière identique ou différente par un groupe méthyle et par un groupe éthyle,
- $R^{III}$ a les significations données pour $R^{II}$,
- $R^{IV}$ représente un atome d'hydrogène, un groupe alkyle à chaîne droite ou ramifiée contenant de 1 à 4 atomes de carbone, un groupe benzyle ou un groupe phénéthyle, chacun étant éventuellement substitué de 1 à 3 fois de manière identique ou différente, dans lequel, comme substituants du groupe phényle, on mentionnera chaque fois : un atome de fluor, un atome de chlore, un groupe méthyle, un groupe méthoxy et un groupe trifluorométhyle; $R^{IV}$ représente, en outre, un groupe acyle contenant de 2 à 9 atomes de carbone, et
- n représente les nombres 0, 1 ou 2,

leurs isomères géométriques et optiques, ainsi que leurs mélanges d'isomères.

**4.** Procédé selon la revendication 1, dans laquelle, dans la formule (I)

- $R^1$ représente le groupement $-B-CO-NR^3R^4$,
- B représente les groupements $-CH_2-$, $-CH(CH_3)-$ ou $-CH_2CH_2-$
- $R^3$ représente un atome d'hydrogène ou un groupe alkyle à chaîne droite ou ramifiée contenant de 1 à 4 atomes de carbone,
- $R^4$ représente un atome d'hydrogène ou un groupe alkyle à chaîne droite ou ramifiée contenant de 1 à 4 atomes de carbone,
- R représente un groupe alkyle substitué contenant 1 ou 2 atomes de carbone, dans lequel, comme substituants, on mentionnera : un atome d'halogène, un groupe alkylcarbonyle contenant de 1 à 4 atomes de carbone dans la fraction alkyle à chaîne droite ou ramifiée, les groupements $-COOR^{I}$, $-CONR^{II}R^{III}$, $-OR^{IV}$, ainsi que les composés hétérocycliques ci-après :

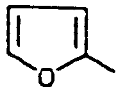 , 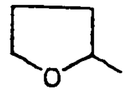 , 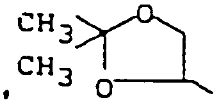 , 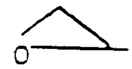 et

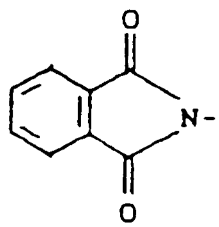 ;

R représente, en outre, un groupe 1-cyclohexényle éventuellement substitué de 1 à 3 fois par un groupe méthyle;

- $R^I$ représente un groupe méthyle, un groupe éthyle, un groupe n- ou i-propyle ou un groupe n-, i-, s- ou t-butyle,
- $R^{II}$ et $R^{III}$ sont identiques ou différents et représentent un atome d'hydrogène, un groupe méthyle, un groupe éthyle, un groupe n- ou i-propyle, un groupe benzyle éventuellement substitué de 1 à 3 fois de manière identique ou différente par un atome de chlore et par un groupe méthyle à titre de substituants, un groupe alcoxycarbonylalkyle contenant 1 ou 2 atomes de carbone dans chaque fraction alkyle, un groupe carbamoylalkyle, un groupe alkylcarbamoylalkyle ou un groupe dialkylcarbamoylalkyle contenant chacun 1 ou 2 atomes de carbone dans chaque fraction alkyle ou encore un groupe cyclohexyle éventuellement substitué de 1 à 3 fois par un groupe méthyle, et
- $R^{IV}$ représente un atome d'hydrogène, un groupe alkyle contenant 1 ou 2 atomes de carbone ou un groupe benzyle éventuellement substitué de 1 à 3 fois de manière identique ou différente, dans lequel, comme substituants du groupe phényle, on mentionnera : un atome de fluor, un atome de chlore et un groupe méthyle, leurs isomères géométriques et optiques, ainsi que leurs mélanges d'isomères.

**5.** Procédé selon la revendication 1, dans laquelle, dans la formule (I)

- $R^1$ représente le groupement $-B-CO-NR^3R^4$,
- B représente les groupements $-CH_2-$, $-CH(CH_3)-$ ou $-CH_2CH_2-$
- $R^3$ représente un atome d'hydrogène, un groupe alkyle à chaîne droite ou ramifiée contenant de 1 à 4 atomes de carbone ou un groupe alkyle contenant de 1 à 3 atomes de carbone, substitué 1 ou 2 fois, dans lequel, comme substituants, on mentionnera : un atome de chlore, un groupe hydroxyle, un groupe alkylcarbonyloxy contenant de 1 à 4 atomes de carbone dans la fraction alkyle à chaîne droite ou ramifiée, un groupe alkylcarbonyle contenant de 1 à 4 atomes de carbone dans la fraction alkyle à chaîne droite ou ramifiée, un groupe alcoxy et un groupe alkylthio contenant chacun 1 ou 2 atomes de carbone, un groupe phényle éventuellement substitué de 1 à 3 fois de manière identique ou différente par un atome de chlore et par un groupe méthyle, un groupe 2-furyle, un groupe 2-pyridyle, un groupe 1-morpholino, un groupe cyclopropyle et un groupe cyclohexyle; $R^3$ représente, en outre, un groupe allyle ou un groupe propargyle, un groupe 1-cyclohexényle ou un groupe cyclohexyle substitué de 1 à 3 fois de manière identique ou différente, dans lequel, comme substituants, on mentionnera : un atome de chlore, un groupe méthyle, un groupe éthyle, un groupe méthoxy, un groupe cyano, un groupe amino, un groupe alkyl- ou dialkylamino contenant chacun 1 ou 2 atomes de carbone dans chaque fraction alkyle, un groupe carbamoyle, un groupe alkyl- ou dialkylcarbamoyle contenant chacun 1 ou 2 atomes de carbone dans chaque fraction alkyle, un groupe cyclohexyle, un groupe cyclohexylalkyle contenant 1 ou 2 atomes de carbone dans la fraction alkyle ou encore une 1-pyrrolidin-2-one;

  $R^3$ représente, en outre, un groupe phényle éventuellement substitué de 1 à 3 fois de manière identique ou différente par un atome de chlore et par un groupe méthyle, un groupe 2-pyridyle ou un groupe 2-pyrimidinyle, chacun étant éventuellement substitué de 1 à 3 fois par un groupe méthyle, un groupe 2-thiazolyle éventuellement substitué par un groupe phényle, un groupe 2-benzothiazolyle éventuellement substitué de 1 à 3 fois de manière

identique ou différente par un atome de chlore, par un groupe méthyle et par un groupe méthoxy, un groupe 1,2,4-triazol-3-yle, un groupe 1,2,4-thiadiazol-5-yle éventuellement substitué par un groupe phényle, un groupe 1,3,4-thiadiazol-5-yle éventuellement substitué de 1 à 3 fois de manière identique ou différente par un groupe mercapto, par un groupe alkyle à chaîne droite ou ramifiée contenant de 1 à 4 atomes de carbone, par un groupe alkylthio, par un groupe alkylsulfinyle et un groupe alkylsulfonyle, chacun étant à chaîne droite ou ramifiée et contenant chacun de 1 à 4 atomes de carbone, ou encore représente le groupement

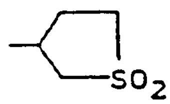 ,

$R^4$ représente un groupe alkyle contenant de 1 à 3 atomes de carbone, substitué 1 ou 2 fois, dans lequel, comme substituants, on mentionnera : un atome de chlore, un groupe hydroxyle, un groupe alkylcarbonyloxy contenant de 1 à 4 atomes de carbone dans la fraction alkyle à chaîne droite ou ramifiée, un groupe alkylcarbonyle contenant de 1 à 4 atomes de carbone dans la fraction alkyle à chaîne droite ou ramifiée, un groupe alcoxy et un groupe alkylthio contenant chacun 1 ou 2 atomes de carbone, un groupe phényle éventuellement substitué de 1 à 3 fois de manière identique ou différente par un atome de chlore et par un groupe méthyle, un groupe 2-furyle, un groupe 2-pyridyle, un groupe 1-morpholino, un groupe cyclopropyle et un groupe cyclohexyle; $R^4$ représente, en outre, un groupe allyle ou un groupe propargyle, un groupe 1-cyclohexényle ou un groupe cyclohexyle substitué de 1 à 3 fois de manière identique ou différente, dans lequel, comme substituants, on mentionnera : un atome de chlore, un groupe méthyle, un groupe éthyle, un groupe méthoxy, un groupe cyano, un groupe amino, un groupe alkyl- ou dialkylamino contenant chacun 1 ou 2 atomes de carbone dans chaque fraction alkyle, un groupe carbamoyle, un groupe alkyl- ou dialkylcarbamoyle contenant chacun 1 ou 2 atomes de carbone dans chaque fraction alkyle, un groupe cyclohexyle, un groupe cyclohexylalkyle contenant 1 ou 2 atomes de carbone dans la fraction alkyle, ainsi que la 1-pyrrolidin-2-one;

$R^4$ représente, en outre, un groupe phényle éventuellement substitué de 1 à 3 fois de manière identique ou différente par un atome de chlore et par un groupe méthyle, un groupe 2-pyridyle ou un groupe 2-pyrimidinyle, chacun étant éventuellement substitué de 1 à 3 fois par un groupe méthyle, un groupe 2-thiazolyle éventuellement substitué par un groupe phényle, un groupe 2-benzothiazolyle éventuellement substitué de 1 à 3 fois de manière identique ou différente par un atome de chlore, par un groupe méthyle et par un groupe méthoxy, un groupe 1,2,4-triazol-3-yle, un groupe 1,2,4-thiadiazol-5-yle éventuellement substitué par un groupe phényle, un groupe 1,3,4-thiadiazol-5-yle éventuellement substitué de 1 à 3 fois de manière identique ou différente par un groupe mercapto, par un groupe alkyle à chaîne droite ou ramifiée contenant de 1 à 4 atomes de carbone, par un groupe alkylthio, par un groupe alkylsulfinyle et un groupe alkylsulfonyle, chacun étant à chaîne droite ou ramifiée et contenant chacun de 1 à 4 atomes de carbone, ou encore représente le groupement

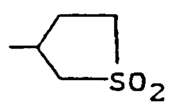 ,

$R^4$ représente, en outre, un groupe hydroxyle, un groupe alcoxy contenant 1 ou 2 atomes de carbone, un groupe benzyloxy éventuellement substitué de 1 à 3 fois de manière identique ou différente par un atome de chlore et par un groupe méthyle, ainsi qu'un groupe 1-adamantyle, un groupe 2-norbornyle, un groupe 1- ou 2-décalyle ou encore un groupe 1- ou 2-tétralyle;

R représente un groupe alkyle substitué contenant 1 ou 2 atomes de carbone, dans lequel, comme substituants, on mentionnera : un atome d'halogène, un groupe alkylcarbonyle contenant de 1 à 4 atomes de carbone dans la fraction alkyle, les groupements -COOR$^{I}$, -CONR$^{II}$R$^{III}$, -OR$^{IV}$, ainsi que les composés hétérocycliques ci-après :

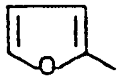 , 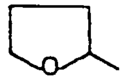 , 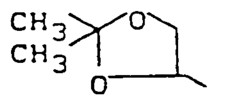 , 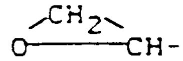

et

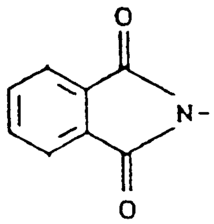 ;

R représente, en outre, un groupe 1-cyclohexényle éventuellement substitué de 1 à 3 fois par un groupe méthyle;

$R^I$ représente un groupe méthyle, un groupe éthyle, un groupe n- ou i-propyle ou un groupe n-, i-, s- ou t-butyle,

$R^{II}$ et $R^{III}$ sont identiques ou différents et représentent un atome d'hydrogène, un groupe méthyle, un groupe éthyle, un groupe n- ou i-propyle, un groupe benzyle éventuellement substitué de 1 à 3 fois de manière identique ou différente par un atome de chlore ou par un groupe méthyle à titre de substituants, un groupe alcoxycarbonylalkyle contenant 1 ou 2 atomes de carbone dans chaque fraction alkyle, un groupe carbamoylalkyle, un groupe alkylcarbamoylalkyle ou un groupe dialkylcarbamoylalkyle contenant chacun 1 ou 2 atomes de carbone dans chaque fraction alkyle ou encore un groupe cyclohexyle éventuellement substitué de 1 à 3 fois par un groupe méthyle, et

$R^{IV}$ représente un atome d'hydrogène, un groupe alkyle contenant 1 ou 2 atomes de carbone ou encore un groupe benzyle éventuellement substitué de 1 à 3 fois de manière identique ou différente, dans lequel, comme substituants du groupe phényle, on mentionnera : un atome de fluor, un atome de chlore et un groupe méthyle,

leurs isomères géométriques et optiques, ainsi que leurs mélanges d'isomères.

**6.** Procédé selon la revendication 1, dans laquelle, dans la formule (I)

$R^1$ représente le groupement $-B-CO-NR^3R^3$,

B représente les groupements $-CH_2-$, $-CH(CH_3)-$ ou $-CH_2CH_2-$

$R^3$ et $R^4$ représentent, conjointement avec l'atome d'azote auquel ils sont liés, les composés hétérocycliques mono-, bi- ou tricycliques ou les composés spirohétérocycliques ci-après :

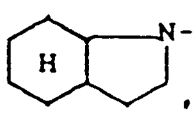 , 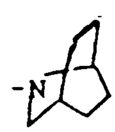 , 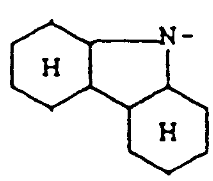 ,

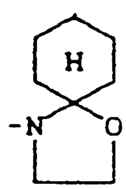 , 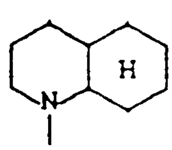 ,

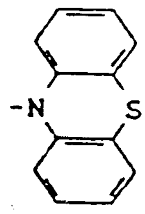 , 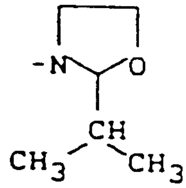 , 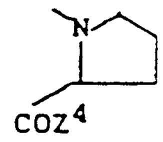

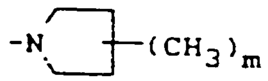 , 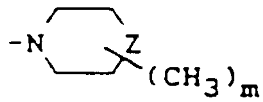

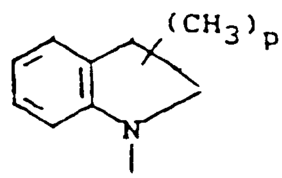 , 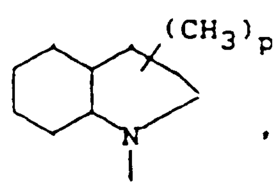 ,

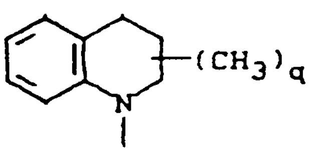 , 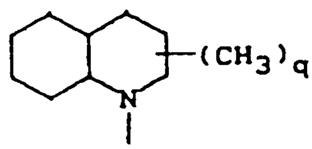 ,

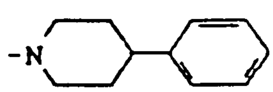 , 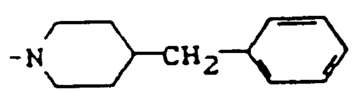 ,

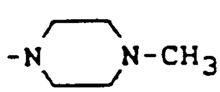 , 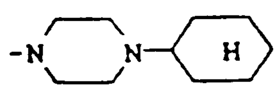 , 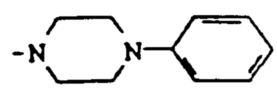 ,

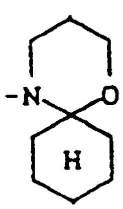 , 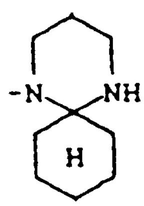 , 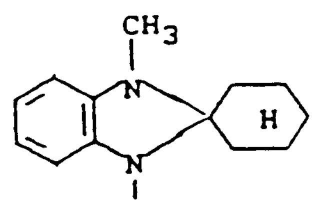 ,

dans lesquels

- Z représente un atome d'oxygène ou le groupe $CH_2$,
- $Z^4$ représente un groupe hydroxyle, un groupe méthoxy, un groupe éthoxy, un groupe amino, un groupe méthylamino, un groupe éthylamino, un groupe diméthylamino ou un groupe éthylméthylamino,
- m représente les nombres 0, 1, 2, 3 ou 4,
- p représente les nombres 0, 1 ou 2 et
- q représente les nombres 0, 1, 2 ou 3 et
- R représente un groupe alkyle substitué contenant 1 ou 2 atomes de carbone, dans lequel, comme substituants, on mentionnera : un atome d'halogène, un groupe alkylcarbonyle contenant de 1 à 4 atomes de carbone dans la fraction alkyle à chaîne droite ou ramifiée, les groupements $-COOR^{I}$, $-CONR^{II}R^{III}$, $-OR^{IV}$, ainsi que les composés hétérocycliques ci-après :

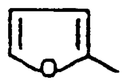 , 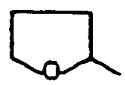 , 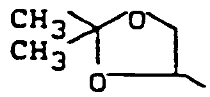 , 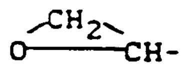

et

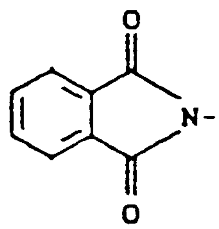 ;

R — représente, en outre, un groupe 1-cyclohexényle éventuellement substitué de 1 à 3 fois par un groupe méthyle;

$R^{I}$ — représente un groupe méthyle, un groupe éthyle, un groupe n- ou i-propyle ou un groupe n-, i-, s- ou t-butyle,

$R^{II}$ et $R^{III}$ — sont identiques ou différents et représentent un atome d'hydrogène, un groupe méthyle, un groupe éthyle, un groupe n- ou i-propyle, un groupe benzyle éventuellement substitué de 1 à 3 fois de manière identique ou différente par un atome de chlore et par un groupe méthyle à titre de substituants, un groupe alcoxycarbonylalkyle contenant 1 ou 2 atomes de carbone dans chaque fraction alkyle, un groupe carbamoylalkyle, un groupe alkylcarbamoylalkyle ou un groupe dialkylcarbamoylalkyle contenant chacun 1 ou 2 atomes de carbone dans chaque fraction alkyle ou encore un groupe cyclohexyle éventuellement substitué de 1 à 3 fois par un groupe méthyle, et

$R^{IV}$ — représente un atome d'hydrogène, un groupe alkyle contenant 1 ou 2 atomes de carbone ou un groupe benzyle éventuellement substitué de 1 à 3 fois de manière identique ou différente, dans lequel, comme substituants du groupe phényle, on mentionnera : un atome de fluor, un atome de chlore et un groupe méthyle,

leurs isomères géométriques et optiques, ainsi que leurs mélanges d'isomères.

**7.** Procédé selon la revendication 1, dans laquelle, dans la formule (I)

$R^1$ — représente le groupement $-A-COOR^2$,

A — représente les groupements $-CH_2-$, $-CH(CH_3)-$ ou $-CH_2CH_2-$

$R^2$ — représente un groupe méthyle ou un groupe éthyle,

R — représente un groupe alkyle contenant 1 ou 2 atomes de carbone, substitué 1 ou 2 fois, dans lequel, comme substituants, on mentionnera : un atome d'halogène, un groupe alkylcarbonyle contenant de 1 à 4 atomes de carbone dans la fraction alkyle à chaîne droite ou ramifiée, $-COOR^{I}$, $-CONR^{II}R^{III}$, $-OR^{IV}$, ainsi que les composés hétérocycliques ci-après :

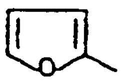 , 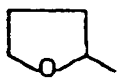 , 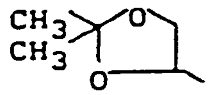 , 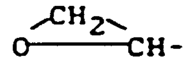

et

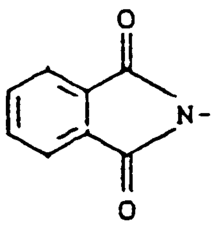 ;

R représente, en outre, un groupe 1-cyclohexényle éventuellement substitué de 1 à 3 fois par un groupe méthyle;

$R^I$ représente un groupe méthyle, un groupe éthyle, un groupe n- ou i-propyle ou un groupe n-, i-, s- ou t-butyle,

$R^{II}$ et $R^{III}$ sont identiques ou différents et représentent un atome d'hydrogène, un groupe méthyle, un groupe éthyle, un groupe n- ou i-propyle, un groupe benzyle éventuellement substitué de 1 à 3 fois de manière identique ou différente par un atome de chlore et par un groupe méthyle à titre de substituants, un groupe alcoxycarbonylalkyle contenant 1 ou 2 atomes de carbone dans chaque fraction alkyle, un groupe carbamoylalkyle, un groupe alkylcarbamoylalkyle ou un groupe dialkylcarbamoylalkyle contenant chacun 1 ou 2 atomes de carbone dans chaque fraction alkyle ou encore un groupe cyclohexyle éventuellement substitué de 1 à 3 fois par un groupe méthyle, et

$R^{IV}$ représente un atome d'hydrogène, un groupe alkyle contenant 1 ou 2 atomes de carbone ou un groupe benzyle éventuellement substitué de 1 à 3 fois de manière identique ou différente, dans lequel, comme substituants du groupe phényle, on mentionnera : un atome de fluor, un atome de chlore et un groupe méthyle,

leurs isomères géométriques et optiques, ainsi que leurs mélanges d'isomères.

**8.** Agents de lutte contre les parasites, caractérisés par une teneur en au moins un dérivé de (2-cyano-2-oximinoacétyl)-aminoacides de formule (I) selon les revendications 1 à 7.

**9.** Utilisation de dérivés de (2-cyano-2-oximinoacétyl)-aminoacides de formule (I) selon les revendications 1 à 7, pour lutter contre les parasites.

**10.** Procédé pour lutter contre les parasites, caractérisé en ce qu'on laisse agir des dérivés de (2-cyano-2-oximinoacétyl)-aminoacides de formule (I) selon les revendications 1 à 7, sur des parasites et/ou sur leur biotope.

**11.** Procédé pour la préparation d'agents de lutte contre les parasites, caractérisé en ce qu'on mélange des dérivés de (2-cyano-2-oximinoacétyl)-aminoacides de formule (I) selon les revendications 1 à 7, avec des diluants et/ou des agents tensioactifs.